(19) Europäisches Patentamt
European Patent Office
Office européen des brevets

(11) EP 4 393 914 A1

(12) EUROPEAN PATENT APPLICATION
published in accordance with Art. 153(4) EPC

(43) Date of publication:
**03.07.2024 Bulletin 2024/27**

(21) Application number: **22860547.3**

(22) Date of filing: **24.08.2022**

(51) International Patent Classification (IPC):
***C07D 401/04*** (2006.01) ***C07D 413/14*** (2006.01)
***C07D 513/04*** (2006.01) ***C07D 405/14*** (2006.01)
***C07D 495/04*** (2006.01) ***A61K 31/4439*** (2006.01)
***A61K 31/444*** (2006.01) ***A61K 31/497*** (2006.01)
***A61K 31/4164*** (2006.01) ***A61P 3/10*** (2006.01)

(52) Cooperative Patent Classification (CPC):
**A61K 31/4164; A61K 31/4439; A61K 31/444; A61K 31/497; A61P 3/10; C07D 401/04; C07D 405/14; C07D 413/14; C07D 495/04; C07D 513/04**

(86) International application number:
**PCT/CN2022/114560**

(87) International publication number:
**WO 2023/025201 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.08.2021 CN 202110976089**
**29.09.2021 CN 202111154964**
**30.09.2021 CN 202111162621**

(71) Applicant: **Dongbao Purple Star (Hangzhou) Biopharmaceutical Co., Ltd.**
**Hangzhou, Zhejiang 310018 (CN)**

(72) Inventors:
- **MAO, Qinghua**
  **Shanghai 200131 (CN)**
- **WANG, Wei**
  **Shanghai 200131 (CN)**
- **TAN, Ye**
  **Shanghai 200131 (CN)**
- **LIANG, Bin**
  **Shanghai 200131 (CN)**
- **WANG, Lu**
  **Shanghai 200131 (CN)**
- **WU, Chengde**
  **Shanghai 200131 (CN)**
- **ZHANG, Yang**
  **Shanghai 200131 (CN)**
- **CHEN, Shuhui**
  **Shanghai 200131 (CN)**

(74) Representative: **Dehns**
**St. Bride's House**
**10 Salisbury Square**
**London EC4Y 8JD (GB)**

# (54) IMIDAZOCYCLIC COMPOUND AND APPLICATION THEREOF

(57) A series of imidazocyclic compounds and an application thereof, which specifically relate to a compound represented by formula (P) and a pharmaceutically acceptable salt thereof.

EP 4 393 914 A1

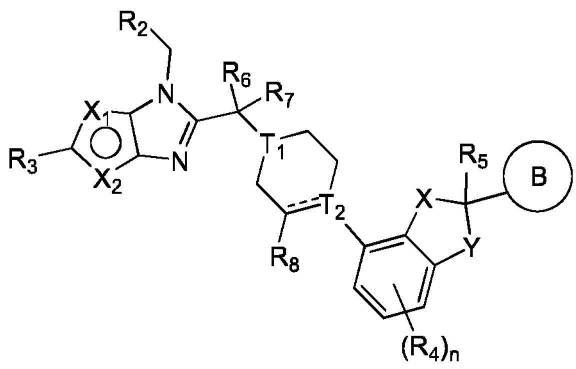

( P )

## Description

The present invention claims the right of the following priorities:

CN202110976089.5, application date: August 24, 2021;
CN202111154964.8, application date: September 29, 2021;
CN202111162621.6, application date: September 30, 2021.

TECHNICAL FIELD

The present disclosure relates to a series of imidazocyclic compounds and a use thereof, specifically to a compound of formula (P) and a pharmaceutically acceptable salt thereof.

BACKGROUND

Type 2 diabetes mellitus (T2DM) is one of the most common chronic metabolic diseases, frequently occurring in middle-aged and elderly people and obese individuals, and seriously affecting patients' physical and mental health and quality of life. The main pathogenic mechanisms of type 2 diabetes mellitus are: pancreatic β-cell dysfunction, insulin resistance, abnormal glucagon secretion, etc. Common hypoglycemic drugs (such as insulin, sulfonylurea drugs, and thiazolidinedione drugs) mainly work through improving patients' pancreatic β-cell secretion function or insulin resistance, but often cause adverse reactions such as hypoglycemia, which in turn have a negative impact on cardiovascular function. In recent years, diabetes-related clinical research guidelines have indicated that, in addition to glycemic control, clinical treatment of T2DM should also provide benefits for patients in terms of weight loss and cardiovascular health. Therefore, it is urgent to develop drugs with novel mechanisms of action for the treatment of T2DM.

Glucagon-like peptide-1 (GLP-1) is a hormone secreted by intestinal L cells after a meal, and is a polypeptide composed of 30 amino acids. After binding to the glucagon-like peptide-1 receptor (GLP-1R), GLP-1 enhances glucose-stimulated insulin secretion (GSIS) by upregulating downstream cAMP, MAPK, and other signals, promotes insulin secretion from pancreatic β cells, reduces the risk of hypoglycemia, promotes β cell proliferation, inhibits β cell apoptosis, inhibits glucagon secretion from pancreatic α cells, protects cardiovascular function, delays gastric emptying, and reduces food intake, thereby leading to weight loss. Therefore, the development of T2DM drugs based on GLP-1 is a new strategy, including GLP-1 analogs, GLP-1R agonists, and DPP-4 inhibitors.

GLP-1R agonists currently available in China, including liraglutide and exenatide, are essentially GLP-1 analogs, which can promote insulin secretion from pancreatic β-cells, protect the function of pancreatic β-cells, and have the obvious effect of reducing the body weight of the patients. However, these drugs need be administered subcutaneously or intravenously, which increases the risk of infection, has poor patient compliance and high costs, making them difficult to be widely applied in clinical practice. The only oral GLP-1R agonist, semaglutide, is of stringent administration conditions, high storage requirements, and high prices. Therefore, the development of more efficient, low-toxic small-molecule oral GLP-1R agonists has a better application prospect.

CONTENT OF THE PRESENT INVENTION

The present disclosure provides a compound of formula (P) or a pharmaceutically acceptable salt thereof,

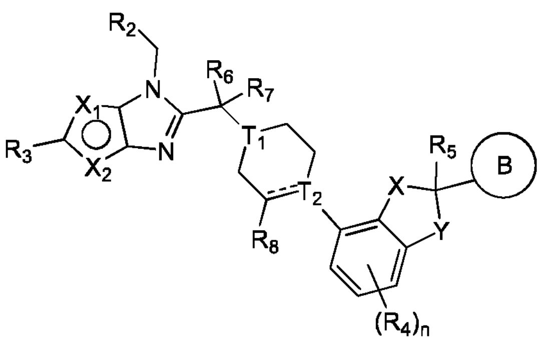

(P)

wherein

$\overline{---}$ is selected from a single bond and a double bond, and when $T_2$ is selected from N, $\overline{---}$ is selected from a single bond;
$T_1$ and $T_2$ are selected from N and $CR_9$;
X and Y are each independently selected from $CH_2$, O-$CH_2$, NH, O, and C(=O);
$X_1$ and $X_2$ are each independently selected from CH, N, O, and S, and the CH is optionally substituted by one F, Cl, Br, and $CH_3$;
ring B is selected from 5- to 6-membered heteroaryl, and the 5- to 6-membered heteroaryl is optionally substituted by 1, 2, or 3 Ri;
or, ring B is selected from phenyl, and the phenyl is optionally substituted by 1, 2, or 3 Ri, and in this case, X and Y are not simultaneously selected from O;
$R_1$ is selected from F, Cl, Br, I, OH, $NH_2$, CN, $CH_3$, and $OCH_3$;
$R_2$ is selected from

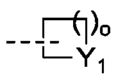 and 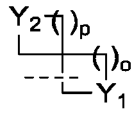,

and the

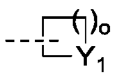 and 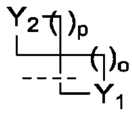

are optionally substituted by 1, 2, or 3 $R_a$;
$Y_1$ and $Y_2$ are each independently selected from $CH_2$, NH, and O;
o and p are each independently selected from 0, 1, 2, and 3;
$R_3$ is selected from -C(=O)-NH-$R_b$, -C(=O)-$R_b$, -C(=O)-NH-S(=O)$_2$-$R_b$, -S(=O)$_2$-NH-$R_b$, -S(=O)$_2$-$R_b$, -P(=O)($R_b$)$_2$, $C_{1-3}$ alkyl, tetrazolyl, isoxazolyl,

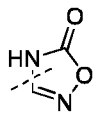, and 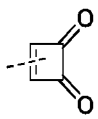,

and the $C_{1-3}$ alkyl, tetrazolyl, isoxazolyl,

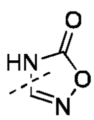, and 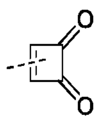

are optionally substituted by 1, 2, or 3 $R_b$;
$R_4$ is selected from D, F, Cl, Br, I, and $C_{1-3}$ alkyl, and the $C_{1-3}$ alkyl is optionally substituted by 1, 2, or 3 R;
$R_5$ is selected from H, D, and $CH_3$, and the $CH_3$ is optionally substituted by 1, 2, or 3 R;
$R_6$, $R_7$, $R_8$, and $R_9$ are each independently selected from H, D, and $C_{1-3}$ alkyl, and the $C_{1-3}$ alkyl is optionally substituted by 1, 2, or 3 R;
n is selected from 0, 1, and 2;
each $R_a$ is independently selected from F, Cl, Br, and I;
each $R_b$ is independently selected from OH, CN, $C_{1-3}$ alkyl, $C_{1-3}$ alkoxy, $C_{1-3}$ alkylamino, and oxazolyl, and the $C_{1-3}$ alkyl, $C_{1-3}$ alkoxy, and oxazolyl are optionally substituted by 1, 2, or 3 R;
each R is independently selected from D, F, Cl, Br, and I.

In some embodiments of the present disclosure, the $R_b$ is independently selected from OH, CN, $CH_3$, $CF_3$, and $OCH_3$, and other variables are as defined in the present disclosure.

In some embodiments of the present disclosure, the $R_2$ is selected from

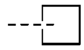

and

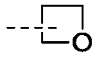,

and the

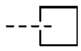 and 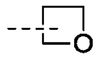

are optionally substituted by 1, 2, or 3 $R_a$, and other variables are as defined in the present disclosure.

In some embodiments of the present disclosure, the $R_2$ is selected from

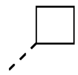

and

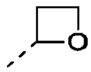,

and other variables are as defined in the present disclosure.

In some embodiments of the present disclosure, the $R_2$ is selected from

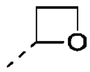,

and other variables are as defined in the present disclosure.

In some embodiments of the present disclosure, the $R_3$ is selected from -COOH, - C(=O)-NH-CN, -C(=O)-NH-OH, -C(=O)-NH-$OCH_3$, -C(=O)-$CF_3$, -$S(=O)_2$-NH-$CH_3$, and - $S(=O)_2$-OH, and other variables are as defined in the present disclosure.

In some embodiments of the present disclosure, the $R_3$ is selected from -COOH, and other variables are as defined in the present disclosure.

In some embodiments of the present disclosure, the $R_4$ is selected from F and $CH_3$, and other variables are as defined in the present disclosure.

In some embodiments of the present disclosure, the $R_5$ is selected from H, D, $CH_3$, $CF_3$, $CHF_2$, $CHD_2$, and $CD_3$, and other variables are as defined in the present disclosure.

In some embodiments of the present disclosure, the $R_5$ is selected from $CH_3$, and other variables are as defined in the present disclosure.

In some embodiments of the present disclosure, the $R_6$, $R_7$, $R_8$, and $R_9$ are each independently selected from H, D, and $CH_3$, and the $CH_3$ is optionally substituted by 1, 2, or 3 R, and other variables are as defined in the present disclosure.

In some embodiments of the present disclosure, the $R_6$ is selected from H, D, and $CH_3$, and other variables are as defined in the present disclosure.

In some embodiments of the present disclosure, the $R_7$ is selected from H, D, and $CH_3$, and other variables are as defined in the present disclosure.

In some embodiments of the present disclosure, the $R_8$ is selected from H, D, and $CH_3$, and other variables are as defined in the present disclosure.

In some embodiments of the present disclosure, the $R_9$ is selected from H, D, and $CH_3$, and other variables are as defined in the present disclosure.

In some embodiments of the present disclosure, the ring B is selected from 6-membered heteroaryl, and the 6-membered heteroaryl is optionally substituted by 1, 2, or 3 Ri, and other variables are as defined in the present disclosure.

In some embodiments of the present disclosure, the ring B is selected from pyridyl, pyrimidinyl, pyrazinyl, pyridazinyl, pyrazolyl, imidazolyl, thiazolyl, and oxazolyl, and the pyridyl, pyrimidinyl, pyrazinyl, pyridazinyl, pyrazolyl, imidazolyl, thiazolyl, and oxazolyl are optionally substituted by 1, 2, or 3 Ri, and other variables are as defined in the present disclosure.

In some embodiments of the present disclosure, the ring B is selected from pyridyl, and the pyridyl is optionally substituted by 1, 2, or 3 Ri, and other variables are as defined in the present disclosure.

In some embodiments of the present disclosure, the ring B is selected from

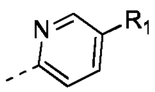, 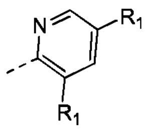, 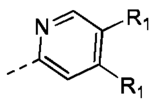, 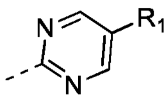, 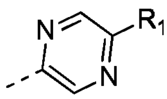, 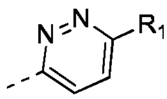,

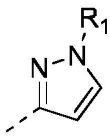, 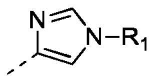, 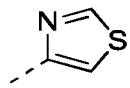, and 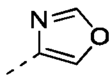,

and other variables are as defined in the present disclosure.

In some embodiments of the present disclosure, the ring B is selected from

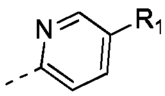, 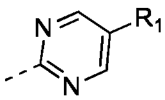, 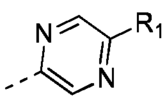, 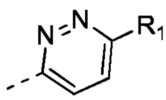, 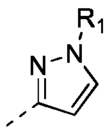, 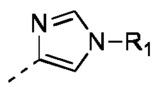, 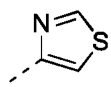,

and

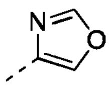,

and other variables are as defined in the present disclosure.

In some embodiments of the present disclosure, the ring B is selected from

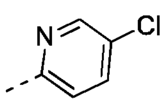, 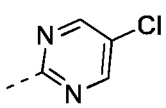, 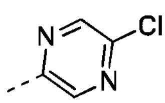, 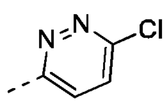, 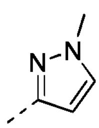, 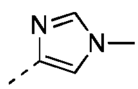, 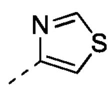,

and

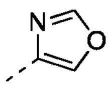,

and other variables are as defined in the present disclosure.

In some embodiments of the present disclosure, the ring B is selected from

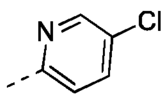 , 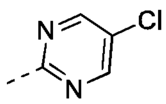 , 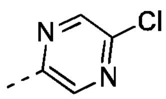 , 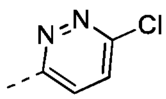 , 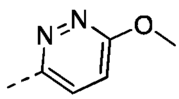 , 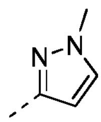 ,

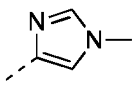 , 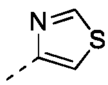 , and 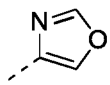 ,

and other variables are as defined in the present disclosure.

In some embodiments of the present disclosure, the ring B is selected from

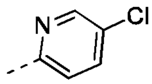 , 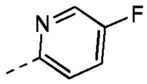 , 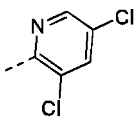 , 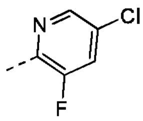 , 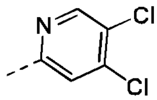 , 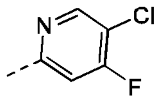 ,

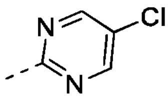 , 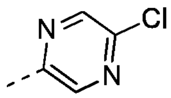 , 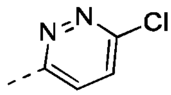 , 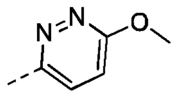 , 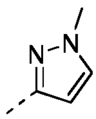 , 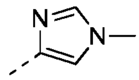 , 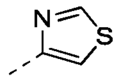 ,

and

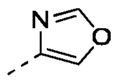 ,

and other variables are as defined in the present disclosure.

In some embodiments of the present disclosure, the ring B is selected from

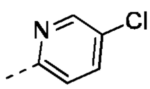 , 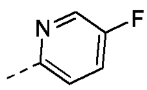 , 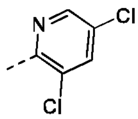 , 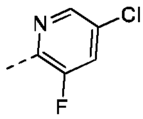 , 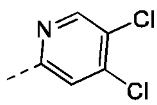 , and 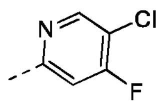 ,

and other variables are as defined in the present disclosure.

In some embodiments of the present disclosure, the ring B is selected from

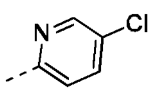 and 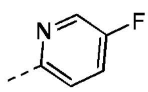 ,

and other variables are as defined in the present disclosure.

In some embodiments of the present disclosure, the structural moiety

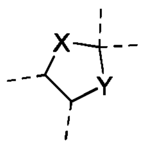

is selected from

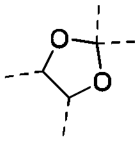, 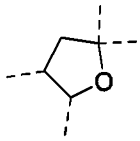, and 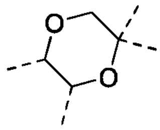,

and other variables are as defined in the present disclosure.

In some embodiments of the present disclosure, the structural moiety

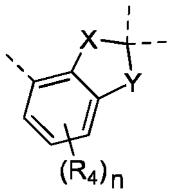

is selected from

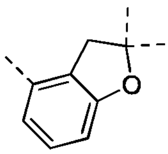, 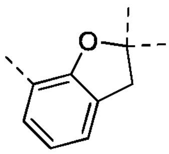, 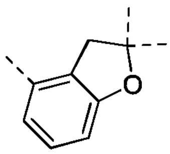, and 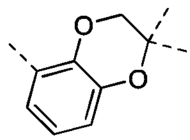,

and other variables are as defined in the present disclosure.

In some embodiments of the present disclosure, the ring B is selected from phenyl, and the phenyl is optionally substituted by 1, 2, or 3 Ri, the structural moiety

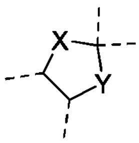

is selected from

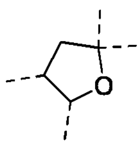 and 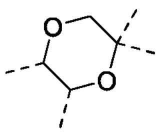,

and other variables are as defined in the present disclosure.

In some embodiments of the present disclosure, the ring B is selected from

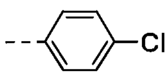 and 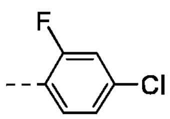,

the structural moiety

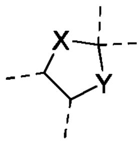

is selected from

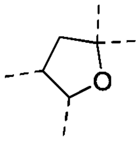 and 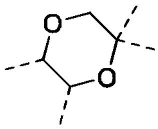 ,

and other variables are as defined in the present disclosure.

In some embodiments of the present disclosure, the structural moiety

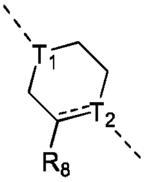 is

selected from

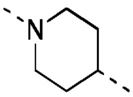, 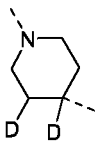, 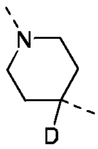, 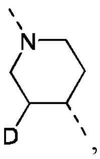, 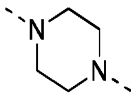, and 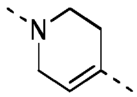,

and other variables are as defined in the present disclosure.

In some embodiments of the present disclosure, the structural moiety

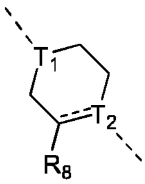

is selected from

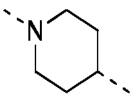, 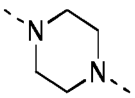, and 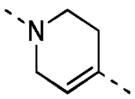,

and other variables are as defined in the present disclosure.

In some embodiments of the present disclosure, the structural moiety

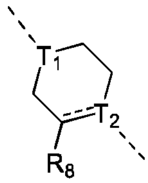

is selected from

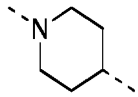,

and other variables are as defined in the present disclosure.

In some embodiments of the present disclosure, the structural moiety

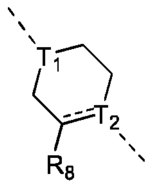

is selected from

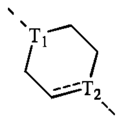,

and other variables are as defined in the present disclosure.

In some embodiments of the present disclosure, the structural moiety

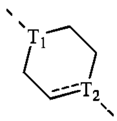

is selected from

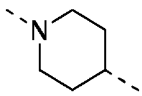, 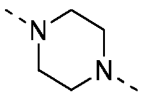, and 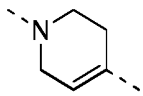,

and other variables are as defined in the present disclosure.

In some embodiments of the present disclosure, the structural moiety

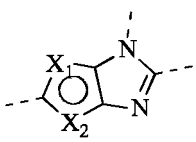

is selected from

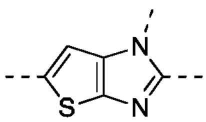, 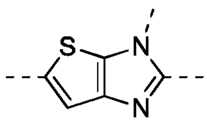, 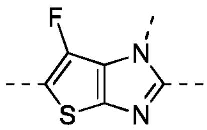, 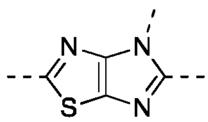,

and

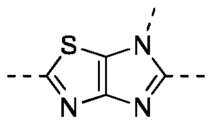,

and other variables are as defined in the present disclosure.

In some embodiments of the present disclosure, the structural moiety

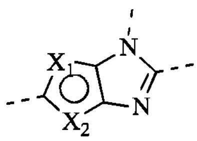

is selected from

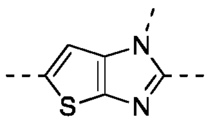,

and other variables are as defined in the present disclosure.

In some embodiments of the present disclosure, the compound or the pharmaceutically acceptable salt thereof is selected from:

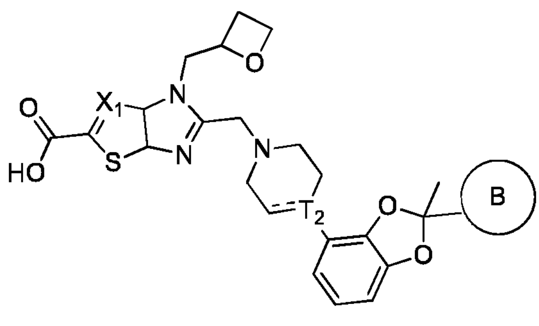

(I-1) ,

wherein

$\overline{---}$ is selected from a single bond and a double bond, and when $T_2$ is selected from N, $\overline{---}$ is selected from a single bond;
Xi, $T_2$, and ring B are as defined in the present disclosure.

In some embodiments of the present disclosure, the compound or the pharmaceutically acceptable salt thereof is selected from:

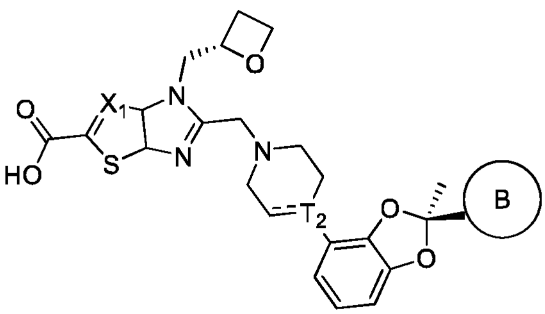

(I-1a) ,

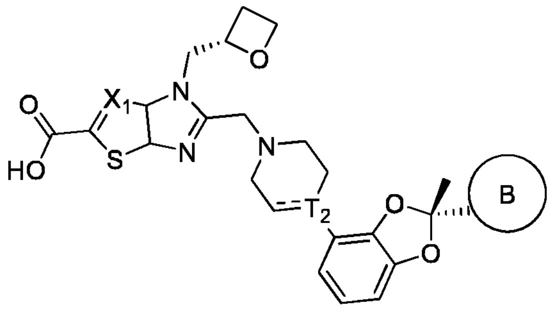

(I-1b) ,

wherein

$\overline{---}$ is selected from a single bond and a double bond, and when $T_2$ is selected from N, $\overline{---}$ is selected from a single bond;
$X_1$, $T_2$, and ring B are as defined in the present disclosure.

The present disclosure further provides a compound of formula (I) or a pharmaceutically acceptable salt thereof,

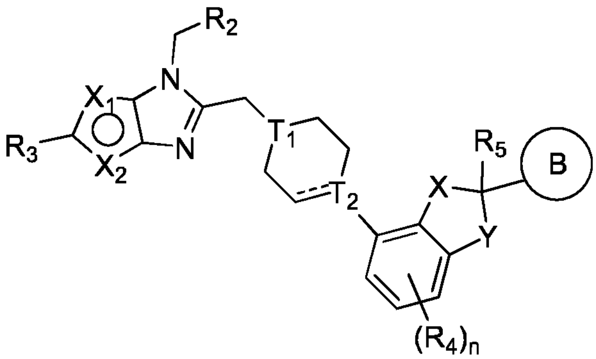

(I)

wherein

$\overline{---}$ is selected from a single bond and a double bond, and when $T_2$ is selected from N, $\overline{---}$ is selected from a single bond;
$T_1$ and $T_2$ are selected from N and CH;
X and Y are each independently selected from $CH_2$, $O-CH_2$, NH, O, and C(=O);
$X_1$ and $X_2$ are each independently selected from CH, N, O, and S, and the CH is optionally substituted by one F, Cl, Br, and $CH_3$;
ring B is selected from 5- to 6-membered heteroaryl, and the 5- to 6-membered heteroaryl is optionally substituted by 1, 2, or 3 Ri;
or, ring B is selected from phenyl, and the phenyl is optionally substituted by 1, 2, or 3 Ri, and in this case, X and Y are not simultaneously selected from O;
$R_1$ is selected from F, Cl, Br, I, OH, $NH_2$, CN, $CH_3$, and $OCH_3$;
$R_2$ is selected from

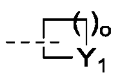 and 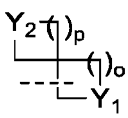 ,

and the

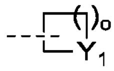 and 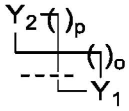

are optionally substituted by 1, 2, or 3 $R_a$;
$Y_1$ and $Y_2$ are each independently selected from $CH_2$, NH, and O;
o and p are each independently selected from 0, 1, 2, and 3;
$R_3$ is selected from -C(=O)-NH-$R_b$, -C(=O)-$R_b$, -C(=O)-NH-S(=O)$_2$-$R_b$, -S(=O)$_2$-NH-$R_b$, -S(=O)$_2$-$R_b$, -P(=O)($R_b$)$_2$, $C_{1-3}$ alkyl, tetrazolyl, isoxazolyl,

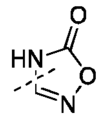, and 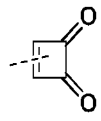,

and the $C_{1-3}$ alkyl, tetrazolyl, isoxazolyl,

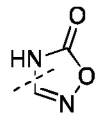, and 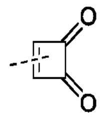

are optionally substituted by 1, 2, or 3 $R_b$;
$R_4$ is selected from F, Cl, Br, I, and $C_{1-3}$ alkyl;
$R_5$ is selected from H and $CH_3$;
n is selected from 0, 1, and 2;
each $R_a$ is independently selected from F, Cl, Br, and I;
each $R_b$ is independently selected from OH, CN, $C_{1-3}$ alkyl, $C_{1-3}$ alkoxy, $C_{1-3}$ alkylamino, and oxazolyl, and the $C_{1-3}$ alkyl, $C_{1-3}$ alkoxy, and oxazolyl are optionally substituted by 1, 2, or 3 R;
each R is independently selected from F, Cl, Br, and I.

The present disclosure further provides a compound of formula (I) or a pharmaceutically acceptable salt thereof,

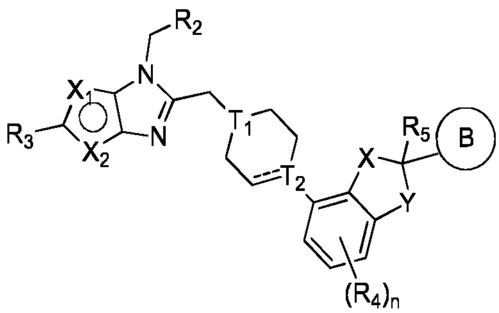

(I)

wherein

$\overline{---}$ is selected from a single bond and a double bond, and when $T_2$ is selected from N, $\overline{---}$ is selected from a single bond;
$T_1$ and $T_2$ are selected from N and CH;
X and Y are each independently selected from $CH_2$, NH, O, and C(=O);
$X_1$ and $X_2$ are each independently selected from CH, N, O, and S, and the CH is optionally substituted by one F, Cl, Br, and $CH_3$;
ring B is selected from 5- to 6-membered heteroaryl, and the 5- to 6-membered heteroaryl is optionally substituted by 1, 2, or 3 Ri;

$R_1$ is selected from F, Cl, Br, I, OH, $NH_2$, CN, $CH_3$, and $OCH_3$;
$R_2$ is selected from

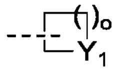 and 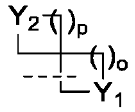,

and the

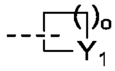 and 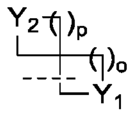

are optionally substituted by 1, 2, or 3 $R_a$;
$Y_1$ and $Y_2$ are each independently selected from $CH_2$, NH, and O;
o and p are each independently selected from 0, 1, 2, and 3;
$R_3$ is selected from -C(=O)-NH-$R_b$, -C(=O)-$R_b$, -C(=O)-NH-S(=O)$_2$-$R_b$, -S(=O)$_2$-NH-$R_b$, -S(=O)$_2$-$R_b$, -P(=O)($R_b$)$_2$, $C_{1-3}$ alkyl, tetrazolyl, isoxazolyl,

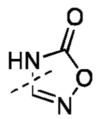, and 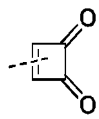,

and the $C_{1-3}$ alkyl, tetrazolyl, isoxazolyl,

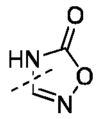, and 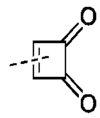

are optionally substituted by 1, 2, or 3 $R_b$;
$R_4$ is selected from F, Cl, Br, I, and $C_{1-3}$ alkyl;
$R_5$ is selected from H and $CH_3$;
n is selected from 0, 1, and 2;
each $R_a$ is independently selected from F, Cl, Br, and I;
each $R_b$ is independently selected from OH, CN, $C_{1-3}$ alkyl, $C_{1-3}$ alkoxy, $C_{1-3}$ alkylamino, and oxazolyl, and the $C_{1-3}$ alkyl, $C_{1-3}$ alkoxy, and oxazolyl are optionally substituted by 1, 2, or 3 R;
each R is independently selected from F, Cl, and Br.

The present disclosure further provides a compound of formula (II) or a pharmaceutically acceptable salt thereof,

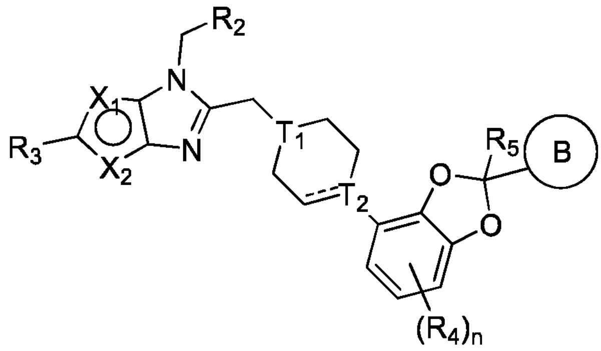

(II)

,

wherein

--- is selected from a single bond and a double bond, and when $T_2$ is selected from N, --- is selected from a single bond;
$T_1$ and $T_2$ are selected from N and CH;
$X_1$ and $X_2$ are each independently selected from CH, N, O, and S, and the CH is optionally substituted by one F, Cl, Br, and $CH_3$;
ring B is selected from 5- to 6-membered heteroaryl, and the 5- to 6-membered heteroaryl is optionally substituted by 1, 2, or 3 Ri;
$R_1$ is selected from F, Cl, Br, I, OH, $NH_2$, CN, $CH_3$, and $OCH_3$;
$R_2$ is selected from

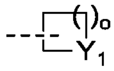 and 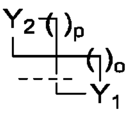,

and the

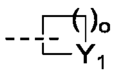 and 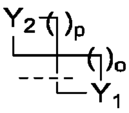

are optionally substituted by 1, 2, or 3 $R_a$;
$Y_1$ and $Y_2$ are each independently selected from $CH_2$, NH, and O;
o and p are each independently selected from 0, 1, 2, and 3;
$R_3$ is selected from $-C(=O)-NH-R_b$, $-C(=O)-R_b$, $-C(=O)-NH-S(=O)_2-R_b$, $-S(=O)_2-NH-R_b$, $-S(=O)_2-R_b$, $-P(=O)(R_b)_2$, $C_{1-3}$ alkyl, tetrazolyl, isoxazolyl,

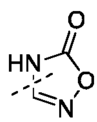, and 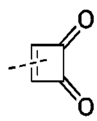,

and the $C_{1-3}$ alkyl, tetrazolyl, isoxazolyl,

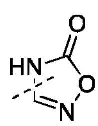, and 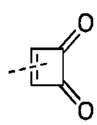

are optionally substituted by 1, 2, or 3 $R_b$;
$R_4$ is selected from F, Cl, Br, I, and $C_{1-3}$ alkyl;
$R_5$ is selected from H and $CH_3$;
n is selected from 0, 1, and 2;
each $R_a$ is independently selected from F, Cl, Br, and I;
each $R_b$ is independently selected from OH, CN, $C_{1-3}$ alkyl, $C_{1-3}$ alkoxy, $C_{1-3}$ alkylamino, and oxazolyl, and the $C_{1-3}$ alkyl, $C_{1-3}$ alkoxy, and oxazolyl are optionally substituted by 1, 2, or 3 R;
each R is independently selected from F, Cl, and Br.

There are still some embodiments of the present disclosure which are obtained by any combination of the above variables.

In some embodiments of the present disclosure, the compound or the pharmaceutically acceptable salt thereof is analyzed by SFC as two independent chromatographic peaks. The SFC analysis method is: column model: Chiralcel OJ-3, 50 × 4.6 mm I.D., 3 μm, mobile phase A is carbon dioxide, and mobile phase B is selected from methanol (0.1% isopropylamine), ethanol (0.2% ammonia water), or methanol.

In some embodiments of the present disclosure, the proportion of mobile phase B is 5% to 35%, 5% to 50%, or a gradient setting in the SFC analysis method. For example, the gradient setting is that the content of mobile phase B increases from 5% to 50% in 0.2 minutes and holds for 2 minutes, and then decreases from 50% to 5% in 2.2 minutes.

In Example 1 of the present disclosure, compound 001 has a retention time of 1.422 minutes by SFC analysis (chromatographic column: Chiralcel OJ-3, 50 × 4.6 mm I.D., 3 μm; mobile phase A: supercritical carbon dioxide, B: methanol (0.1% isopropylamine); gradient: the content of B increases from 5% to 50% in 0.2 minutes and holds for 2 minutes, and then decreases from 50% to 5% in 2.2 minutes).

In Example 2 of the present disclosure, compound 002 has a retention time of 1.264 minutes by SFC analysis (chromatographic column: Chiralcel OJ-3, 50 × 4.6 mm I.D., 3 μm; mobile phase A: supercritical carbon dioxide, B: methanol (0.1% isopropylamine); gradient: the content of B increases from 5% to 50% in 0.2 minutes and holds for 2 minutes, and then decreases from 50% to 5% in 2.2 minutes).

In some examples of the present disclosure, compounds **002** and **002'** were analyzed by chiral HPLC (chromatographic column: Chiralpak IG-U, 50 × 3.0 mm I.D., 1.6 μm; mobile phase A: n-hexane, B: ethanol (0.1% trifluoroacetic acid); gradient: mobile phase A:B = 85:15), showing that compound **002** has a retention time of 2.931 minutes; compound **002'** has a retention time of 4.354 minutes.

In Example 3 of the present disclosure, compound 003 has a retention time of 1.856 minutes by SFC analysis (chromatographic column: Chiralcel OJ-3, 50 × 4.6 mm I.D., 3 μm; mobile phase A: supercritical carbon dioxide, B: methanol (0.1% isopropylamine); gradient: the content of B increases from 5% to 50% in 0.2 minutes and holds for 2 minutes, and then decreases from 50% to 5% in 2.2 minutes).

In Example 8 of the present disclosure, compound 008 has a retention time of 1.215 minutes by SFC analysis (chromatographic column: Chiralcel OJ-3, 50 × 4.6 mm I.D., 3 μm; mobile phase: A: carbon dioxide, B: methanol (0.1% isopropylamine); gradient: (B%): 5% to 50%)).

In Example 9 of the present disclosure, compound 009 has a retention time of 1.373 minutes by SFC analysis (chromatographic column: Chiralcel OJ-3, 50 × 4.6 mm I.D., 3 μm; mobile phase: A: carbon dioxide, B: methanol (0.1% isopropanol); gradient: (B%): 5% to 50%)).

In Example 10 of the present disclosure, compound 010 has a retention time of 1.264 minutes by SFC analysis (chromatographic column: Chiralpak AD-3, 50 × 4.6 mm I.D., 3 μm; mobile phase: phase A was supercritical carbon dioxide, phase B was methanol (0.1% isopropanol); gradient: the content of B increases from 5% to 50% in 0.2 minutes and holds for 2 minutes, and then decreases from 50% to 5% in 2.2 minutes).

In Example 12 of the present disclosure, compound 012 has a retention time of 1.142 minutes by SFC analysis (chromatographic column: Chiralpak AD-3, 50 × 4.6 mm I.D., 3 μm; mobile phase: A: carbon dioxide, B: ethanol (0.1% isopropanol); gradient: the content of B increases from 5% to 50% in 0.2 minutes and holds for 2 minutes, and then decreases from 50% to 5% in 2.2 minutes).

The present disclosure also provides a compound of the following formula or a pharmaceutically acceptable salt thereof,

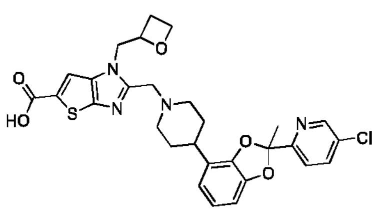 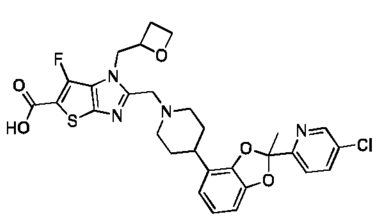 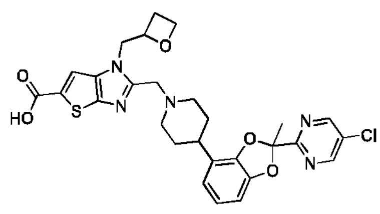

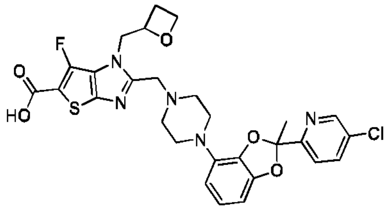
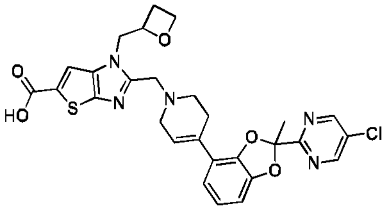
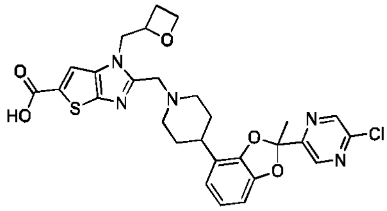
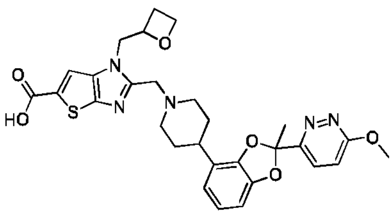
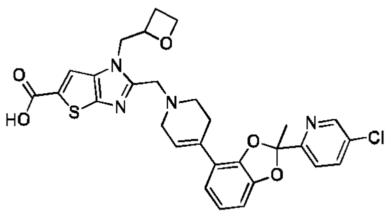
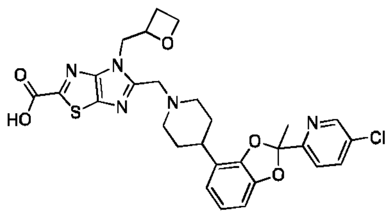
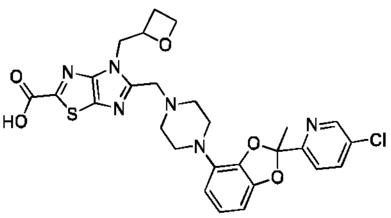
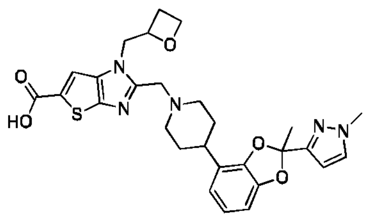
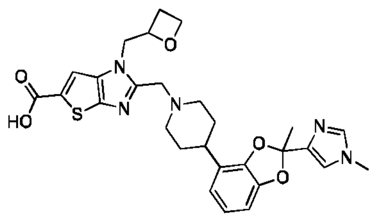
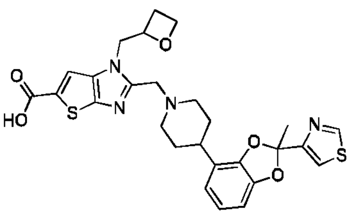
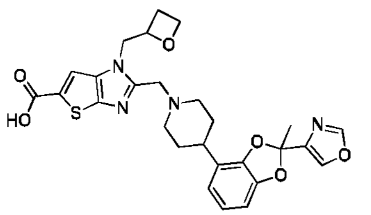
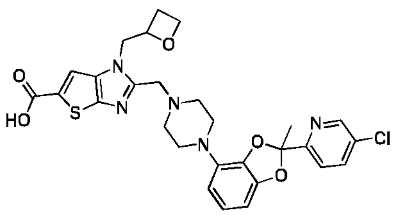
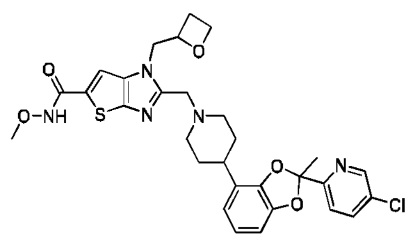
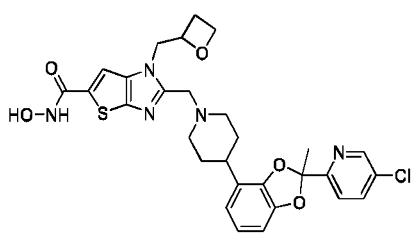
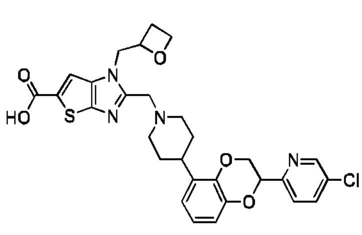
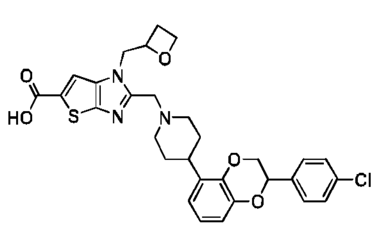
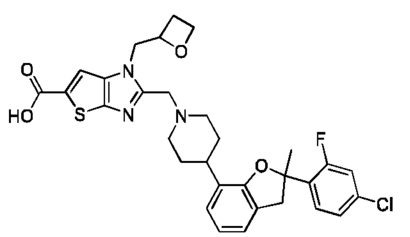
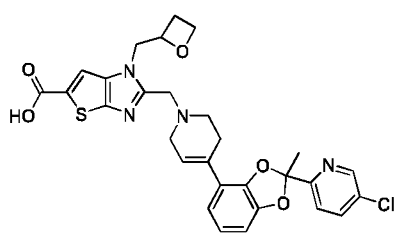
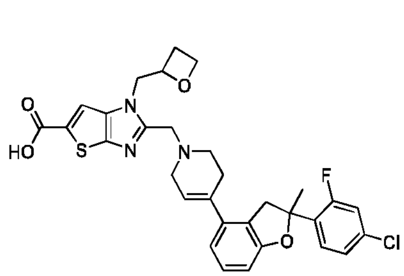
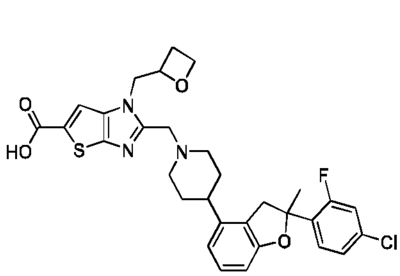
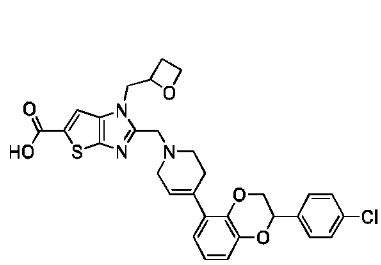

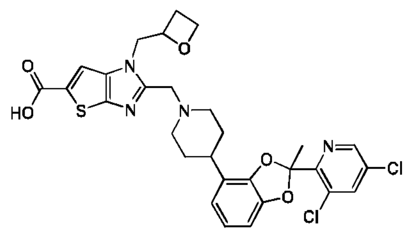
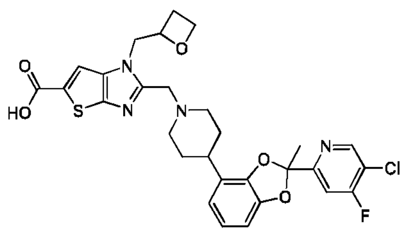
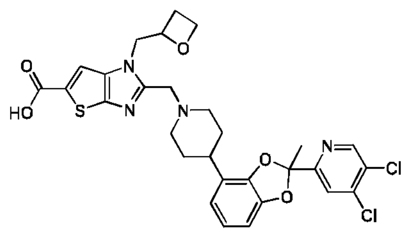

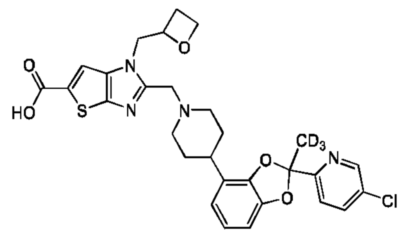
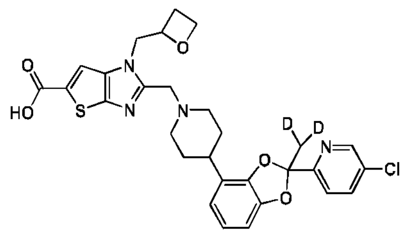
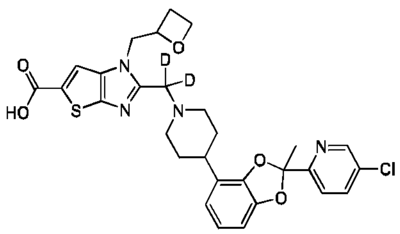

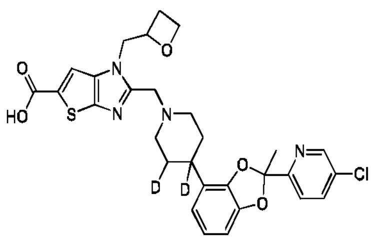
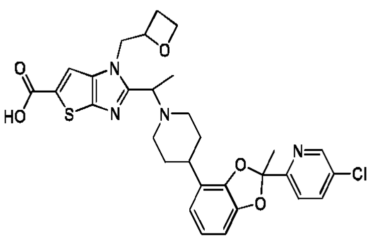
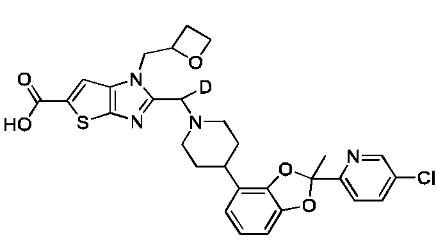
.

In some embodiments of the present disclosure, the compound or the pharmaceutically acceptable salt thereof is selected from:

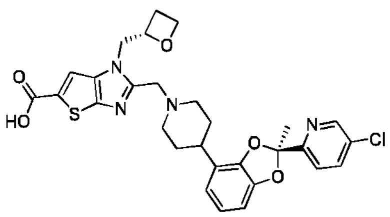
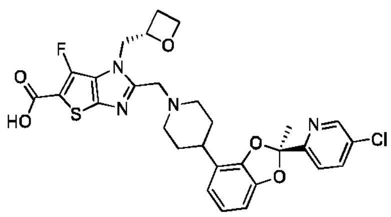
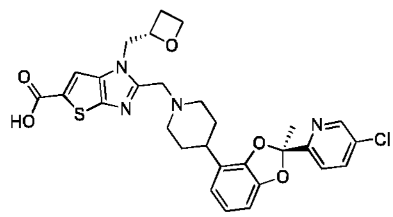

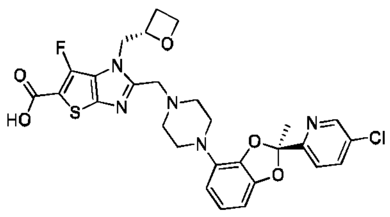
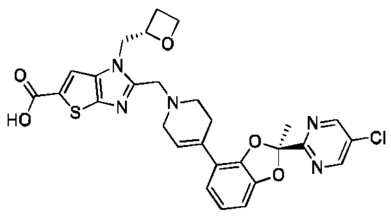
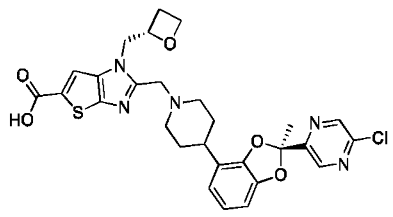

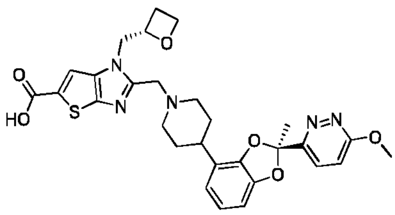
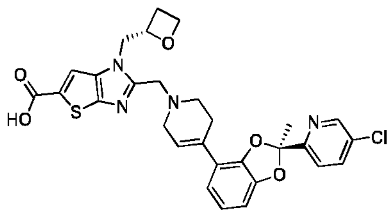
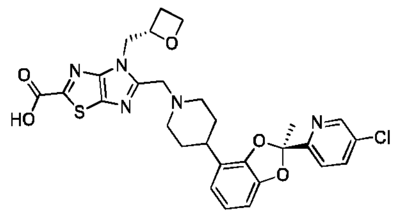

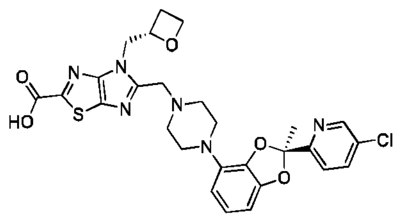
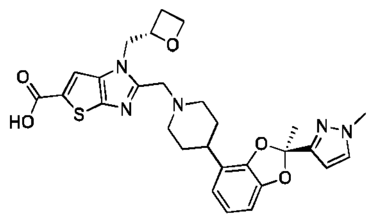
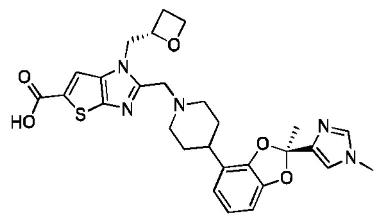

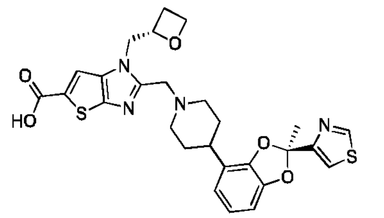
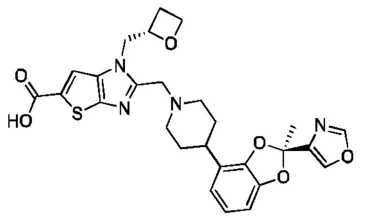
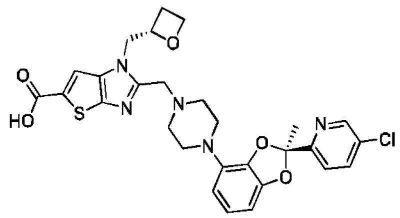

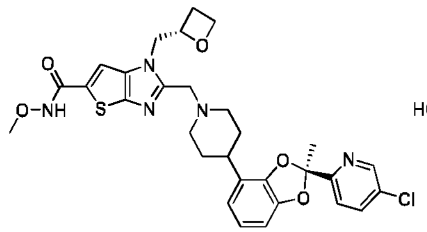
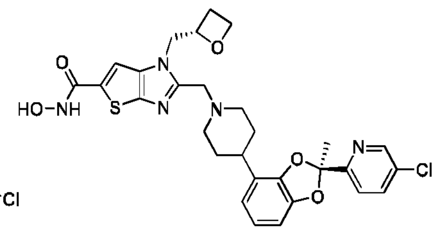
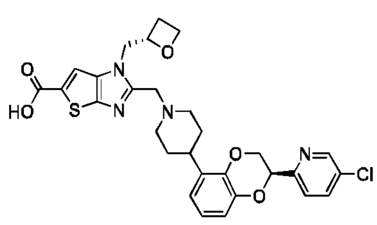

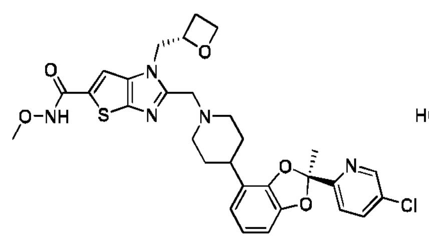
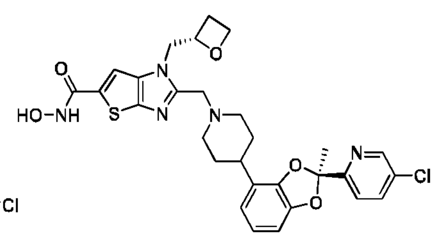
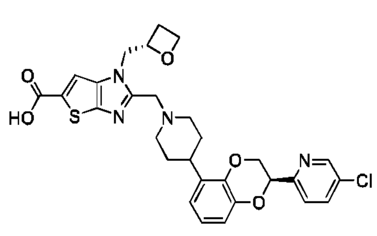

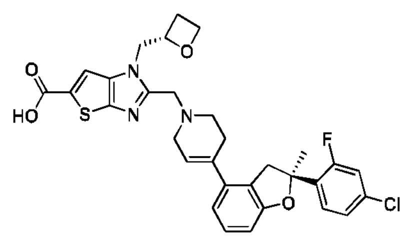
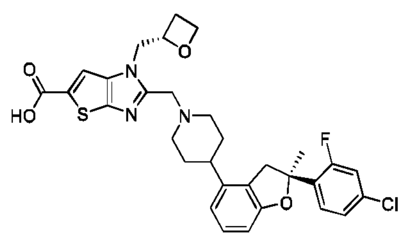
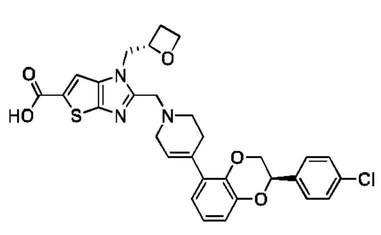

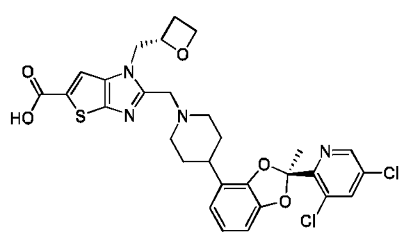
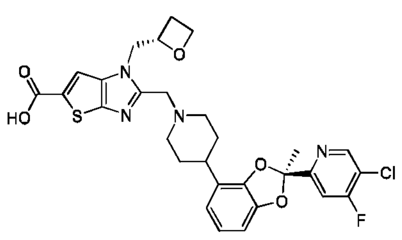
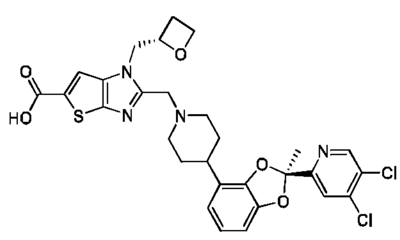

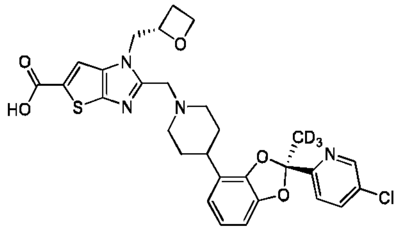
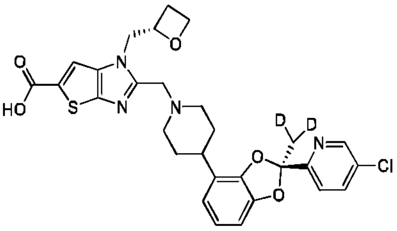
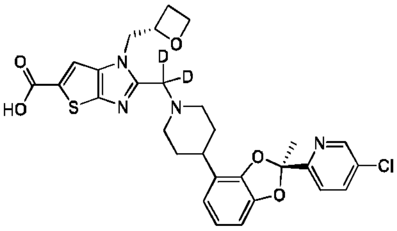

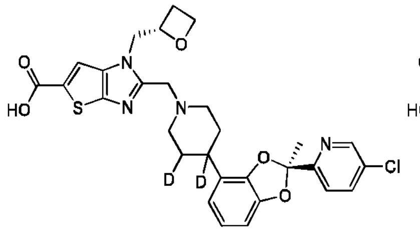
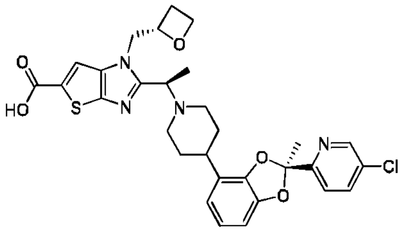
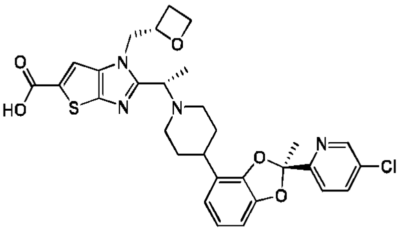

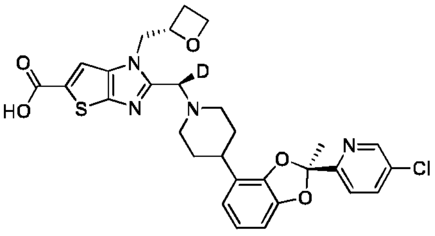
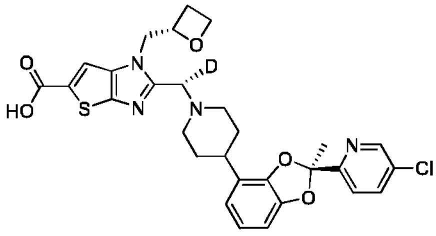

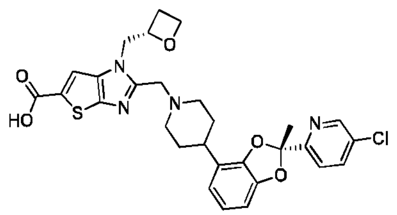
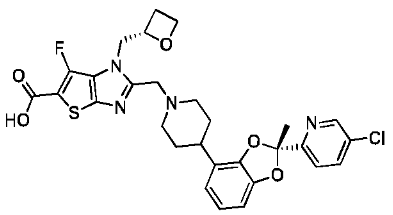
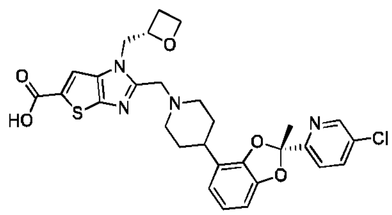

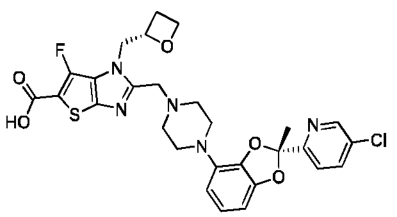
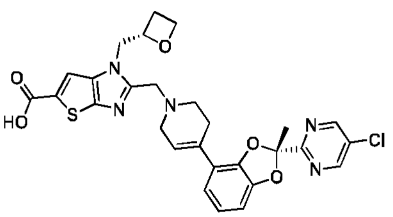
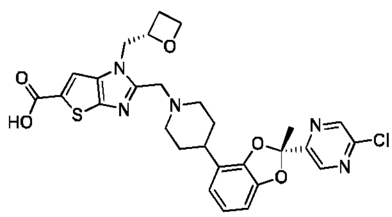

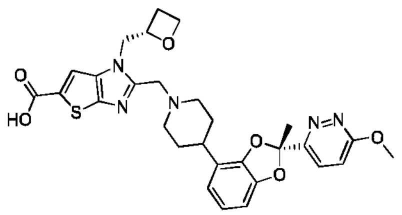
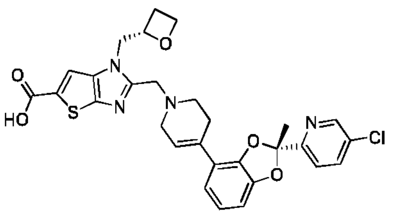
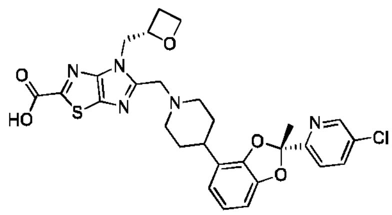

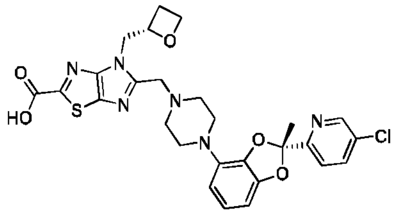
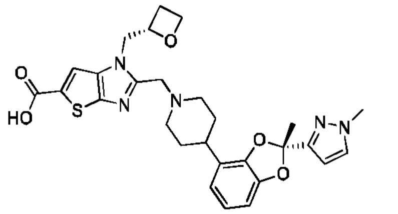
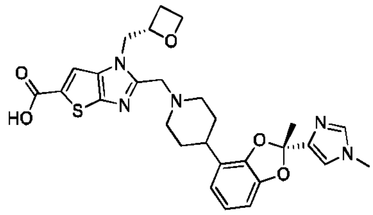

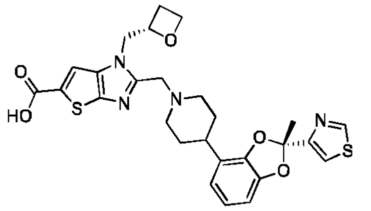
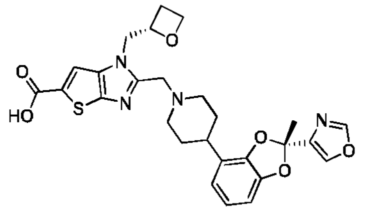
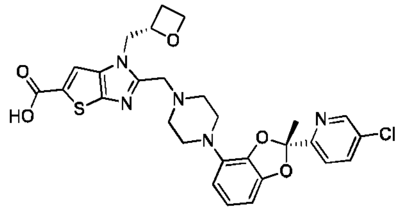

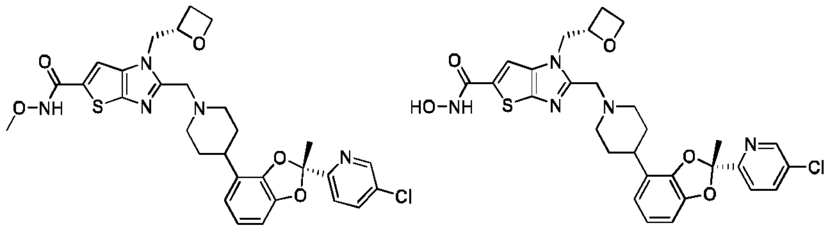
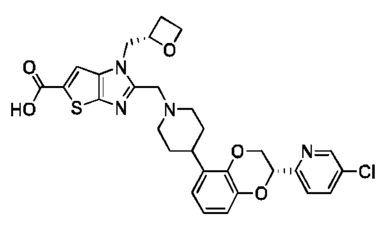

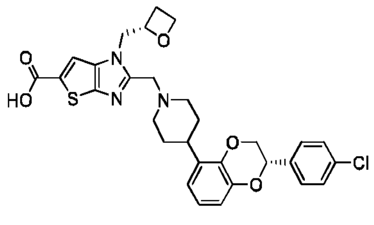
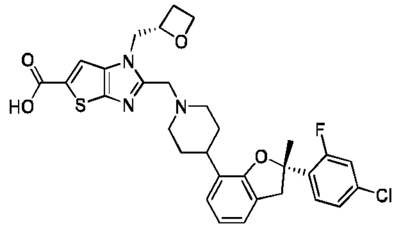
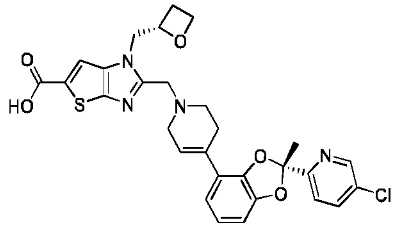

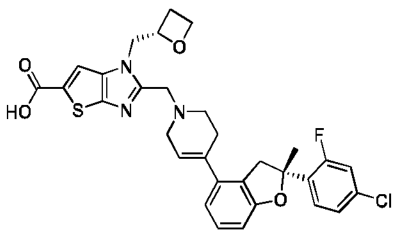
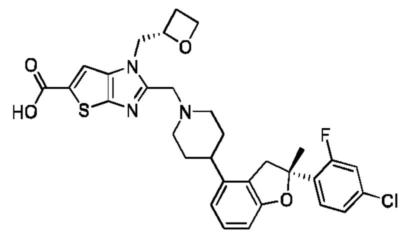
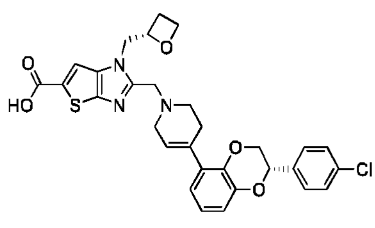

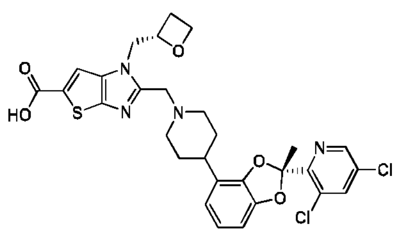
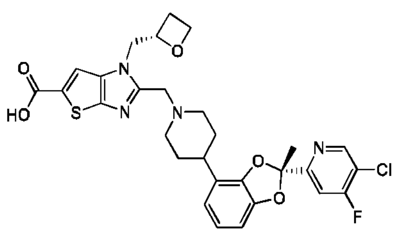
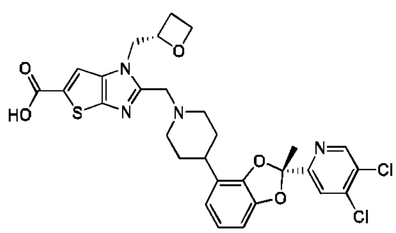

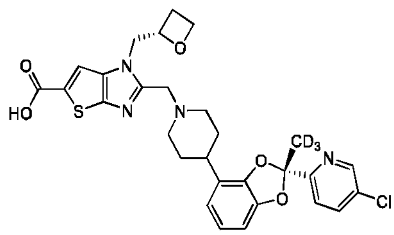
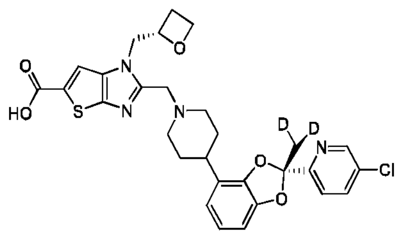
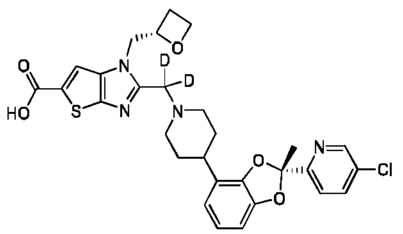

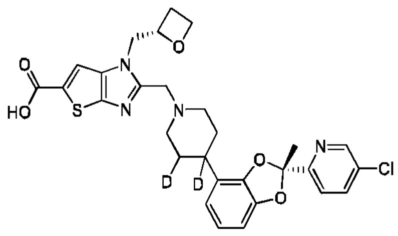
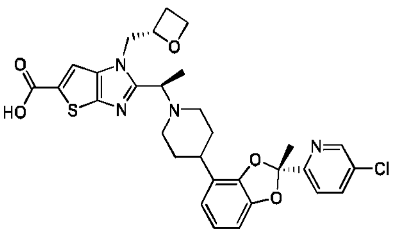
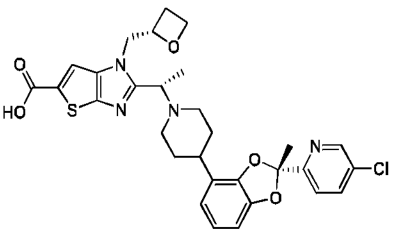
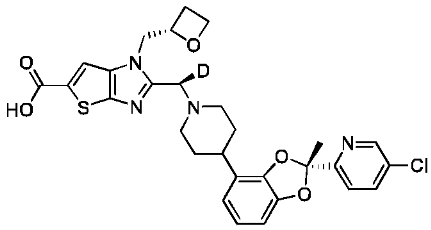
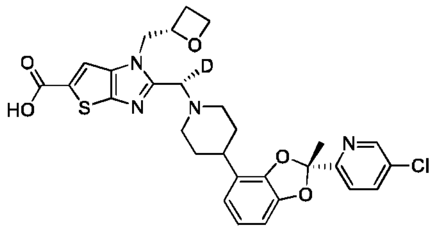
.

The present disclosure further provides a use of the compound or the pharmaceutically acceptable salt thereof in the manufacture of a medicament for treating diabetes.

**Definition and description**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting such compounds with a sufficient amount of acid in a pure solution or a suitable inert solvent. Certain specific compounds of the present disclosure contain both basic and acidic functional groups, and thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "($\pm$)" refers to racemic.

Unless otherwise specified, the absolute configuration of a stereogenic center is represented by a wedged solid

bond ( ) and a wedged dashed bond ( ), and the relative configuration of a stereogenic center is represented by a straight solid bond ( ) and a straight dashed bond ( ), a wave line ( ) is used to represent a wedged solid bond ( ) or a wedged dashed bond ( ), or the wave line ( ) is used to represent a straight solid bond ( )or a straight dashed bond ( ).

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound and its substituent are represented by

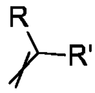
,

this refers to the (*Z*) isomer, (*E*) isomer or a mixture of two isomers of the compound.

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can

be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in

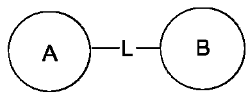

is -M-W-, then -M-W- can link ring A and ring B to form

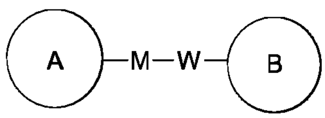

in the direction same as left-to-right reading order, and form

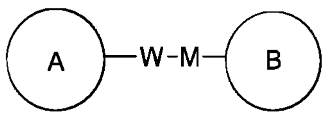

in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond linking the site to other groups can be represented by a straight solid bond (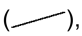), straight dashed bond (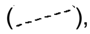), or wave line (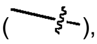), respectively, wherein the straight dashed bond (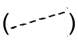) or wave line (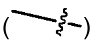) can represent single, double, or triple bonds linked to other groups. Correspondingly, the site will be reduced by 1, 2, or 3 H to become a monovalent, bivalent, or trivalent group. For example, the straight solid bond in $-OCH_3$ means that it is linked to other groups through the oxygen atom in the group; the straight dashed bond in

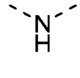

means that it is linked to other groups through the two ends of the nitrogen atom in the group; the wave lines in

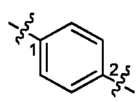

means that the phenyl group is linked to other groups through carbon atoms at position 1 and position 2.

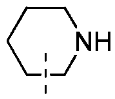

means that it can be linked to other groups through any linkable sites on the piperidinyl by one chemical bond, including at least four types of linkage, including

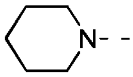, 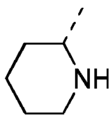, 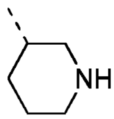, 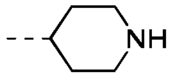.

Even though the H atom is drawn on the -N-,

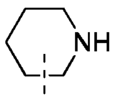

still includes the linkage of

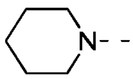,

merely when one chemical bond was connected, the H of this site will be reduced by one to the corresponding monovalent piperidinyl.

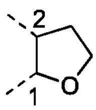

can be linked to other groups through the carbon atoms at positions 1 and 2 of the tetrahydrofuran group, either by a single bond, such as

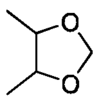,

or by a double bond, such as

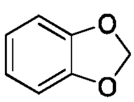.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ and $C_{2-3}$ alkyl, etc.; it can be monovalent (such as methyl), divalent (such as methylene), or multivalent (such as methine). Examples of $C_{1-3}$ alkyl include, but are not limited to, methyl (Me), ethyl (Et), propyl (including *n*-propyl and isopropyl), etc.

Unless otherwise specified, the term "$C_{1-3}$ alkoxy" refers to an alkyl group containing 1 to 3 carbon atoms that are connected to the rest of the molecule through an oxygen atom. The $C_{1-3}$ alkoxy includes $C_{1-2}$, $C_{2-3}$, $C_3$, $C_2$ alkoxy,

etc. Examples of $C_{1-3}$ alkoxy include, but are not limited to, methoxy, ethoxy, propoxy (including *n*-propoxy and isopropoxy), etc.

Unless otherwise specified, the term "$C_{1-3}$ alkylamino" refers to an alkyl group containing 1 to 3 carbon atoms that are connected to the rest of the molecule through an amino group. The $C_{1-3}$ alkylamino includes $C_{1-2}$, $C_3$, $C_2$ alkylamino, etc. Examples of $C_{1-3}$ alkylamino include, but are not limited to, $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, etc.

Unless otherwise specified, the terms "5- to 6-membered heteroaryl ring" and "5- to 6- membered heteroaryl" are used interchangeably. The term "5- to 6-membered heteroaryl" refers to a monocyclic group consisting of 5 to 6 ring atoms with a conjugated π-electron system, in which 1, 2, 3, or 4 ring atoms are heteroatoms independently selected from O, S, and N, and the rest are carbon atoms, Here, the nitrogen atom is optionally quaternized, and nitrogen and sulfur heteroatoms can be optionally oxidized (i.e., NO and $S(O)_p$, wherein p is 1 or 2). The 5- to 6-membered heteroaryl can be linked to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including *N*-pyrrolyl, 2-pyrrolyl, and 3-pyrrolyl, etc.), pyrazolyl (including 2-pyrazolyl and 3-pyrazolyl, etc.), imidazolyl (including *N*-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (including 1*H*-1,2,3-triazolyl, 2*H*-1,2,3-triazolyl, 1*H*-1,2,4-triazolyl, and 4*H*-1,2,4-triazolyl, etc.), tetrazolyl, isoxazolyl (including 3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyll and 3-thienyl, etc.), pyridyl (including 2-pyridyl, 3-pyridyl, and 4-pyridyl, etc.), pyrazinyl, pyrimidinyl (including 2-pyrimidinyl, 4-pyrimidinyl, etc.).

Unless otherwise specified, the term "aromatic ring" means a cyclic group having a conjugated π-electron system, the atoms of which are covered by a delocalized π-electron cloud. In the structural formula, it can be written in the form of alternating single and double bonds, or

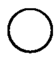

can be used to represent the delocalized π-electron cloud when the rules of atomic valence and covalent bonding are met. For example, the structures represented by the structural formulas

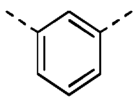
,
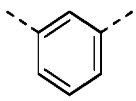
, and
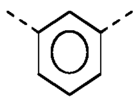

are all the same; the structures represented by the structural formulas

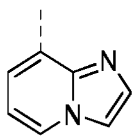
,
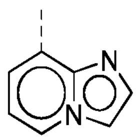
,
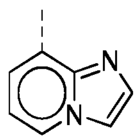
, and
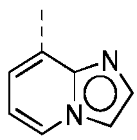

are all the same.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and any range from n to n+m is also included, for example $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, etc.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: φ/ω scan, and after collecting the relevant data, the crystal structure can be further analyzed by

the direct method (Shelxs97).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The solvent used in the present disclosure is commercially available. The following abbreviations are used in the present disclosure: aq represents water; *eq* represents equivalent, equal; DCM represents dichloromethane; PE represents petroleum ether; DMF represents *N,N*-dimethylformamide; DMSO represents dimethyl sulfoxide; EtOAc and EA both represent ethyl acetate; EtOH represents ethanol; MeOH represents methanol; BOC represents tert-butyloxycarbonyl, which is an amine protecting group; HOAc represents acetic acid; RT and Rt both represent retention time; O/N represents overnight; hr represents hour; THF represents tetrahydrofuran; BoczO represents di-*tert*-butyl dicarbonate; TFA represents trifluoroacetic acid; DIPEA represents diisopropylethylamine; Xantphos represents 4,5-bis(diphenylphosphino)-9,9-dimethyloxanthene; BINAP represents 1,1'-binaphthyl-2,2'-bisdiphenylphosphine; SEMCl represents 2-(trimethylsilyl)ethoxymethyl chloride; TBDPSCl represents *tert*-butyldiphenylchlorosilane; NBS represents *N*-bromosuccinimide; i-PrMgCl represents isopropylmagnesium chloride; $N_2$ represents nitrogen; $NaBH_4$ represents sodium borohydride; DIAD represents diisopropyl azodicarboxylate; and $Pd(PPh_3)_4$ represents tetrakis(triphenylphosphine)palladium.

The compounds of the present disclosure are named according to the conventional naming principles in the art or by ChemDraw® software, and the commercially available compounds use the supplier catalog names.

**Technical effect**

The compound of the present disclosure exhibits excellent agonistic activity to GLP-1 receptor, has low risk of time-dependent inhibition of human liver microsomal cytochrome P450 isoenzyme 2C19, and metabolizes slowly in hepatocytes of various species, and show little species difference. The compound of the present disclosure has high oral exposure, long half-life, high bioavailability, and good *in vivo* pharmacokinetic properties.

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the spirit and scope of the present disclosure.

**Reference example 1: Fragment B-1**

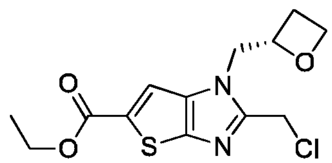

**B-1**

Synthetic route:

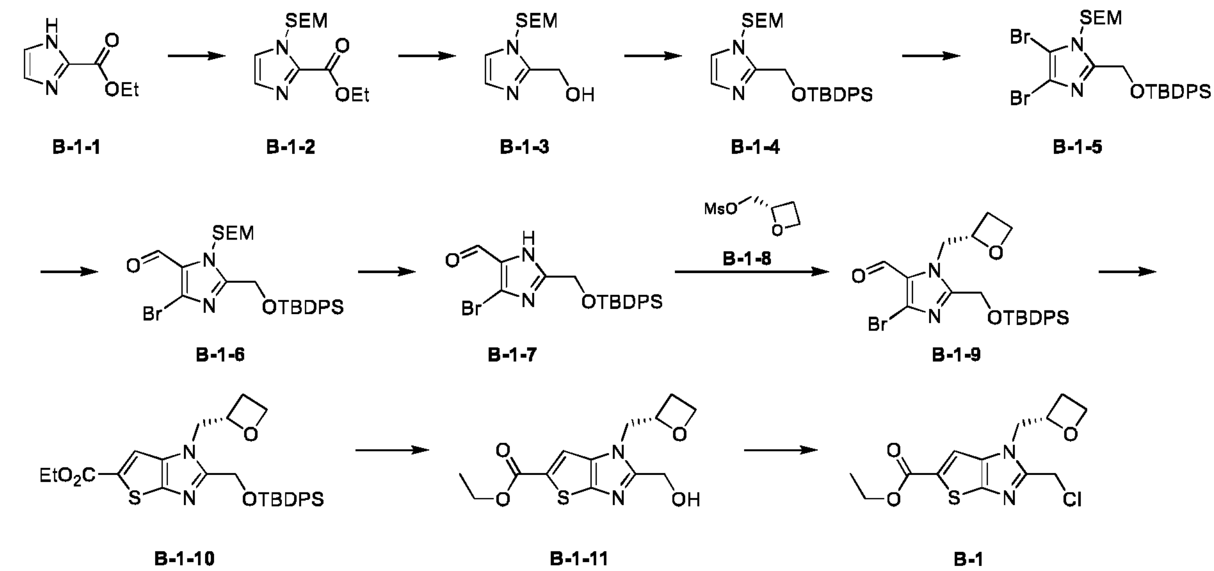

B-1-1 B-1-2 B-1-3 B-1-4 B-1-5

B-1-8

B-1-6 B-1-7 B-1-9

B-1-10 B-1-11 B-1

Step 1: Synthesis of compound **B-1-2**

**B-1-1** (12.9 g, 92.05 mmol, 1 *eq*) was dissolved in THF (150.0 mL), then sodium hydride (5.52 g, 138.08 mmol, a content of 60%, 1.5 *eq*) was slowly added thereto in batches at 0°C under $N_2$ atmosphere, and the mixture was stirred to homogeneity, and then SEMCl (23.55 g, 141.25 mmol, 25 mL, 1.53 *eq*) was added thereto. The reaction system was gradually warmed to 20°C and stirred for 16 hours. The reaction mixture was slowly poured into ice water (300.0 mL) and stirred thoroughly, extracted with ethyl acetate (150.0 mL * 3), and the organic phase was washed with saturated brine (100 mL) and dried over anhydrous sodium sulfate. The resulting mixture was filtered, and the filtrate was concentrated to obtain a crude product, which was purified by silica gel column chromatography (petroleum ether: ethyl acetate = 1:0 to 1:1) to obtain compound **B-1-2.** LCMS: m/z=271.1 $[M+1]^+$.

Step 2: Synthesis of compound **B-1-3**

Lithium aluminium hydride (712.91 mg, 18.78 mmol, 1.5 *eq*) was dissolved in THF (60 mL) and stirred thoroughly, and then a solution of **B-1-2** (3.39 g, 12.52 mmol, 1 *eq*) in THF (10 mL) was slowly added thereto at 0°C under $N_2$ atmosphere. The reaction mixture was warmed to 20°C and stirred for 45 minutes. The reaction mixture was cooled to 0°C, then 1 mL of water and 1 mL of 15% sodium hydroxide solution were sequentially added thereto, and the reaction mixture was warmed to 20°C and stirred for 15 minutes, then added with a small amount of anhydrous magnesium sulfate, stirred for another 15 minutes, and filtered. The resulting filtrate was washed with saturated brine (350 mL), the aqueous phase was extracted with ethyl acetate (50 mL * 3), the resulting organic phase was washed with saturated brine (50 mL), dried over anhydrous sodium sulfate, filtered, and the resulting filtrate was concentrated under reduced pressure to obtain **B-1-3.** $^1H$ NMR (400 MHz, $CDCl_3$) $\delta$ ppm 6.95 - 7.00 (m, 1H), 6.92 (s, 1H), 5.37 (s, 2H), 4.71 (s, 2H), 3.49-3.56 (m, 2H), 0.88-0.94 (m, 2H), 0.02-0.05 (m, 9H). LCMS: m/z=229.1 $[M+1]^+$.

Step 3: Synthesis of compound **B-1-4**

**B-1-3** (2.4 g, 10.51 mmol, 1 *eq*) was dissolved in DMF (30 mL) at 20°C and stirred thoroughly, and then TBDPSCl (1.3 g, 13.63 mmol, 1.3 *eq*) and imidazole (1.8 g, 26.44 mmol, 2.52 *eq*) were sequentially added thereto. The reaction mixture was stirred for 16 hours under nitrogen atmosphere. The reaction mixture was quenched by pouring into water (50 mL) and extracted with ethyl acetate (40 mL * 3). The resulting organic phase was washed with saturated brine (40 mL) and dried over anhydrous sodium sulfate. The resulting mixture was filtered, and the filtrate was concentrated under reduced pressure to obtain a crude product, which was purified by column chromatography (petroleum ether: ethyl acetate = 1:0 to 3:1) to obtain compound **B-1-4.** $^1H$ NMR (400 MHz, $CDCl_3$) $\delta$ ppm 7.67 - 7.72 (m, 4H), 7.37 - 7.46 (m, 6H), 7.00-7.03 (m, 1H), 6.98 (d, *J*=1.25 Hz, 1H), 5.41 (s, 2H), 4.84 (s, 2H), 3.42-3.47 (m, 2H), 1.06 (s, 9H), 0.85-0.90 (m, 2H), 0.03 (s, 9H). LCMS: m/z=467.2 $[M+1]^+$.

Step 4: Synthesis of compound **B-1-5**

**B-1-4** (10.9 g, 223.42 mmol, 1 *eq*) was dissolved in THF (120 mL), then cooled to 0°C, NBS (15 g, 84.28 mmol, 3.60 *eq*) was added thereto in batches, and then the reaction mixture was warmed to 20°C and stirred for 16 hours. The reaction mixture was slowly poured into water (150 mL), stirred thoroughly, and extracted with ethyl acetate (100 mL * 2). The organic phase was washed with saturated brine (200 mL), dried over anhydrous sodium sulfate, filtered, and the filtrate was concentrated under reduced pressure to obtain the crude product. The crude product was purified by neutral alumina column chromatography (petroleum ether: ethyl acetate = 1:0 to 20:1) to obtain compound **B-1-5.** LCMS: m/z = 622.9 $[M+1]^+$.

Step 5: Synthesis of compound **B-1-6**

**B-1-5** (2.4 g, 3.79 mmol, 1 *eq*) was dissolved in THF (24 mL) and cooled to -70°C under nitrogen atmosphere. *i*-PrMgCl (2 M, 2.09 mL, 1.1 *eq*) was added dropwise thereto and stirred for 1.5 hours. Then DMF (55.47 g, 758.94 mmol, 58.39 mL, 200 *eq)* was added dropwise thereto, and the reaction mixture was warmed to 20°C and stirred for 0.5 hours. The reaction mixture was slowly poured into a saturated ammonium chloride solution (200 mL) to quench, and extracted with ethyl acetate (200 mL * 3). The organic phase was washed with saturated brine (200 mL), dried over anhydrous sodium sulfate, and filtered. The resulting filtrate was concentrated under reduced pressure to obtain the crude product. The crude product was purified by column chromatography (petroleum ether: ethyl acetate = 1:0 to 10:1) to obtain compound **B-1-6.** $^1H$ NMR (400 MHz, $CDCl_3$) $\delta$ ppm 9.76(s,1H), 7.66 (dd, *J*=7.91, 1.38 Hz, 4H), 7.36 - 7.47 (m, 6H), 5.85 (s, 2 H), 4.86 (s, 2H), 3.48-3.54 (m, 2H), 1.07 (s, 9H), 0.84-0.89 (m, 2H), 0.02-0.06 (m, 9H). LCMS: m/z = 573.0 $[M+1]^+$.

Step 6: Synthesis of compound **B-1-7**

**B-1-6** (1.1 g, 1.97 mmol, 1 *eq*) was dissolved in dichloromethane (5 mL), and then TFA (8.98 g, 78.79 mmol, 5.83 mL, 40 *eq*) was added dropwise thereto and stirred at 20°C for 16 hours. The reaction mixture was poured into a saturated sodium bicarbonate solution (70 mL) to adjust the pH of the reaction mixture to 5-6, and extracted with ethyl acetate (40 mL * 3). The organic phase was washed with water (30 mL) and saturated brine (30 mL), dried over anhydrous sodium sulfate, and filtered. The resulting filtrate was concentrated to obtain a crude product, which was purified by column chromatography (petroleum ether: ethyl acetate = 1:0 to 10:1) to obtain compound **B-1-7.** $^1H$ NMR (400 MHz, $CDCl_3$) $\delta$ ppm 9.65 (s, 1 H), 7.63 (dd, *J*=7.94, 1.44 Hz, 4H), 7.40-7.50 (m, 6H), 4.83 (s, 2H), 1.13 (s, 9H). LCMS: m/z = 442.9 $[M+H]^+$.

Step 7: Synthesis of compound **B-1-9**

**B-1-7** (0.02 g, 45.11 μmol, 1 *eq*) was dissolved in acetonitrile (0.5 mL), then cesium carbonate (16.17 mg, 49.62 μmol, 1.1 *eq*) and **B-1-8** (11.24 mg, 67.66 μmol, 1.5 *eq*) were sequentially added thereto. The reaction mixture was heated to 80°C, stirred for 16 hours, and concentrated to obtain the crude product, which was purified by a preparative thin-layer chromatography silica gel plate (petroleum ether: ethyl acetate = 5:1) to obtain **B-1-9.** $^1H$ NMR (400 MHz, $CDCl_3$) $\delta$ ppm 9.70 (s, 1 H), 7.67 (ddd, *J*=12.38, 7.82, 1.31 Hz, 4 H), 7.38-7.48 (m, 6H), 4.84-5.00 (m, 3H), 4.65- 4.75 (m,1 H), 4.46-4.65 (m, 2H), 4.19 (dt, *J*=9.07, 6.10 Hz, 1H), 2.62-2.75 (m, 1 H), 2.17-2.29 (m, 1 H), 1.07-1.13 (m, 9 H). LCMS: m/z = 512.9$[M+H]^+$.

Step 8: Synthesis of compound **B-1-10**

Sodium ethoxide (13 mg, 191.04 μmol, 3.77 *eq*) was dissolved in ethanol (0.5 mL), then ethyl thioglycolate (183.07 μmol, 20 μL, 3.62 *eq*) and **B-1-9** (26 mg, 50.63 μmol, 1 *eq*) were sequentially added thereto, and the reaction mixture was heated to 80°C and stirred for 20 hours. The pH of the reaction mixture was adjusted to 2-3 with 1 M hydrochloric acid, the aqueous phase was extracted with ethyl acetate (5 mL * 3), and the organic phase was washed with saturated brine and then concentrated to obtain a crude product, which was purified by a preparative thin-layer chromatography silica gel plate (petroleum ether: ethyl acetate = 5:1) to obtain **B-1-10.** $^1H$ NMR (400 MHz, $CDCl_3$) $\delta$ ppm 7.74 (s, 1 H), 7.63 - 7.70 (m, 4 H), 7.37-7.47 (m, 6H), 4.95 (s,2H), 4.03-4.71 (m,7H), 2.57-2.71 (m,1H), 2.28-2.39 (m, 1H), 1.39 (t, *J*=7.15 Hz, 3H), 1.08 (s, 9H). LCMS: m/z = 535.0 $[M+H]^+$.

Step 9: Synthesis of compound **B-1-11**

**B-1-10** (2 g, 3.15 mmol, purity of 84.3%, 1 *eq*) was added to THF (20 mL) in a dry reaction flask, then triethylamine trihydrofluoride (15.77 mmol, 2.57 mL, 5 *eq*) was added thereto, and the reaction mixture was stirred for 10 hours at

20°C. The reaction mixture was diluted with water (30 mL), and extracted with ethyl acetate (30 mL * 3). After the phase separation, the organic phase was collected. The resulting organic phase was sequentially washed with saturated sodium bicarbonate (30 mL) and saturated brine (30 mL * 3), dried over anhydrous sodium sulfate, filtered, and the resulting filtrate was concentrated under reduced pressure. Methyl *tert*-butyl ether (10 mL) and ethyl acetate (1 mL) were added thereto, and the resulting mixture was stirred and filtered. The filter cake was washed again with methyl *tert*-butyl ether (10 mL), and the resulting filter cake was concentrated under reduced pressure to obtain **B-1-11.** LCMS: m/z = 297.1 $[M+1]^+$.

Step 10: Synthesis of compound **B-1**

**B-1-11** (0.8 g, 2.70 mmol, 1 *eq*), dichloromethane (10 mL), and triethylamine (819.51 mg, 8.10 mmol, 1.13 mL, 3 *eq*) were added to a dry reaction flask, the reaction system was replaced with nitrogen and cooled to 0°C, and methylsulfonyl chloride (463.86 mg, 4.05 mmol, 313.42 μL, 1.5 *eq*) was added thereto and stirred for 3 hours at 20°C. The reaction mixture was quenched by pouring into water (20 mL), and extracted with dichloromethane (20 mL * 3). The organic phase was collected after phase separation, sequentially washed with saturated brine (20 mL), dried over anhydrous sodium sulfate, and filtered. The resulting filtrate was concentrated under reduced pressure to obtain the crude product. The crude product was purified by column chromatography (petroleum ether: ethyl acetate = 1:0 to 3:1) to obtain **B-1.** LCMS: m/z = 315.1 $[M+1]^+$.

**Reference example 2: Fragment B-2**

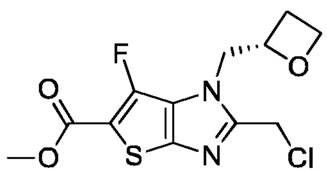

**B-2**

Synthetic route:

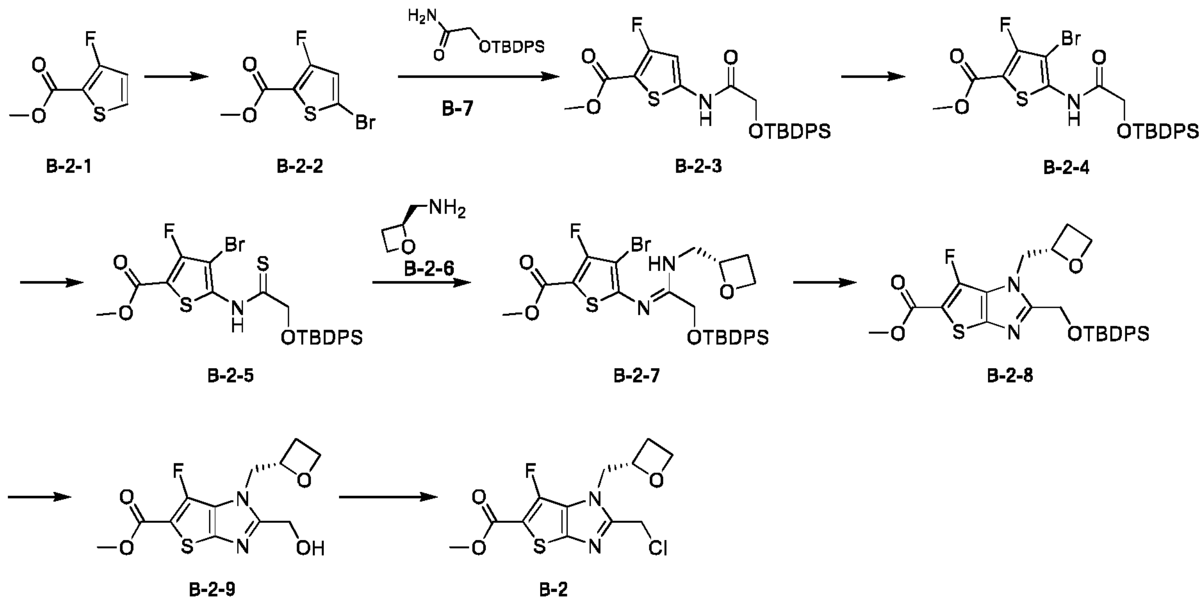

Step 1: Synthesis of compound **B-2-2**

**B-2-1** (2.00 g, 12.49 mmol, 1 *eq*) and anhydrous tetrahydrofuran (100 mL) were added to a reaction flask, and 2,2,6,6-tetramethylpiperidinylmagnesium chloride lithium chloride complex (1 M, 24.00 mL, 1.92 *eq*) was added thereto at -40°C. The reaction system was stirred for 0.5 hours at -40°C, carbon tetrabromide (4.14 g, 12.49 mmol, 1 *eq*) was added thereto, and the reaction system was stirred for 0.5 hours at -40°C and then gradually warmed to 20°C and stirred for 11 hours. The reaction mixture was quenched with hydrochloric acid (0.5 M, 10 mL) and extracted with ethyl acetate (50 mL * 3). The organic phases were combined, and the resulting organic phase was washed with saturated brine (50 mL), dried over anhydrous sodium sulfate, and filtered. The filtrate was concentrated to obtain the crude product. The crude product was purified by column chromatography (petroleum ether: ethyl acetate = 1:0 to 20:1) to obtain compound **B-2-2.** LCMS: m/z = 238.9 $[M+1]^+$.

Step 2: Synthesis of compound **B-2-3**

**B-2-2** (1.70 g, 7.11 mmol, 1 *eq*), **B-7** (2.23 g, 7.11 mmol, 1.0 *eq*), and potassium carbonate (1.97 g, 14.22 mmol, 2 *eq*) were dissolved in anhydrous toluene (50 mL), then tris(dibenzylideneacetone)dipalladium (0.65 g, 709.82 μmol, 0.10 *eq*) and Xantphos (0.82 g, 1.42 mmol, 0.2 *eq*) were added thereto under nitrogen atmosphere, and then the reaction mixture was heated to 100°C and stirred for 12 hours. The reaction mixture was filtered, and the filtrate was added with water (50 mL) and extracted with ethyl acetate (50 mL * 3). The organic phases were combined, and the resulting organic phase was washed with saturated brine (50 mL), dried over anhydrous sodium sulfate, filtered, and the filtrate was concentrated to obtain the crude product. The crude product was purified by column chromatography (petroleum ether: ethyl acetate = 1:0 to 4:1) to obtain **B-2-3.** LCMS: m/z = 472.1 $[M+1]^+$.

Step 3: Synthesis of compound **B-2-4**

**B-2-3** (2.60 g, 2.84 mmol, purity of 51.53%, 1 *eq*) and NBS (1.00 g, 5.62 mmol, 1.98 *eq*) were dissolved in anhydrous THF (50 mL), and the reaction system was stirred for 12 hours at 20°C. The reaction mixture was added with saturated sodium bicarbonate solution (50 mL) and extracted with ethyl acetate (50 mL * 3). The organic phases were combined, and the resulting organic phase was washed with saturated brine (50 mL), dried over anhydrous sodium sulfate, and filtered, the filtrate was concentrated to obtain the crude product. The crude product was purified by column chromatography (petroleum ether: ethyl acetate = 1:0 to 10:1) to obtain **B-2-4.** $^1$H NMR (400 MHz, $CDCl_3$) $\delta$ ppm 9.65 (br s, 1 H), 7.66 (br d, *J* = 6.78 Hz, 4 H), 7.42 - 7.50 (m, 6 H), 4.31 (s, 2 H), 3.89 (s, 3 H), 1.19 (s, 9 H). LCMS: m/z = 550.0 $[M+1]^+$.

Step 4: Synthesis of compound **B-2-5**

**B-2-4** (1.80 g, 3.27 mmol, 1 *eq*) and Lawesson's reagent (1.35 g, 3.34 mmol, 1.02 *eq*) were dissolved in anhydrous dioxane (30 mL), and the reaction system was stirred for 6 hours at 110°C. The reaction mixture was cooled to room temperature and concentrated to obtain the crude product. The crude product was purified by column chromatography (petroleum ether: ethyl acetate = 1:0 to 10:1) to obtain compound **B-2-5.** $^1$H NMR (400 MHz, $CDCl_3$) $\delta$ ppm 11.18 (br s, 1 H), 7.66 (dd, *J*=7.91, 1.38 Hz, 4H), 7.40 - 7.51 (m, 6H), 4.60 (s, 2 H), 3.91 (s, 3 H), 1.20 (s, 9 H). LCMS: m/z = 566.0 $[M+1]^+$.

Step 5: Synthesis of compound **B-2-7**

**B-2-5** (1.50 g, 2.65 mmol, 1 *eq*), **B-2-6** (0.60 g, 6.89 mmol, 2.60 *eq*), and silver acetate (0.90 g, 5.39 mmol, 276.07 μL, 2.04 *eq*) were dissolved in anhydrous DMF (20 mL), and the reaction system was stirred for 16 hours at 20°C. The reaction mixture was filtered, and the filtrate was added with water (50 mL) and extracted with ethyl acetate (50 mL * 3). The organic phases were combined, washed with saturated brine (50 mL), dried over anhydrous sodium sulfate, filtered, and the resulting filtrate was concentrated to obtain the crude product. The crude product was purified by column chromatography (petroleum ether: ethyl acetate = 1:0 to 5:1) to obtain **B-2-7.** $^1$H NMR (400 MHz, $CDCl_3$) $\delta$ ppm 7.63 (br t, *J* = 5.65 Hz, 4 H), 7.39 - 7.52 (m, 6 H), 6.95 (br s, 1 H), 5.05 - 5.17 (m, 1 H), 4.68 - 4.78 (m, 1 H), 4.53 (dt, *J* = 9.22, 5.93 Hz, 1 H), 4.31 - 4.45 (m, 2 H), 3.77 - 3.88 (m, 4 H), 3.56 - 3.66 (m, 1 H), 2.67 - 2.78 (m, 1 H), 2.55 - 2.66 (m, 1 H), 1.10 (s, 9 H). LCMS: m/z = 619.1 $[M+1]^+$.

Step 6: Synthesis of compound **B-2-8**

**B-2-7** (0.80 g, 1.29 mmol, 1 *eq*) and *N,N'*-dimethylethylenediamine (0.16 g, 1.82 mmol, 195.36 μL, 1.41 *eq*)

were dissolved in acetonitrile (10 mL), then cuprous iodide (0.16 g, 840.12 μmol, 0.65 *eq*) was added thereto under nitrogen atmosphere, and the reaction system was stirred at 80°C for 10 hours. The reaction mixture was filtered, added with water (20 mL), and extracted with ethyl acetate (20 mL * 3). The organic phases were combined, washed with saturated brine (20 mL), dried over anhydrous sodium sulfate, filtered, and the filtrate was concentrated to obtain the crude product. The crude product was purified by a preparative thin-layer chromatography silica gel plate (petroleum ether: ethyl acetate = 3:1) to obtain compound **B-2-8.** LCMS: m/z = 539.2 $[M+1]^+$.

Step 7: Synthesis of compound **B-2-9**

**B-2-8** (0.40 g, 742.52 μmol, 1 *eq*) was dissolved in anhydrous tetrahydrofuran (5 mL), then tetrabutylammonium fluoride (1 M, 1.00 mL, 1.35 *eq*) was added thereto, and the reaction system was stirred for 1 hour at 20°C. The reaction mixture was added with water (20 mL) and extracted with ethyl acetate (20 mL * 3). The organic phases were combined, washed with saturated brine (20 mL), dried over anhydrous sodium sulfate, filtered, and the filtrate was concentrated to obtain the crude product. The crude product was purified by column chromatography (petroleum ether: ethyl acetate = 1:0 to 0:1) to obtain compound **B-2-9.** 1H NMR (400 MHz, $CDCl_3$) $\delta$ ppm 5.19 (qd, *J* = 6.90, 2.89 Hz, 1 H), 4.80 - 4.90 (m, 2 H), 4.67 - 4.74 (m, 1 H), 4.40 - 4.56 (m, 3 H), 3.91 (s, 3 H), 2.78 - 2.89 (m, 1 H), 2.48 - 2.58 (m, 1 H). LCMS: m/z = 301.1 $[M+1]^+$.

Step 8: Synthesis of compound **B-2**

**B-2-9** (30 mg, 99.90 μmol, 1 *eq*) was dissolved in anhydrous dichloromethane (2 mL), then methanesulfonyl chloride (261.89 μmol, 20.27 μL, 2.62 *eq)* and triethylamine (296.47 μmol, 41.27 μL, 2.97 *eq*) were added thereto at 0°C (ice-water bath), and the reaction system was stirred for 1 hour at 20°C. The reaction mixture was quenched with saturated sodium bicarbonate aqueous solution (0.5 mL) and concentrated to obtain compound **B-2.** LCMS: m/z=318.8 $[M+1]^+$.

**Reference example 3: Fragments B-3 and B-4**

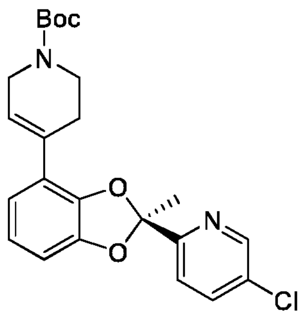

B-3 or B-4

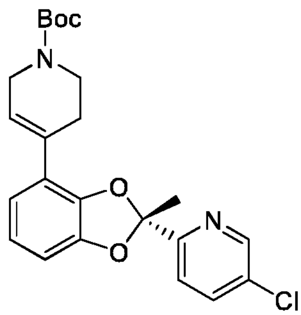

B-4 or B-3

Synthetic route:

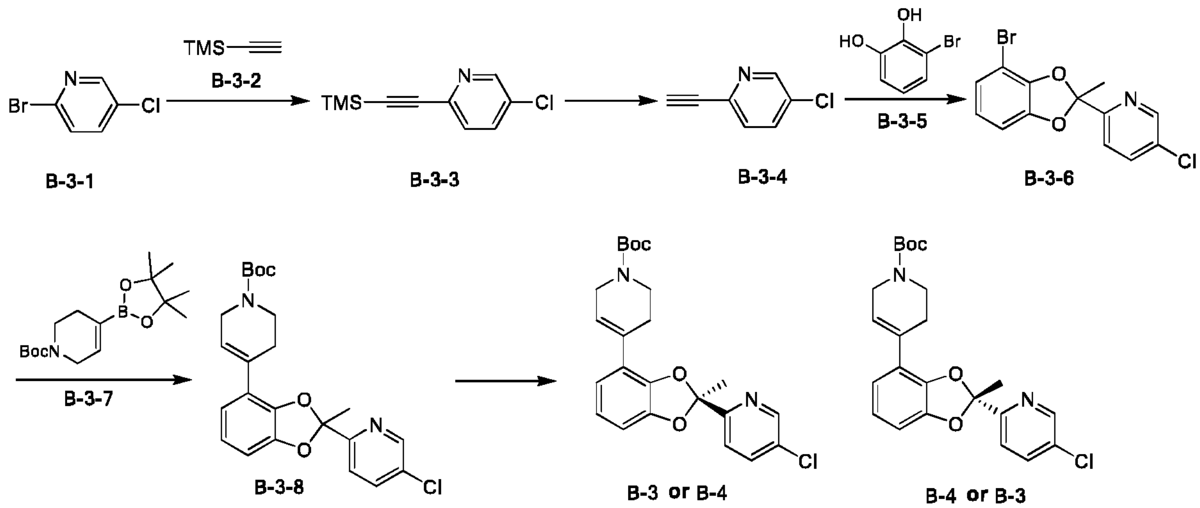


B-3-1 B-3-3 B-3-4 B-3-6

B-3-8 B-3 or B-4 B-4 or B-3

Step 1: Synthesis of compound **B-3-3**

**B-3-2** (519.64 mmol, 71.99 mL, 2 *eq*), cuprous iodide (989.65 mg, 5.20 mmol, 0.02 *eq*), bis(triphenylphosphine)palladium(II) chloride (1.82 g, 2.60 mmol, 0.01 *eq*), and **B-3-1** (50 g, 259.82 mmol, 1 *eq*) were dissolved in triethylamine (550 mL), and the reaction mixture was reacted for 8 hours at 60°C under nitrogen atmosphere. The reaction mixture was diluted with saturated brine (400 mL) and extracted with ethyl acetate (400 mL * 3), and the organic phase was collected after phase separation, dried over anhydrous sodium sulfate, filtered, and concentrated under reduced pressure to obtain a crude product. The crude product was purified by column chromatography (petroleum ether: ethyl acetate = 1:0 to 20:1) to obtain **B-3-3.** LCMS: m/z=210.2 $[M+1]^+$.

Step 2: Synthesis of compound **B-3-4**

Potassium hydroxide (6.29 g, 95.35 mmol, purity of 85%, 1 *eq*) was added to a solution of **B-3-3** (20 g, 95.35 mmol, 1 *eq*) in methanol (400 mL), and the reaction mixture was stirred for 1 hour at 25°C. The reaction mixture was poured into saturated brine (500 mL) and extracted with ethyl acetate (600 mL * 3). The organic phases were combined. The organic phase was dried over anhydrous sodium sulfate, filtered, and the resulting filtrate was concentrated to obtain **B-3-4.** LCMS: m/z=138.2 $[M+1]^+$.

Step 3: Synthesis of compound **B-3-6**

Triruthenium dodecacarbonyl (464.74 mg, 726.92 μmol, 0.02 *eq*), **B-3-5** (6.87 g, 36.35 mmol, 1 *eq*), and **B-3-4** (5 g, 36.35 mmol, 1 *eq*) were dissolved in toluene (100 mL), and the reaction mixture was replaced three times with nitrogen and reacted for 16 hours at 100°C. The reaction mixture was filtered with diatomite, and the filter cake was washed with ethyl acetate (20 mL * 3). The filtrates were combined and concentrated under reduced pressure to obtain the crude product. The crude product was purified by column chromatography (petroleum ether: ethyl acetate = 1:0 to 50:1) to obtain **B-3-6.** LCMS: m/z=326.0 $[M+1]^+$.

Step 4: Synthesis of compound **B-3-8**

**B-3-6** (4.5 g, 13.78 mmol, 1 *eq*), **B-3-7** (6.39 g, 20.67 mmol, 1.5 *eq*), [1,1'-bis(diphenylphosphino)ferrocene]dichloropalladium(II) (2.02 g, 2.76 mmol, 0.2 *eq),* and potassium carbonate (7.62 g, 55.12 mmol, 4 *eq*) were dissolved in dioxane (100 mL)/water (10 mL), and the reaction mixture was replaced three times with nitrogen and reacted for 12 hours at 90°C. The reaction mixture was poured into water (200 mL) and extracted with ethyl acetate (300 mL * 3). The organic phases were combined, dried over anhydrous sodium sulfate, filtered, and the resulting filtrate was concentrated to obtain the crude product. The crude product was purified by column chromatography (petroleum ether: ethyl acetate = 1:0 to 10:1) to obtain **B-3-8.** LCMS: m/z=373.1 $[M-55]^+$.

Step 5: Synthesis of compounds **B-3** and **B-4**

**B-3-8** was purified by preparative SFC [column model: DAICEL CHIRALPAK IC (250 mm * 30 mm * 10 μm);

mobile phase: phase A was supercritical carbon dioxide, phase B was ethanol (0.1% ammonia water); gradient (B%): 35% to 35%] to obtain compound **B-3** and compound **B-4.**

According to SFC detection [column model: Chiralpak AD-3, 50 × 4.6 mm I.D., 3 μm); mobile phase: phase A was supercritical carbon dioxide, phase B was ethanol (0.1% isopropylamine); gradient (B%): 5% to 50%], the retention time of the compound **B-3** was 0.920 minutes, and the e.e. value was 100%; and the retention time of the compound **B-4** was 1.197 minutes, and the e.e. value was 98.4%.

**Reference example 4: Fragments B-5 and B-6**

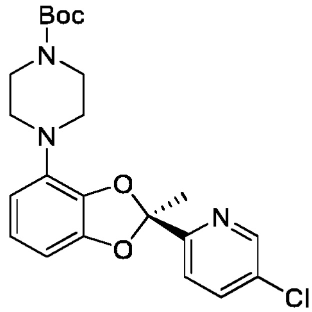

**B-5 or B-6**

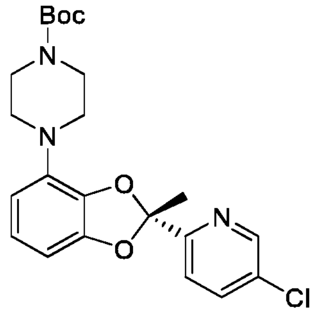

**B-6 or B-5**

Synthetic route:

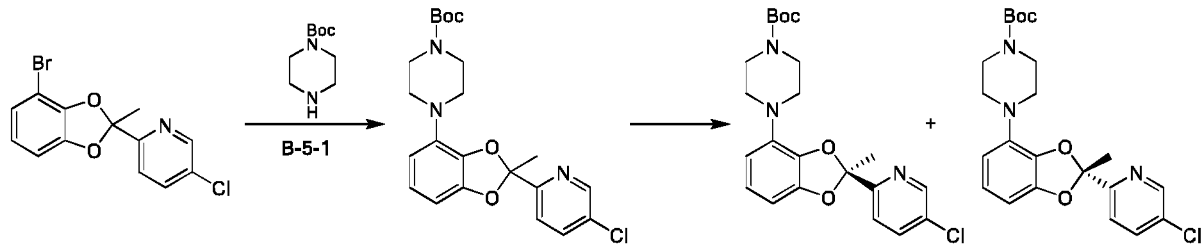


B-3-6 B-5-2 B-5 or B-6 B-6 or B-5

Step 1: Synthesis of compound **B-5-2**

**B-3-6** (1.6 g, 4.90 mmol, 1 *eq*), **B-5-1** (1.31 g, 5.88 mmol, 1.2 *eq*), and cesium carbonate (4.79 g, 14.70 mmol, 3 *eq*) were added to toluene (16 mL), the system was replaced with nitrogen, and then palladium acetate (55.00 mg, 244.97 μmol, 0.05 *eq*) and BINAP (213.55 mg, 342.96 μmol, 0.07 *eq*) were added thereto. The reaction mixture was heated to 100°C, and stirred for 10 hours. The reaction mixture was diluted with water (20 mL) and extracted with ethyl acetate (20 mL * 3). The organic phase was collected after phase separation, washed with saturated brine (20 mL * 3), and dried over anhydrous sodium sulfate, and the resulting filtrate was concentrated under reduced pressure to obtain the crude product. The crude product was purified by column chromatography (petroleum ether: ethyl acetate = 1:0 to 5:1) to obtain **B-5-2.** LCMS: m/z=432.2 $[M+1]^+$.

Step 2: Synthesis of compounds **B-5** and **B-6**

**B-5-2** was purified by preparative SFC [column model: DAICEL CHIRALPAK IC (250 mm * 30 mm * 10 μm); mobile phase: phase A was supercritical carbon dioxide, and phase B was methanol (neutral); gradient (B%): 35% to 35%] to obtain compound **B-5** and compound **B-6.**

According to SFC detection [column model: Chiralpak IG-3, 50 × 4.6 mm I.D., 3 μm); mobile phase: phase A was supercritical carbon dioxide, phase B was methanol (0.1% isopropylamine); gradient (B%): 5% to 50%], the retention time of the compound **B-5** was 1.015 minutes, and the e.e. value was 99.5%; and the retention time of the compound **B-6** was 1.134 minutes, and the e.e. value was 99.6%.

**Reference example 5: Fragment B-7**

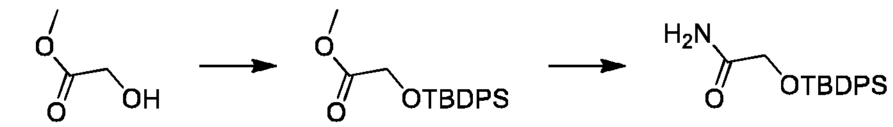

**B-7-1** **B-7-2** **B-7**

Step 1: Synthesis of compound **B-7-2**

**B-7-1** (10.00 g, 111.02 mmol, 1 eq), *tert*-butyldiphenylchlorosilane (36.62 g, 133.22 mmol, 34.22 mL, 1.2 *eq*), and imidazole (8.92 g, 131.00 mmol, 1.18 *eq*) were dissolved in anhydrous DMF (150.00 mL) and the reaction system was stirred for 3 hours at 0°C. The reaction mixture was concentrated to obtain the crude product, which was dissolved with ethyl acetate (200 mL), sequentially washed with water (200 mL * 2) and saturated brine (30 mL). The organic phase was dried over anhydrous sodium sulfate, filtered, and the filtrate was concentrated to obtain the crude product. The crude product was purified by silica gel column chromatography (petroleum ether: ethyl acetate = 1:0 to 10:1) to obtain compound **B-7-2.** $^1$H NMR (400 MHz, $CDCl_3$) $\delta$ ppm 7.69 (dd, *J* = 7.88, 1.38 Hz, 4 H), 7.37 - 7.45 (m, 6 H), 4.26 (s, 2 H), 3.69 (s, 3 H), 1.10 (s, 9 H).

Step 2: Synthesis of compound **B-7**

In a dry reaction flask, **B-7-2** (220 g, 669.76 mmol, 1 *eq*) was added to a methanol solution of ammonia (7 M, 1.58 L, 16.5 *eq*) and stirred for 10 hours at 50°C. The reaction mixture was concentrated and purified by silica gel column chromatography (petroleum ether: ethyl acetate =1:0 to 3:1) to obtain compound **B-7.** $^1$H NMR (400 MHz, DMSO-$d_6$) $\delta$ ppm 7.64 (dd, *J* = 7.91, 1.63 Hz, 4 H), 7.42 - 7.52 (m, 6 H), 7.40 (br s, 1 H), 7.11 (br s, 1 H), 3.94 (s, 2H), 1.02 (s, 9H).

**Reference example 6: Fragment B-8**

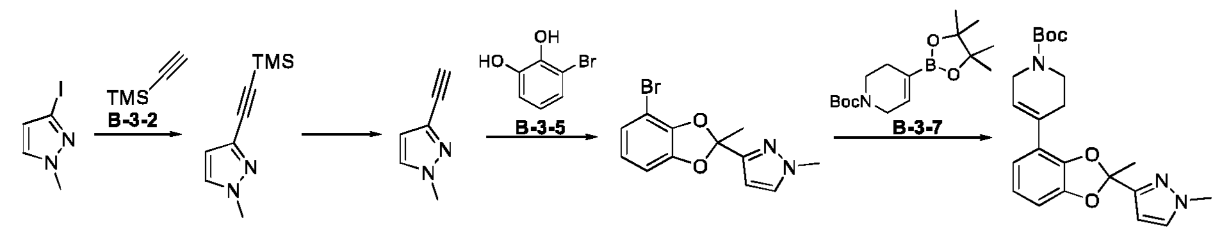


**B-8-1** **B-8-2** **B-8-3** **B-8-4** **B-8**

Step 1: Synthesis of **B-8-2**

**B-8-1** (8.8 g, 42.31 mmol, 1 *eq*) was dissolved in acetonitrile (88.0 mL), and then triethylamine (35.3 mL), cuprous iodide (161.15 mg, 0.85 mmol, 0.02 *eq*), bis(triphenylphosphine)palladium(II) chloride (0.3 g, 0.42 mmol, 0.01 *eq*), and **B-3-2** (59.23 mmol, 8.21 mL, 1.4 *eq*) were sequentially added thereto, and the reaction mixture was reacted for 3 hours at 75°C under nitrogen atmosphere. The reaction mixture was diluted with saturated brine (80 mL) and extracted with ethyl acetate (80 mL * 3), and the organic phase was collected after phase separation, dried over anhydrous sodium sulfate, and concentrated under reduced pressure. The crude product was purified by column chromatography (petroleum ether: ethyl acetate = 1:0 to 10:1) to obtain **B-8-2.** LCMS: m/z=179.0 $[M+1]^+$.

Step 2: Synthesis of compound **B-8-3**

**B-8-2** (6.5 g, 36.45 mmol, 1 *eq*) was dissolved in tetrahydrofuran (60.0 mL), then a solution of potassium hydroxide (2.05 g) in water (10.0 mL) was added thereto, and stirred at 25°C for 12 hours. The reaction mixture was diluted with saturated brine (70 mL) and extracted with ethyl acetate (70 mL * 2). The organic phases were combined,

washed with water (70 mL), dried over anhydrous sodium sulfate, and then filtered, and the filtrate was concentrated to obtain **B-8-3.** $^1$H NMR (400 MHz, $CDCl_3$) $\delta$ppm 7.31 (d, *J*=2.26 Hz, 1 H), 6.42 (d, *J*=2.26 Hz, 1 H), 3.91 (s, 3 H), 3.06 (s, 1 H).

Step 3: Synthesis of compound **B-8-4**

**B-8-3** (2.6 g, 24.50 mmol, 1 *eq*) and **B-3-5** (4.17 g, 22.05 mmol, 0.9 *eq*) were dissolved in toluene (30.0 mL), then triruthenium dodecacarbonyl (313.26 mg, 0.49 mmol, 0.02 *eq*) was added thereto under nitrogen atmosphere, and the reaction mixture was reacted for 12 hours at 100°C. The reaction mixture was filtered with diatomite, and the filter cake was washed with ethyl acetate (100 mL * 2). The filtrates were combined and concentrated under reduced pressure to obtain the crude product. The crude product was purified by column chromatography (petroleum ether: ethyl acetate = 1:0 to 4:1) to obtain **B-8-4.** LCMS: m/z=295.0 $[M+1]^+$.

Step 4: Synthesis of compound **B-8**

**B-8-4** (7.2 g, 24.40 mmol, 1 *eq*), **B-3-7** (9.05 g, 29.28 mmol, 1.2 *eq*), and sodium carbonate (10.34 g, 97.58 mmol, 4 *eq*) were dissolved in dioxane (70 mL)/water (30 mL), then tetrakis(triphenylphosphine)palladium (1.41 g, 1.22 mmol, 0.05 *eq*) was added thereto under nitrogen atmosphere, and the reaction mixture was reacted for 2 hours at 100°C. The reaction mixture was filtered with diatomite, and the filter cake was washed with ethyl acetate (70 mL * 3). The resulting filtrate was washed with water (70 mL), and then concentrated under reduced pressure to obtain the crude product. The crude product was purified by column chromatography (petroleum ether: ethyl acetate = 1:0 to 4:1) to obtain **B-8.** LCMS: m/z=420.0 $[M+23]^+$.

**Reference example 7: Fragment B-9**

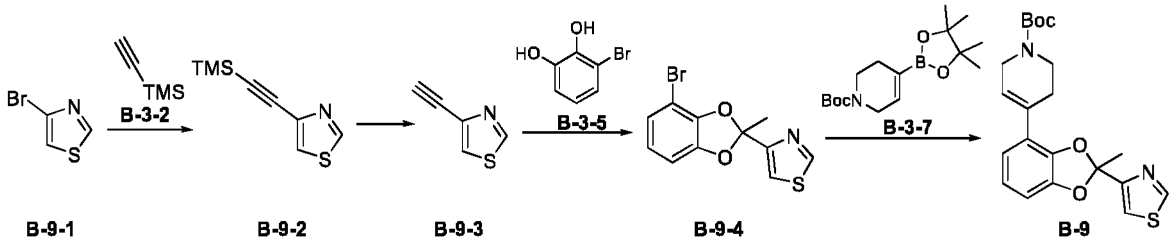


B-9-1 B-9-2 B-9-3 B-9-4 B-9

Step 1: Synthesis of **B-9-2**

**B-9-1** (6.94 g, 42.31 mmol, 1 *eq*) was dissolved in acetonitrile (88.0 mL), and then triethylamine (35.3 mL), cuprous iodide (161.15 mg, 0.85 mmol, 0.02 *eq*), bis(triphenylphosphine)palladium(II) chloride (0.3 g, 0.42 mmol, 0.01 *eq*), and **B-3-2** (59.23 mmol, 8.21 mL, 1.4 *eq*) were sequentially added thereto, and the reaction mixture was reacted for 3 hours at 75°C under nitrogen atmosphere. The reaction mixture was diluted with saturated brine (80 mL) and extracted with ethyl acetate (80 mL * 3), and the organic phase was collected after phase separation, dried over anhydrous sodium sulfate, and concentrated under reduced pressure. The crude product was purified by column chromatography (petroleum ether: ethyl acetate = 1:0 to 15:1) to obtain **B-9-2.** $^1$H NMR (400 MHz, DMSO-$d_6$) $\delta$ *ppm* 9.06 (d, *J*=2.0 Hz, 1 H), 8.05 (s, 1 H), 0.20 (s, 9 H). LCMS: m/z = 181.8 $[M+1]^+$.

Step 2: Synthesis of compound **B-9-3**

**B-9-2** (6.61 g, 36.45 mmol, 1 *eq*) was dissolved in tetrahydrofuran (60.0 mL), then a solution of potassium hydroxide (2.05 g) in water (10.0 mL) was added thereto, and stirred at 25°C for 12 hours. The reaction mixture was diluted with saturated brine (70 mL) and extracted with ethyl acetate (70 mL * 2). The organic phases were combined, washed with water (70 mL), dried over anhydrous sodium sulfate, and then filtered, and the filtrate was concentrated to obtain **B-9-3.** $^1$H NMR (400 MHz, $CDCl_3$) $\delta$ *ppm* 8.76 (s, 1 H), 7.58 (s, 1 H), 3.15 (s, 1 H).

Step 3: Synthesis of compound **B-9-4**

**B-9-3** (2.67 g, 24.50 mmol, 1 *eq*) and **B-3-5** (4.17 g, 22.05 mmol, 0.9 *eq*) were dissolved in toluene (30.0 mL), then triruthenium dodecacarbonyl (313.26 mg, 0.49 mmol, 0.02 *eq*) was added thereto under nitrogen atmosphere, and

the reaction mixture was reacted for 12 hours at 100°C. The reaction mixture was filtered with diatomite, and the filter cake was washed with ethyl acetate (100 mL * 2). The filtrates were combined and concentrated under reduced pressure to obtain the crude product. The crude product was purified by column chromatography (petroleum ether: ethyl acetate = 1:0 to 6:1) to obtain **B-9-4.** LCMS: m/z=298.1 $[M+1]^+$.

Step 4: Synthesis of compound **B-9-5**

**B-9-4** (7.3 g, 24.40 mmol, 1 *eq*), **B-3-7** (9.05 g, 29.28 mmol, 1.2 *eq*), and sodium carbonate (10.34 g, 97.58 mmol, 4 *eq*) were dissolved in dioxane (70 mL)/water (30 mL), then tetrakis(triphenylphosphine)palladium (1.41 g, 1.22 mmol, 0.05 *eq*) was added thereto under nitrogen atmosphere, and the reaction mixture was reacted for 2 hours at 100°C. The reaction mixture was filtered with diatomite, and the filter cake was washed with ethyl acetate (70 mL * 3). The resulting filtrate was washed with water (70 mL), and then concentrated under reduced pressure to obtain the crude product. The crude product was purified by column chromatography (petroleum ether: ethyl acetate = 1:0 to 6:1) to obtain **B-9-5.** LCMS: m/z=401.2 $[M+1]^+$.

**Reference example 8: Fragment B-10**

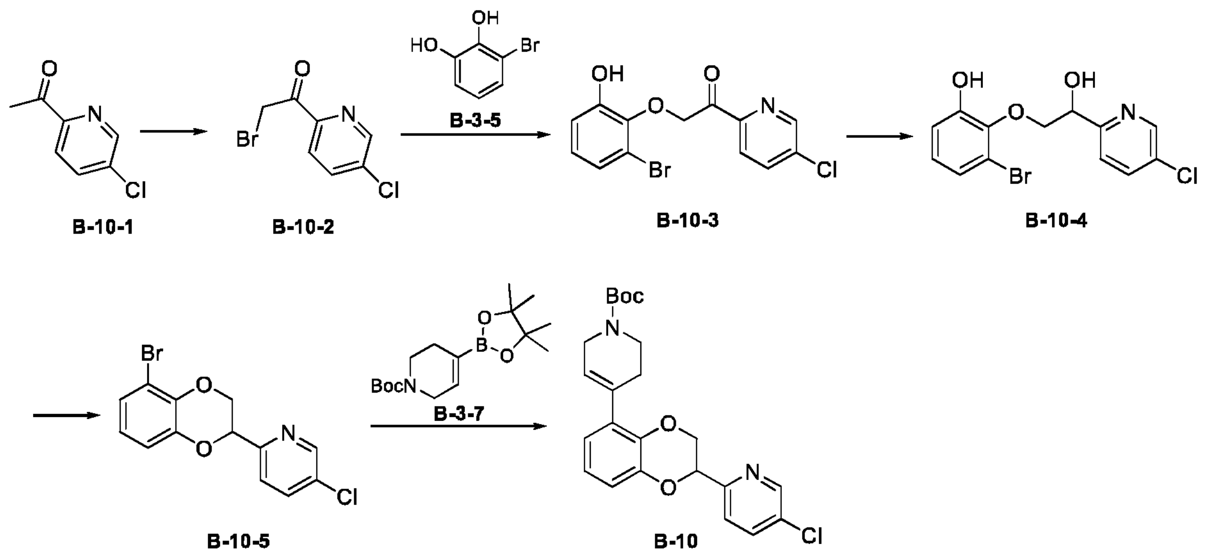


B-10-1 B-10-2 B-10-3 B-10-4 B-10-5 B-10

Step 1: Synthesis of **B-10-2**

**B-10-1** (20 g, 128.55 mmol, 1 *eq*) was dissolved in THF (200 mL), and the reaction system was replaced with nitrogen. Phenyltrimethylammonium tribromide (50.74 g, 134.98 mmol, 1.05 *eq*) was then added to the reaction flask. The reaction mixture was gradually heated to 50°C and stirred for 12 hours. The reaction mixture was quenched with water (50 mL) and then extracted by adding ethyl acetate (50 mL * 2) and the organic phase was collected. The organic phase was washed with saturated brine (50 mL), dried over anhydrous sodium sulfate, filtered, and then concentrated under reduced pressure. A mixture (150 mL) of petroleum ether and ethyl acetate with a ratio of 10:1 was added to the crude product, and the reaction mixture was stirred for 1 hour at 25°C. The reaction mixture was filtered. The filter cake was collected and dried to obtain compound **B-10-2,** which was directly used in the next reaction step. LCMS: m/z=234.0 $[M+1]^+$.

Step 2: Synthesis of **B-10-3**

**B-10-2** (13.70 g, 72.50 mmol, 1 *eq*) was dissolved in anhydrous DMF (170 mL), then potassium carbonate (10.02 g, 72.50 mmol, 1 *eq*) was added thereto, and the reaction mixture was stirred for 18 hours at 25°C. The reaction mixture was diluted with ethyl acetate (170 mL), then sequentially washed with water (170 mL) and saturated aqueous sodium chloride solution (170 mL). The organic phase was dried over anhydrous sodium sulfate and filtered, and the filtrate was concentrated under reduced pressure. The crude product was purified by column chromatography (petroleum ether: ethyl acetate = 50:1 to 10:1) to obtain **B-10-3.** $^1H$ NMR (400 MHz, DMSO-$d_6$) $\delta$ *ppm* 8.69 (d, *J* = 2.4 Hz, 1H), 8.06

(dd, *J* = 2.4, 8.5 Hz, 1H), 7.90 (s, 1H), 7.79 (d, *J* = 8.5 Hz, 1H), 7.18 (d, *J*= 7.9 Hz, 1H), 7.01 (d, *J* = 8.1 Hz, 1H), 6.90 - 6.78 (m, 1H), 4.40 (s, 2H). LCMS: m/z=342.0 $[M+1]^+$.

Step 3: Synthesis of **B-10-4**

**B-10-3** (13 g, 37.95 mmol, 1 *eq*) was dissolved in EtOH (260 mL), the reaction system was replaced with nitrogen, and $NaBH_4$ (2.87 g, 75.90 mmol, 2 *eq*) was added to the reaction flask, and the reaction mixture was stirred for 2 hours at 25°C. The reaction mixture was quenched with saturated aqueous ammonium chloride solution (300 mL) and extracted with ethyl acetate (300 mL * 2). The organic phase was collected, washed with saturated aqueous sodium chloride solution (300 mL), dried over anhydrous sodium sulfate, and filtered. The filtrate was concentrated under reduced pressure to obtain the crude product. The crude product was purified by column chromatography (petroleum ether: ethyl acetate = 100:1 to 10:1) to obtain **B-10-4.** $^1H$ NMR (400 MHz, DMSO-$d_6$) $\delta$ *ppm* 10.31 - 9.59 (m, 1H), 8.57 (d, *J* = 2.3 Hz, 1H), 7.97 (dd, *J* = 2.5, 8.4 Hz, 1H), 7.64 (d, *J* = 8.5 Hz, 1H), 7.00 - 6.95 (m, 1H), 6.90 - 6.85 (m, 2H), 4.97 (dd, *J* = 4.3, 6.4 Hz, 1H), 4.24 (dd, *J* = 4.3, 10.1 Hz, 1H), 4.12 (dd, *J* = 6.6, 10.0 Hz, 1H). LCMS: m/z=344.0 $[M+1]^+$.

Step 4: Synthesis of **B-10-5**

Compound **B-10-4** (3 g, 8.71 mmol, 1 *eq*), triphenylphosphine (4.57 g, 17.41 mmol, 2 *eq*), and tetrahydrofuran (60 mL) were added to a dry reaction flask, and the system was replaced with nitrogen. The mixture was cooled to 0°C, then a solution of DIAD (3.52 g, 17.41 mmol, 2 *eq*) in tetrahydrofuran (15 mL) was added thereto, and the reaction mixture was stirred for 12 hours at 20°C. The reaction system was quenched with water (100 mL), then added with sodium hypochlorite solution (10 mL), and stirred for 10 minutes. The reaction mixture was extracted with ethyl acetate (100 mL $\times$ 2), and the organic phases were combined after phase separation. The organic phase was washed with saturated brine (100 mL), dried over anhydrous sodium sulfate, filtered, and concentrated under reduced pressure to obtain the crude product. The crude product was purified by column chromatography (petroleum ether: ethyl acetate = 100:1 to 10:1) to obtain **B-10-5.** LCMS: m/z=326.0 $[M+1]^+$.

Step 5: Synthesis of **B-10**

Compound **B-10-5** (1 g, 3.06 mmol, 1 *eq*), **B-3-7** (2.84 g, 9.19 mmol, 3 *eq*), 1,4-dioxane (25 mL), $H_2O$ (5 mL), sodium carbonate (973.66 mg, 9.19 mmol, 3 *eq*), and $Pd(PPh_3)_4$ (353.84 mg, 306.21 $\mu$mol, 0.1 *eq*) were sequentially added to a dry reaction flask, and the reaction system was replaced with nitrogen and stirred for 10 hours at 90°C. The reaction system was quenched with water (50 mL), then sodium hypochlorite aqueous solution (10 mL) was added thereto and stirred for 10 minutes, then the resulting mixture was extracted with ethyl acetate (50 mL $\times$ 2), and the organic phases were combined after phase separation. The organic phase was washed with saturated brine (50 mL), dried over anhydrous sodium sulfate, filtered, and concentrated under reduced pressure to obtain the crude product. The crude product was purified by column chromatography (petroleum ether: ethyl acetate= 100:1 to 10:1) to obtain **B-10.** LCMS: m/z=451.2 $[M+23]^+$.

**Reference example 9: Fragment B-11**

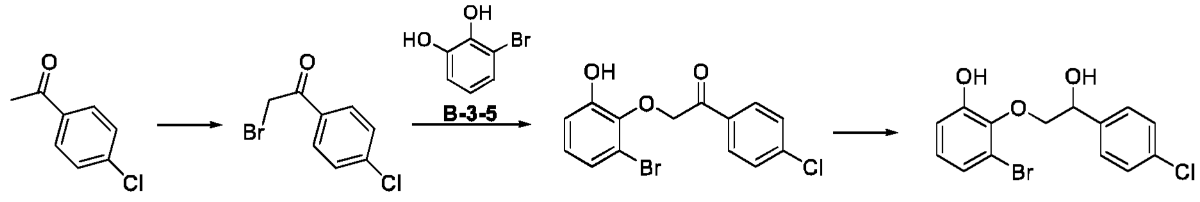


B-11-1 B-11-2 B-11-3 B-11-4

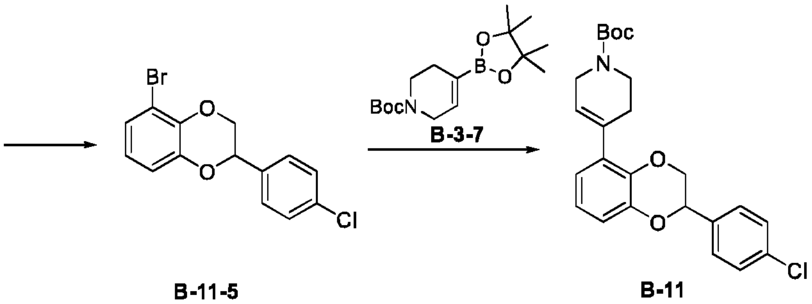


B-11-5 B-11

Step 1: Synthesis of **B-11-2**

Compound **B-11-1** (29 g, 187.59 mmol, 1 *eq*) was dissolved in tetrahydrofuran (290 mL), and the reaction system was replaced with nitrogen. Phenyltrimethylammonium tribromide (74.05 g, 196.97 mmol, 1.05 *eq*) was then added to the reaction flask. The reaction mixture was gradually heated to 50°C and stirred for 12 hours. The reaction mixture was quenched with water (500 mL) and then extracted by adding ethyl acetate (500 mL * 2) and the organic phase was collected. The organic phase was washed with saturated brine (500 mL), dried over anhydrous sodium sulfate, filtered, and concentrated under reduced pressure. A mixture (150 mL) of petroleum ether and ethyl acetate with a ratio of 10:1 was added to the crude product, and the reaction mixture was stirred for 1 hour at 25°C. The resulting mixture was filtered. The filter cake was collected and dried to obtain **B-11-2,** which was directly used in the next reaction step. LCMS: m/z = 233.0 $[M+H]^+$.

Step 2: Synthesis of **B-11-3**

**B-11-2** (10 g, 42.83 mmol, 1 *eq*) and **B-3-5** (8.09 g, 42.83 mmol, 1 *eq*) were dissolved in anhydrous DMF (100 mL), then potassium carbonate (5.92 g, 42.83 mmol, 1 *eq*) was added thereto, and the reaction mixture was stirred for 18 hours at 25°C. The reaction mixture was diluted with ethyl acetate (10 mL), then sequentially washed with water (10 mL) and saturated aqueous sodium chloride solution (10 mL). The organic phase was dried over anhydrous sodium sulfate and filtered, and the filtrate was concentrated under reduced pressure. The crude product was purified by column chromatography (petroleum ether: ethyl acetate= 1:0 to 20:1) to obtain **B-11-3.** $^1$H NMR (400 MHz, DMSO-$d_6$) $\delta$ *ppm* 7.75 (d, *J* = 1.6 Hz, 1H), 7.63 - 7.59 (m, 2H), 7.54 - 7.50 (m, 2H), 7.19 (dd, *J* = 1.4, 7.9 Hz, 1H), 7.00 (dd, *J* = 1.5, 8.1 Hz, 1H), 6.90 - 6.83 (m, 1H), 4.27 (d, *J*= 11.1 Hz, 1H), 4.06 - 4.03 (m, 1H). LCMS: m/z = 341.0 $[M+H]^+$.

Step 3: Synthesis of **B-11-4**

Compound **B-11-3** (6 g, 17.57 mmol, 1 *eq*) was dissolved in EtOH (120 mL), the reaction system was replaced with nitrogen, and sodium borohydride (1.33 g, 35.13 mmol, 2 *eq*) was added to the reaction flask and stirred for 2 hours at 25°C. The reaction mixture was quenched with saturated aqueous ammonium chloride solution (120 mL) and extracted with ethyl acetate (100 mL * 2). The organic phase was collected, washed with saturated aqueous sodium chloride solution (200 mL), dried over anhydrous sodium sulfate, and filtered. The organic phase was concentrated under reduced pressure. The crude product was purified by column chromatography (petroleum ether: ethyl acetate= 100:1 to 10:1) to obtain **B-11-4.** $^1$H NMR (400 MHz, DMSO-$d_6$) $\delta$ *ppm* 9.97 - 9.68 (m, 1H), 7.48 - 7.44 (m, 2H), 7.42 - 7.39 (m, 2H), 7.10 - 6.95 (m, 1H), 6.87 (d, *J*= 5.0 Hz, 2H), 6.30 - 6.09 (m, 1H), 4.98 (dd, *J*= 4.6, 7.2 Hz, 1H), 4.06 (dd, *J* = 4.5, 10.0 Hz, 1H), 3.91 (dd, *J* = 7.3, 10.0 Hz, 1H). LCMS: m/z = 343.0 $[M+H]^+$.

Step 4: Synthesis of **B-11-5**

**B-11-4** (1 g, 2.91 mmol, 1 *eq*) and triphenylphosphine (1.53 g, 5.82 mmol, 2 *eq*) were dissolved in THF (20 mL). The reaction system was replaced with nitrogen, cooled to 0°C, and then a solution of diisopropyl azodicarboxylate

(1.18 g, 5.82 mmol, 2 eq) in THF (5 mL) was slowly added dropwise to the reaction flask at 20°C, and the reaction mixture stirred for 10 hours. The reaction mixture was quenched with water (50 mL) and extracted with ethyl acetate (50 mL * 3). The organic phase was washed with saturated brine (50 mL), dried over anhydrous sodium sulfate, filtered, and the filtrate was concentrated under reduced pressure to obtain the crude product. The crude product was purified by column chromatography (petroleum ether: ethyl acetate= 20:1 to 10:1) to obtain **B-11-5.** $^1$H NMR (400 MHz, DMSO-$d_6$) $\delta$ *ppm* 7.52 (s, 4H), 7.18 (dd, *J* = 1.4, 8.0 Hz, 1H), 7.01 (dd, *J* = 1.4, 8.1 Hz, 1H), 6.88 - 6.79 (m, 1H), 5.33 (dd, *J* = 2.4, 8.3 Hz, 1H), 4.57 (dd, *J* = 2.4, 11.5 Hz, 1H), 4.19 (dd, *J* = 8.3, 11.4 Hz, 1H). LCMS: m/z = 325.0 $[M+H]^+$.

Step 5: Synthesis of **B-11**

**B-11-5** (720 mg, 2.21 mmol, 1 *eq*) and compound **B-3-7** (2.05 g, 6.63 mmol, 3 *eq*) were dissolved in 1,4-dioxane (20 mL) and $H_2O$ (4 mL), and then sodium carbonate (703.15 mg, 6.63 mmol, 3 *eq*) and tetrakis(triphenylphosphine)palladium (255.54 mg, 221.14 μmol, 0.1 *eq*) were sequentially added thereto. The reaction system was replaced with nitrogen, and the reaction mixture was stirred for 10 hours at 90°C. The reaction mixture was diluted with water (20 mL) and extracted with ethyl acetate (20 mL * 3). The resulting organic phase was washed with saturated brine (50 mL), dried over anhydrous sodium sulfate, filtered, and the filtrate was concentrated under reduced pressure to obtain the crude product. The crude product was purified by column chromatography (petroleum ether: ethyl acetate= 100:1 to 20:1) to obtain **B-11.** $^1$H NMR (400 MHz, DMSO-$d_6$) $\delta$ *ppm* 7.50 (s, 4H), 6.92 - 6.88 (m, 1H), 6.84 (t, *J* = 7.8 Hz, 1H), 6.78 - 6.74 (m, 1H), 5.92 - 5.77 (m, 1H), 5.27 (dd, *J* = 2.4, 8.2 Hz, 1H), 4.46 (dd, *J*= 2.5, 11.5 Hz, 1H), 4.10 - 4.00 (m, 2H), 3.95 (br s, 2H), 3.86 (br s, 1H), 3.53 - 3.45 (m, 2H), 1.42 (s, 9H). LCMS: m/z= 450.2 $[M+Na]^+$.

**Reference example 10: Fragment B-12**

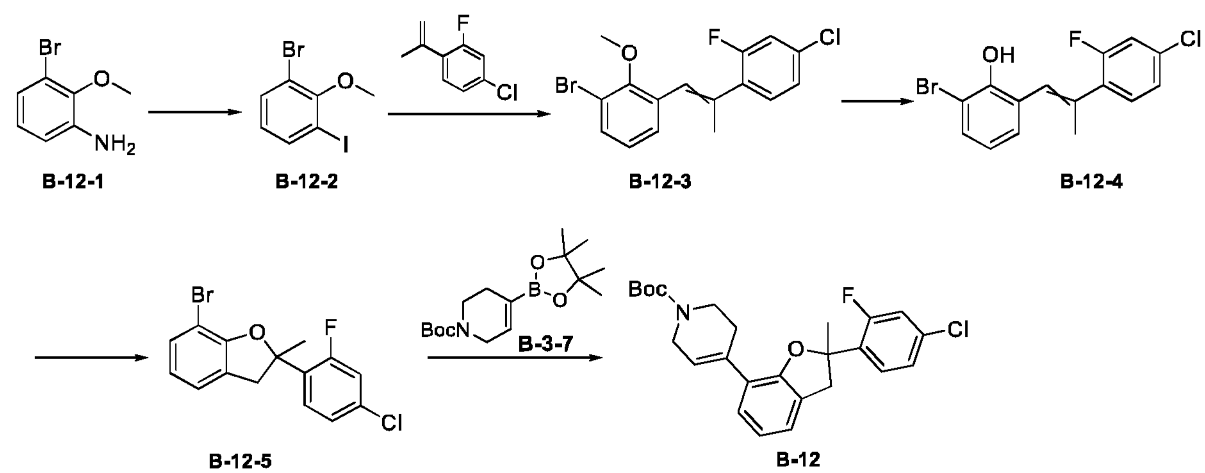

**B-12-1** **B-12-2** **B-12-3** **B-12-4** **B-3-7** **B-12-5** **B-12**

Step 1: Synthesis of **B-12-2**

**B-12-1** (15 g, 74.24 mmol, 1 *eq*) was dissolved in water (113 mL) and hydrochloric acid (75 mL), and the mixture was stirred to homogeneity. A solution of sodium nitrite (6.15 g, 89.09 mmol, 1.2 *eq*) in water (75 mL) was added dropwise to the system at 0°C and stirred for 0.5 hours until completely dissolved. A solution of potassium iodide (24.65 g, 148.48 mmol, 2 *eq*) in water (150 mL) was added to the system and stirred for 4 hours at 0°C. 200 mL of ethyl acetate was added thereto, and the phases were separated. The organic phase was washed with saturated sodium sulfite solution (200 mL), dried over anhydrous sodium sulfate, filtered, and the filtrate was concentrated under reduced pressure to obtain the crude product. The crude product was purified by column chromatography (petroleum ether: ethyl acetate= 1:0 to 10:1) to obtain **B-12-2.** LCMS: m/z=313.0 $[M+1]^+$.

Step 2: Synthesis of **B-12-3**

**B-12-2** (20.8 g, 66.47 mmol, 1 *eq*), 4-chloro-2-fluoro-1-isopropenylbenzene (13.84 g, 81.09 mmol, 1.22 *eq*), cesium carbonate (64.97 g, 199.41 mmol, 3 *eq*), and chloro[(tri-tert-butylphosphine)-2-(2-aminobiphenyl)]palladium(II) (3.41 g, 6.65 mmol, 0.1 *eq*) were dissolved in toluene (125 mL), the reaction system was replaced three times with nitrogen and stirred for 16 hours at 120°C. The system was washed with ethyl acetate (100 mL × 3) and water (100

mL), and the organic phase was dried over saturated anhydrous sodium sulfate and concentrated under reduced pressure to obtain the crude product. The crude product was purified by column chromatography (petroleum ether) to obtain **B-12-3.** LCMS: m/z=355.0 [M+1]$^+$.

Step 3: Synthesis of **B-12-4**

**B-12-3** (2.85 g, 8.01 mmol, 1 *eq*) was dissolved in DCM (20 mL), then boron tribromide (6.02 g, 24.04 mmol, 2.32 mL, 3 *eq*) was added thereto, and the reaction mixture was reacted for 2 hours at 20°C. The system was slowly added to 200 mL of water, and the phases were separated. The organic phase was washed with saturated brine (200 mL × 3), dried over anhydrous sodium sulfate, filtered, and the resulting filtrate was concentrated under reduced pressure to obtain the crude product of **B-12-4.** The crude product was directly used in the next step without further purification. LCMS: m/z=341.0 [M+1]$^+$.

Step 4: Synthesis of **B-12-5**

**B-12-4** (2.96 g, 8.67 mmol, 1 *eq*) was dissolved in formic acid (30 mL) and the reaction mixture was heated to 120°C and reacted for 16 hours. 40 mL of water and 40 mL of ethyl acetate were added to the system, and the phases were separated. The organic phase was washed with saturated brine, dried over anhydrous sodium sulfate, filtered, and the filtrate was concentrated under reduced pressure to obtain the crude product. The crude product was purified by column chromatography (petroleum ether) to obtain **B-12-5.** LCMS: m/z=341.0 [M+1]$^+$.

Step 5: Synthesis of **B-12**

**B-12-5** (1.1 g, 3.22 mmol, 1 *eq*) and **B-3-7** (1.05 g, 3.38 mmol, 1.05 *eq*) were dissolved in 1,4-dioxane (22 mL) and water (2.2 mL), and then [1,1'-bis(diphenylphosphino)ferrocene]dichloropalladium(II) (117.81 mg , 161.00 μmol, 0.05 *eq*) and potassium carbonate (890.08 mg, 6.44 mmol, 2 *eq*) were sequentially added thereto. The system was replaced three times with nitrogen, and the reaction was heated to 120°C and reacted for 16 hours. 30 mL of water and 30 mL of ethyl acetate were added to the system. The resulting organic phase was washed with saturated brine, dried over anhydrous sodium sulfate, filtered, and the filtrate was concentrated under reduced pressure to obtain the crude product. The crude product was purified by column chromatography (petroleum ether: ethyl acetate = 1:0 to 5:1) to obtain **B-12.** LCMS: m/z=444.2 [M+1]$^+$.

**Reference example 11: Fragment B-13**

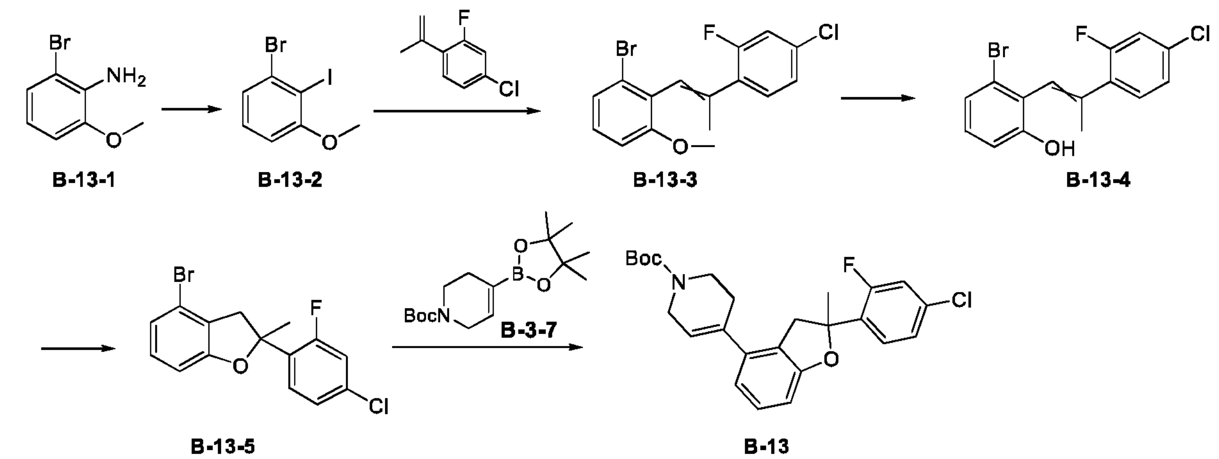
B-13-1
B-13-2
B-13-3
B-13-4
B-3-7
B-13-5
B-13

Step 1: Synthesis of **B-13-2**

**B-13-1** (20 g, 98.99 mmol, 1 *eq*) was dissolved in water (150 mL) and hydrochloric acid (100 mL), and the mixture was stirred to homogeneity. A solution of sodium nitrite (8.20 g, 118.78 mmol, 1.2 *eq*) in water (100 mL) was added dropwise to the system at 0°C and stirred for 0.5 hours until completely dissolved. A solution of potassium iodide (32.86 g, 197.97 mmol, 2 *eq*) in water (200 mL) was added to the system and stirred for 4 hours at 0°C. 200 mL of ethyl

acetate was added thereto, and the phases were separated. The organic phase was washed with saturated sodium sulfite solution (200 mL), dried over anhydrous sodium sulfate, filtered, and the filtrate was concentrated under reduced pressure to obtain the crude product. The crude product was purified by column chromatography (petroleum ether: ethyl acetate= 1:0 to 10:1) to obtain **B-13-2.** LCMS: m/z=313.0 $[M+1]^+$.

Step 2: Synthesis of **B-13-3**

**B-13-2** (26, 83.09 mmol, 1 *eq*), 4-chloro-2-fluoro-1-isopropenylbenzene (17.29 g, 101.36 mmol, 1.22 *eq*), bis(triphenylphosphine)palladium(II) chloride (5.83 g, 8.31 mmol, 0.1 *eq*), and sodium acetate (20.45 g, 249.26 mmol, 3 *eq*) were dissolved in DMF (420 mL), the reaction system was replaced three times with nitrogen and stirred for 16 hours at 120°C. The system was washed with ethyl acetate (400 mL) and water (400 mL), and the organic phase was dried over saturated anhydrous sodium sulfate and concentrated under reduced pressure to obtain the crude product. The crude product was purified by column chromatography (petroleum ether) to obtain **B-13-3.** LCMS: m/z=355.0 $[M+1]^+$.

Step 3: Synthesis of **B-13-4**

**B-13-3** (4.65 g, 13.08 mmol, 1 *eq*) was dissolved in DCM (93 mL), then boron tribromide (9.83 g, 39.23 mmol, 3.78 mL, 3 *eq*) was added thereto, and the reaction mixture was reacted for 3 hours at 25°C. The system was slowly added to 110 mL of water, and the phases were separated. The organic phase was washed with saturated brine (100 mL $\times$ 3), dried over anhydrous sodium sulfate, filtered, and the resulting filtrate was concentrated under reduced pressure to obtain the crude product of **B-13-4.** The system was directly used in the next step without further purification. LCMS: m/z=341.0 $[M+1]^+$.

Step 4: Synthesis of **B-13-5**

**B-13-4** (4.7 g, 13.76 mmol, 1 *eq*) was dissolved in formic acid (47 mL) and the reaction mixture was heated to 110°C and reacted for 16 hours. 50 mL of water and 50 mL of ethyl acetate were added to the system, and the pH of the system was adjusted to about 7 with 1 M lithium hydroxide, and the phases were separated. After phase separation, the organic phase was washed with saturated brine, dried over anhydrous sodium sulfate, filtered, the filtrate was concentrated under reduced pressure to obtain the crude product. The crude product was purified by column chromatography (petroleum ether) to obtain **B-13-5.** LCMS: m/z=341.0 $[M+1]^+$.

Step 5: Synthesis of **B-13**

**B-13-5** (2.9 g, 8.49 mmol, 1 *eq*) and **B-3-7** (2.63 g, 8.49 mmol, 1 *eq*) were dissolved in 1,4-dioxane (30 mL) and water (3.0 mL), and then [1,1'-bis(diphenylphosphino)ferrocene]dichloropalladium(II) (310.59 mg , 424.50 μmol, 0.05 *eq*) and potassium carbonate (2.35 g, 16.98 mmol, 2 *eq*) were sequentially added thereto. The system was replaced three times with nitrogen, and the reaction was heated to 120°C and reacted for 16 hours. 40 mL of water and 40 mL of ethyl acetate were added to the system. The resulting organic phase was washed with saturated brine, dried over anhydrous sodium sulfate, filtered, and the filtrate was concentrated under reduced pressure to obtain the crude product. The crude product was purified by column chromatography (petroleum ether: ethyl acetate = 1:0 to 5:1) to obtain **B-13.** LCMS: m/z=444.2 $[M+1]^+$.

**Example 1**

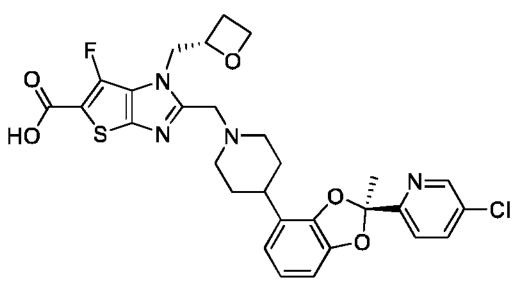

**001 or 001'**

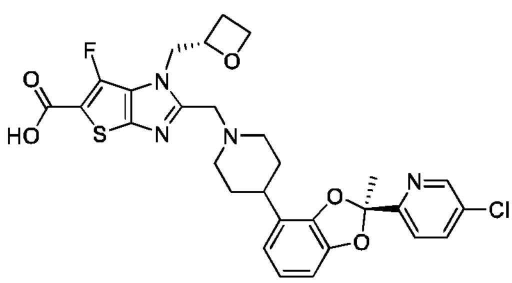

**001' or 001**

Synthetic route:

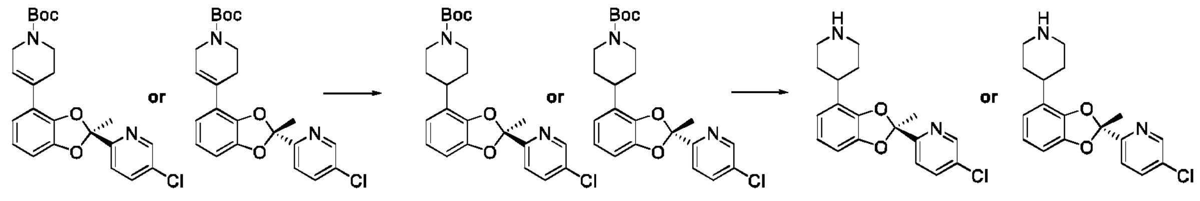

B-3 or B-4 B-4 or B-3 001-1 001-2

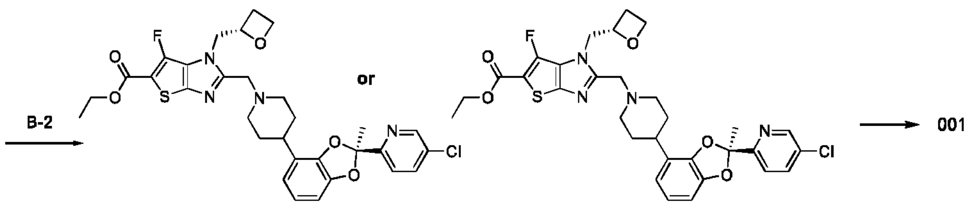


001-3

Step 1: Synthesis of Compound **001-1**

**B-3** (0.3 g, 699.45 μmol, 1 *eq*), methanol (6 mL), and tris(triphenylphosphine)rhodium(I) chloride (64.71 mg, 69.95 μmol, 0.1 *eq*) were sequentially added to a dry reaction flask. The reaction mixture was stirred for 12 hours under hydrogen atmosphere (60°C, 50 psi). The reaction mixture was filtered with diatomite, and the filter cake was washed with methanol (3 mL * 3). The resulting filtrate was concentrated to obtain the crude product of **001-1.** LCMS: m/z = 431.1 $[M+1]^+$.

Step 2: Synthesis of Compound **001-2**

Trifluoroacetic acid (2.33 g, 20.42 mmol, 1.51 mL, 20 *eq*) was added to a solution of **001-1** (440 mg, 1.02 mmol, 1 *eq*) in dichloromethane (4 mL), and the reaction mixture was reacted for 15 minutes at 25°C. The reaction mixture was directly concentrated to obtain the trifluoroacetate salt of the crude product of **001-2.** LCMS: m/z = 331.1 $[M+1]^+$.

Step 3: Synthesis of Compound **001-3**

**001-2** (50.00 mg, crude TFA salt), **B-2** (38.54 mg, 120.92 μmol, 0.8 *eq*), potassium carbonate (83.56 mg, 604.60 μmol, 4.00 *eq*), and acetonitrile (2 mL) were added to a dry reaction flask, and the reaction mixture was heated to 60°C and stirred for 10 hours. The reaction mixture was diluted with water (10 mL) and extracted with ethyl acetate (10 mL * 3). After phase separation, the organic phase was collected, sequentially washed with saturated brine (10 mL), dried over anhydrous sodium sulfate, filtered, and the resulting filtrate was concentrated under reduced pressure to obtain the crude product of **001-3.** LCMS: m/z = 627.1 $[M+1]^+$.

Step 4: Synthesis of Compound **001**

**001-3** (80 mg, 130.48 μmol, 1 *eq*), methanol (1 mL), tetrahydrofuran (1 mL), water (1 mL), and lithium hydroxide monohydrate (16.43 mg, 391.45 μmol, 3 *eq*) were sequentially added to a reaction flask, and the reaction mixture was stirred for 6 hours at 20°C. The reaction mixture was concentrated under reduced pressure and purified by preparative high performance liquid chromatography (method: chromatographic column: Waters Xbridge BEH C18 100 * 30 mm * 10 μm; mobile phase: [Water (ammonia water + ammonium bicarbonate)-acetonitrile]; B (acetonitrile)%: 10% to 45%) to obtain compound **001.** Chiral analysis SFC detection (instrument: CAS-TJ-ANA-SFC-G (Waters UPCC with PDA); chromatographic column: Chiralcel OJ-3, 50 × 4.6 mm I.D., 3 μm; mobile phase A: supercritical carbon dioxide, B: methanol (0.1% isopropylamine); gradient: the content of B increased from 5% to 50% in 0.2 minutes and held for 2 minutes, and then decreased from 50% to 5% in 2.2 minutes) showed a retention time of 1.422 minutes for compound **001** with an ee value of 100%. $^1H$ NMR (400 MHz, $CD_3OD$) $\delta$ *ppm* ppm 8.67 - 8.56 (m, 1H), 7.94 - 7.87 (m, 1H), 7.68 (br d, *J* = 8.4 Hz, 1H), 6.87 - 6.72 (m, 3H), 5.27 - 5.22 (m, 2H), 4.74 - 4.68 (m, 3H), 4.50 - 4.43 (m, 1H), 4.15 - 4.07 (m, 2H), 3.35 - 3.31 (m, 1H), 3.27 - 3.20 (m, 1H), 2.87 - 2.80 (m, 2H), 2.64 - 2.50 (m, 3H), 2.07 - 1.92 (m, 6H). LCMS:

m/z=599.1 [M+1]$^+$.

Using compound **B-4** as raw material, compound **001'** was synthesized by referring to the synthesis method of steps 1 to 4 of Example 1. Chiral analysis SFC detection (instrument: CAS-TJ-ANA-SFC-G (Waters UPCC with PDA); chromatographic column: Chiralcel OJ-3, 50 × 4.6 mm I.D., 3 μm; mobile phase A: supercritical carbon dioxide, B: methanol (0.1% isopropylamine); gradient: the content of B increased from 5% to 50% in 0.2 minutes and held for 2 minutes, and then decreased from 50% to 5% in 2.2 minutes) showed a retention time of 1.030 minutes for compound **001'** with an ee value of 100%. $^1$H NMR (400 MHz, $CD_3OD$) *δ ppm* 8.72 - 8.51 (m, 1H), 8.00 - 7.79 (m, 1H), 7.66 (d, *J*= 8.5 Hz, 1H), 6.91 - 6.66 (m, 4H), 5.29 - 5.15 (m, 2H), 4.50 - 4.35 (m, 3H), 4.22 - 4.06 (m, 3H), 3.77 (s, 1H), 3.64 (s, 1H), 2.94 - 2.74 (m, 3H), 2.71 - 2.57 (m, 3H), 2.54 - 2.42 (m, 1H), 1.97 - 1.85 (m, 3H). LCMS: m/z=599.1 [M+1]$^+$.

Example 2

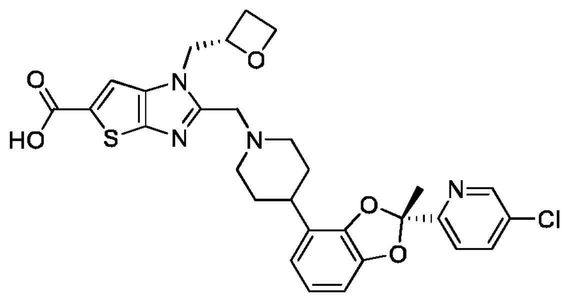

**002 or 002'**

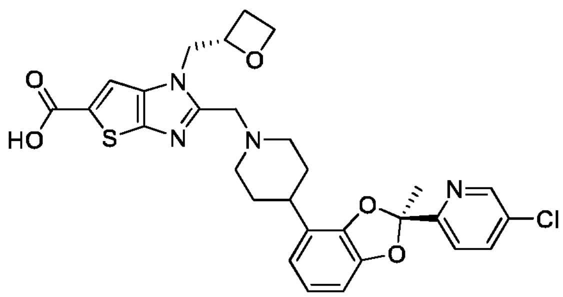

**002' or 002**

Synthetic route:

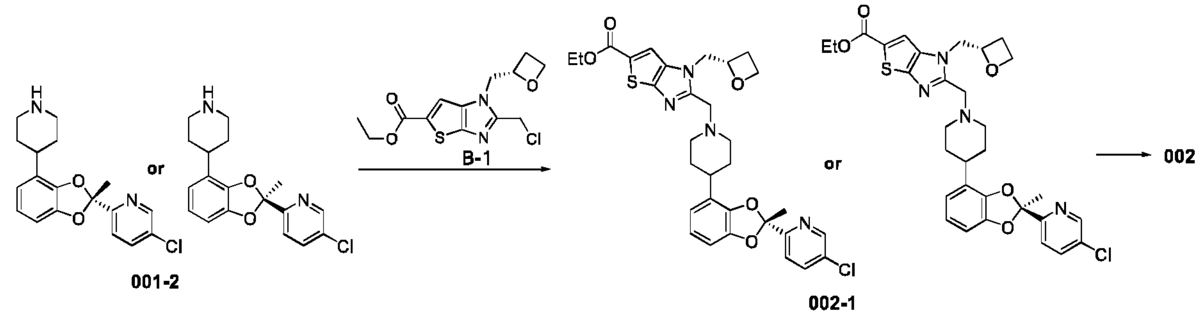


Step 1: Synthesis of Compound **002-1**

**001-2** (46.00 mg, 139.05 μmol, 1 *eq*), **B-1** (43.77 mg, 139.05 μmol, 1 *eq*), potassium carbonate (76.87 mg, 556.21 μmol, 4 *eq*), and acetonitrile (2 mL) were added to a dry reaction flask, and the reaction mixture was heated to 60°C and stirred for 10 hours. The reaction mixture was diluted with water (10 mL) and extracted with ethyl acetate (3 * 10 mL). After phase separation, the organic phase was collected, sequentially washed with saturated brine (10 mL), dried over anhydrous sodium sulfate, filtered, and the resulting filtrate was concentrated under reduced pressure to obtain the crude product. The crude product was purified by preparative thin-layer chromatography (PE:EA = 1:0 to 2:1) to obtain **002-1.** LCMS: m/z = 609.2 [M+1]$^+$.

Step 2: Synthesis of Compound **002**

**002-1** (27.00 mg, 44.33 μmol, 1 *eq*), methanol (0.5 mL), tetrahydrofuran (0.5 mL), water (0.5 mL), and lithium hydroxide monohydrate (5.58 mg, 132.98 μmol, 3 *eq*) were sequentially added to a dry reaction flask, and the reaction system was replaced with nitrogen and stirred for 6 hours at 20°C. The reaction mixture was concentrated under reduced

pressure and purified by preparative high performance liquid chromatography (method: chromatographic column: Waters Xbridge BEH C18 100 * 30 mm * 10 μm; mobile phase: [Water (ammonium bicarbonate)-acetonitrile]; B(acetonitrile)%: 15% to 45%) to obtain compound **002.** Chiral analysis SFC detection (instrument: CAS-TJ-ANA-SFC-H (Waters UPCC with SQ Detector 2); chromatographic column: Chiralpak AD-3, 50 × 4.6 mm I.D., 3 μm; mobile phase A: supercritical carbon dioxide, B: methanol (0.1% isopropylamine); gradient: the content of B increased from 5% to 50% in 0.2 minutes and held for 2 minutes, and then decreased from 50% to 5% in 2.2 minutes) showed a retention time of 1.264 minutes for compound **002** with an ee value of 100%. $^1$H NMR (400 MHz, $CD_3OD$) *δ ppm* 8.60 (d, *J* = 1.9 Hz, 1H), 7.69 - 7.65 (m, 1H), 7.61 - 7.58 (m, 1H), 7.42 (s, 1H), 6.81 - 6.67 (m, 3H), 5.17 - 5.04 (m, 1H), 4.67 - 4.52 (m, 3H), 4.39 - 4.33 (m, 1H), 4.15 - 4.01 (m, 2H), 3.57 - 3.42 (m, 2H), 2.88 - 2.79 (m, 1H), 2.73 - 2.65 (m, 1H), 2.60 - 2.51 (m, 2H), 2.43 - 2.37 (m, 1H), 2.11 (br s, 1H), 2.03 (s, 3H), 1.93 (br s, 2H), 1.65 (s, 1H). LCMS: m/z = 581.1 $[M+1]^+$.

Using compound **B-4** as raw material, compound **002'** was synthesized by referring to the synthesis methods of steps 1 to 2 in Example 1 and steps 1 to 2 of Example 2. Chiral analysis SFC detection (instrument: CAS-TJ-ANA-SFC-H (Waters UPCC with SQ Detector 2); chromatographic column: Chiralpak AD-3, 50 × 4.6 mm I.D., 3 μm; mobile phase A: supercritical carbon dioxide, B: methanol (0.1% isopropylamine); gradient: the content of B increased from 5% to 50% in 0.2 minutes and held for 2 minutes, and then decreased from 50% to 5% in 2.2 minutes) showed a retention time of 1.246 minutes for compound **002'** with an ee value of 100%. $^1$H NMR (400 MHz, $CD_3OD$) *δ ppm* 8.68 (d, *J* = 2.4 Hz, 1H), 7.97 - 7.92 (m, 1H), 7.78 - 7.70 (m, 2H), 6.93 - 6.85 (m, 1H), 6.79 - 6.78 (m, 1H), 6.82 - 6.78 (m, 1H), 5.38 - 5.20 (m, 1H), 4.79 - 4.61 (m, 4H), 4.53 - 4.47 (m, 1H), 4.27 (br d, *J* = 2.1 Hz, 2H), 3.46 (br d, *J* = 11.6 Hz, 1H), 2.94 - 2.75 (m, 4H), 2.62 - 2.53 (m, 1H), 2.09 (s, 4H), 1.99 - 1.98 (m, 1H), 2.04 - 1.96 (m, 2H). LCMS: m/z = 581.1 $[M+1]^+$.

**Example 3**

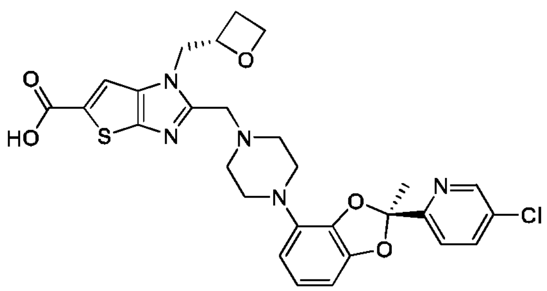

**003 or 003'**

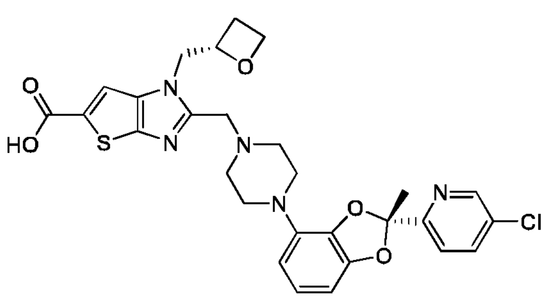

**003' or 003**

Synthetic route:

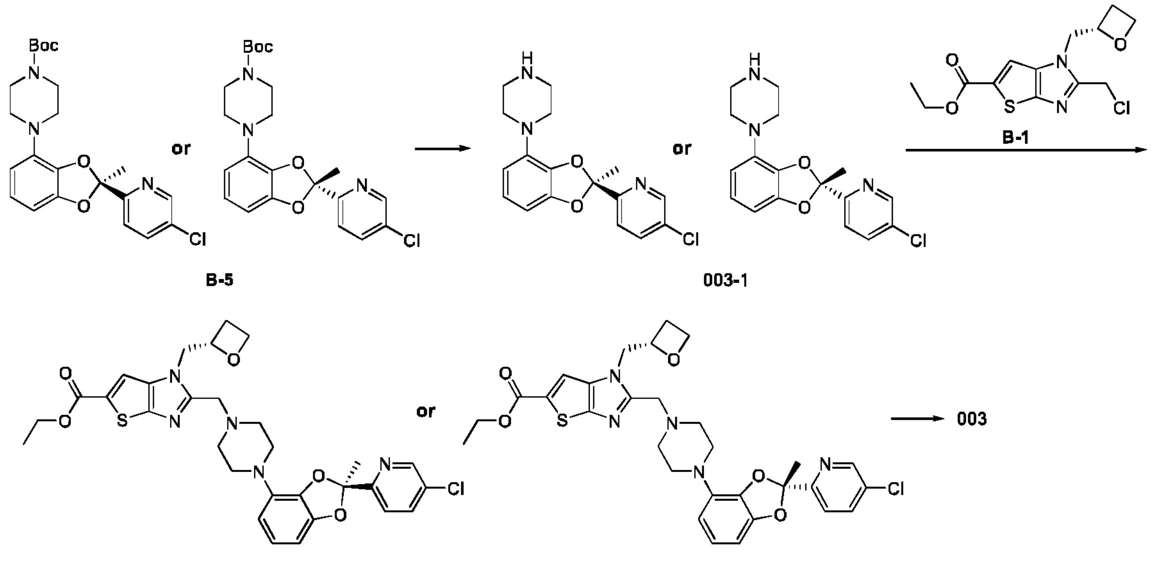

B-5 or B-5 → 003-1 or 003-1 (B-1) → 003-2 or 003-2 → 003

Step 1: Synthesis of Compound **003-1**

**B-5** (100 mg, 231.53 μmol, 1 *eq*) was dissolved in dichloromethane (1 mL), then trifluoroacetic acid (0.2 mL) was added thereto, and the reaction mixture was stirred for 1 hour at 20°C. The reaction mixture was directly concentrated under reduced pressure to obtain crude trifluoroacetate salt of **003-1.** LCMS: m/z = 332.1 $[M+1]^+$.

Step 2: Synthesis of Compound **003-2**

**003-1** (77.00 mg, crude trifluoroacetate salt), **B-1** (54.37 mg, 172.72 μmol, 1 *eq*), potassium carbonate (143.23 mg, 1.04 mmol, 6 *eq*), and acetonitrile (2 mL) were sequentially added to a dry reaction flask, and the reaction mixture was heated to 60°C and stirred for 10 hours. The reaction mixture was added with water (10 mL) and extracted with ethyl acetate (10 mL * 3). The organic phase was collected after phase separation, washed with saturated brine (10 mL * 3), then dried over anhydrous sodium sulfate, and the resulting filtrate was concentrated under reduced pressure to obtain the crude product. The crude product was purified by thin-layer chromatography silica gel plate (petroleum ether: ethyl acetate = 2:1) to obtain **003-2.** LCMS: m/z = 610.2 $[M+1]^+$.

Step 3: Synthesis of Compound **003**

**003-2** (60 mg, 98.34 μmol, 1 *eq*) was dissolved in tetrahydrofuran (0.5 mL), methanol (0.5 mL), and water (0.5 mL), then lithium hydroxide monohydrate (12.38 mg, 295.02 μmol, 3 *eq*) was added thereto, and the reaction mixture was stirred for 6 hours at 20°C. The reaction mixture was concentrated under reduced pressure to obtain the crude product, which was purified by preparative high performance liquid chromatography to obtain **003.** Method: chromatographic column: Waters Xbridge BEH C18 100 * 30 mm * 10 μm; mobile phase: [Water (ammonium bicarbonate)-acetonitrile]; B (acetonitrile)%: 30% to 60%. Chiral analysis SFC detection (instrument: CAS-TJ-ANA-SFC-H (Waters UPCC with SQ Detector 2); chromatographic column: Chiralpak IG-3, 50 × 4.6 mm I.D., 3 μm; mobile phase A: supercritical carbon dioxide, B: methanol (0.1% isopropylamine); gradient: the content of B increased from 5% to 50% in 0.2 minutes and held for 1 minute, and then decreased from 50% to 5% in 2.2 minutes) showed a retention time of 1.856 minutes with an ee value of 100%. $^1$H NMR (400 MHz, $CD_3OD$ ) $\delta$ ppm 8.59 (d, *J* = 2.4 Hz, 1H), 7.87 (dd, *J* = 2.4, 8.5 Hz, 1H), 7.79 (s, 1H), 7.66 (dd, *J* = 0.6, 8.5 Hz, 1H), 6.83 - 6.71 (m, 1H), 6.56 - 6.41 (m, 2H), 5.22 (br dd, *J* = 2.6, 7.4 Hz, 1H), 4.74 - 4.64 (m, 2H), 4.60 - 4.54 (m, 1H), 4.43 (td, *J* = 6.0, 9.2 Hz, 1H), 4.02 - 3.80 (m, 2H), 3.20 (br s, 4H), 2.83 - 2.75 (m, 1H), 2.74 - 2.63 (m, 4H), 2.48 (tdd, *J* = 7.2, 9.1, 11.4 Hz, 1H), 2.02 (s, 3H). LCMS: m/z = 582.1 $[M+1]^+$.

Using compound **B-6** as raw material, compound **003'** was synthesized by referring to the synthesis method of steps 1 to 3 of Example 3. Chiral analysis SFC detection (instrument: CAS-TJ-ANA-SFC-H (Waters UPCC with SQ Detector 2); chromatographic column: Chiralpak IG-3, 50 × 4.6 mm I.D., 3 μm; mobile phase A: supercritical carbon dioxide, B: methanol (0.1% isopropylamine); gradient: the content of B increased from 5% to 50% in 0.2 minutes and held for 1 minute, and then decreased from 50% to 5% in 2.2 minutes) showed a retention time of 1.730 minutes with an ee value of 100%. $^1$H NMR (400 MHz, $CD_3OD$) $\delta$ *ppm* 8.59 (d, *J* = 2.4 Hz, 1H), 7.87 (dd, *J* = 2.4, 8.4 Hz, 1H), 7.78 (s, 1H), 7.66 (d, *J* = 8.5 Hz, 1H), 6.83 - 6.70 (m, 1H), 6.49 (dd, *J* = 8.0, 13.3 Hz, 2H), 5.22 (dq, *J* = 2.8, 7.1 Hz, 1H), 4.72

- 4.63 (m, 2H), 4.61 - 4.55 (m, 1H), 4.43 (td, *J* = 5.9, 9.1 Hz, 1H), 3.99 - 3.82 (m, 2H), 3.19 (br s, 4H), 2.83 - 2.75 (m, 1H), 2.71 (br d, *J* = 4.4 Hz, 4H), 2.48 (tdd, *J* = 7.2, 9.1, 11.3 Hz, 1H), 2.02 (s, 3H). LCMS: m/z = 581.1 $[M+1]^+$.

**Example 4**

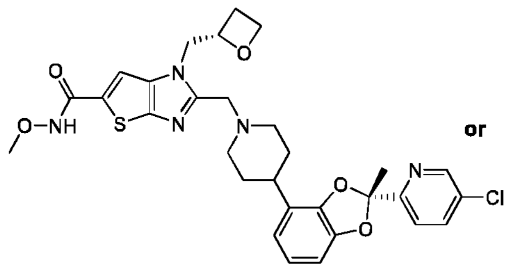

or

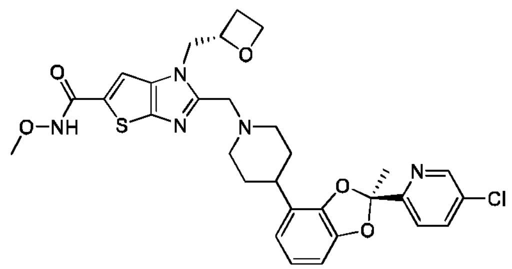

**004**

Synthetic route:

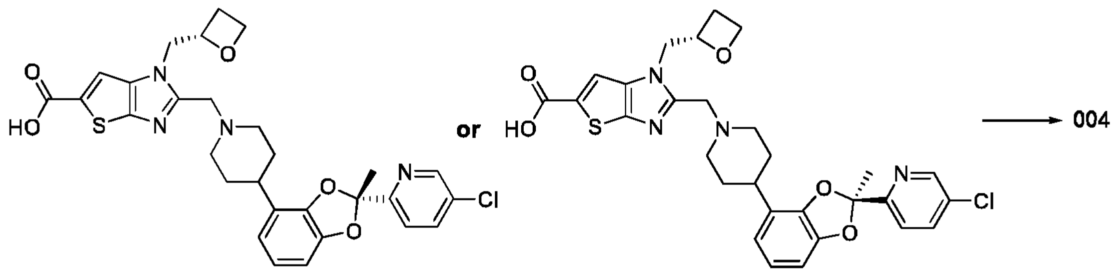

or

004

**002**

**002** (150 mg, 0.26 mmol, 1 *eq),* dichloromethane (12.0 mL), HATU (147.2 mg, 0.39 mmol, 1.5 *eq),* and DIPEA (157.6 μL, 0.91 mmol, 3.5 *eq)* were sequentially added to a dry reaction flask, and the reaction mixture was stirred at 24°C for 0.5 hours. Methoxyamine hydrochloride (32.4 mg, 0.26 mmol, 1.5 *eq)* was then added thereto, and the reaction mixture was stirred for another 0.5 hours at 24°C. The reaction mixture was added with water (10 mL) and extracted with dichloromethane (10 mL * 3). The organic phase was collected after phase separation, washed with saturated brine (10 mL * 2), and dried over anhydrous sodium sulfate, and the resulting filtrate was concentrated under reduced pressure to obtain the crude product. The crude product was purified by thin-layer chromatography silica gel plate (dichloromethane: methanol = 20:1) and preparative high performance liquid chromatography, method: chromatographic column: Waters Xbridge BEH C18 100 * 30 mm * 10 μm; mobile phase: [water (ammonium bicarbonate)-acetonitrile]; gradient (acetonitrile)%: 30% to 60%, to obtain **004.** $^1$H NMR (400 MHz, $CDCl_3$) *δ ppm* 8.61 - 8.53 (m, 1H), 7.70 - 7.62 (m, 2H), 7.58 - 7.52 (m, 1H), 6.79 - 6.72 (m, 1H), 6.71 - 6.63 (m, 2H), 5.30 (s, 3H), 5.22 - 5.12 (m, 1H), 4.64 - 4.57 (m, 1H), 4.55 - 4.44 (m, 2H), 4.42 - 4.34 (m, 1H), 3.82 (s, 3H), 3.79 - 3.74 (m, 1H), 3.62 - 3.54 (m, 2H), 3.06 - 3.02 (m, 2H), 2.97 - 2.93 (m, 1H), 2.99 - 2.92 (m, 1H), 2.91 - 2.82 (m, 1H), 2.78 - 2.66 (m, 2H), 2.49 - 2.41 (m, 1H), 2.30 - 2.12 (m, 2H). LCMS: m/z = 610.2 $[M+1]^+$.

**Example 5**

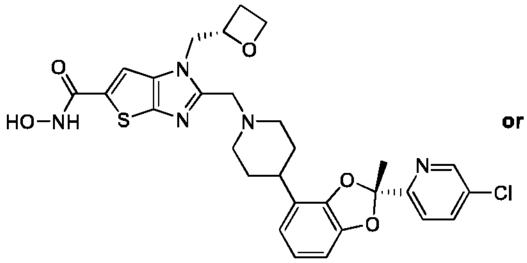

or

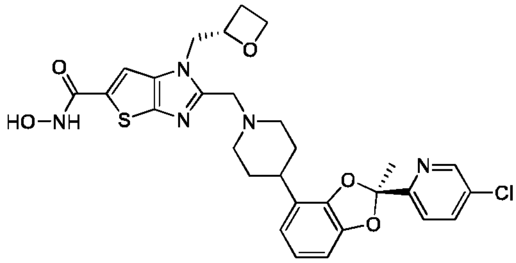

005

Synthetic route:

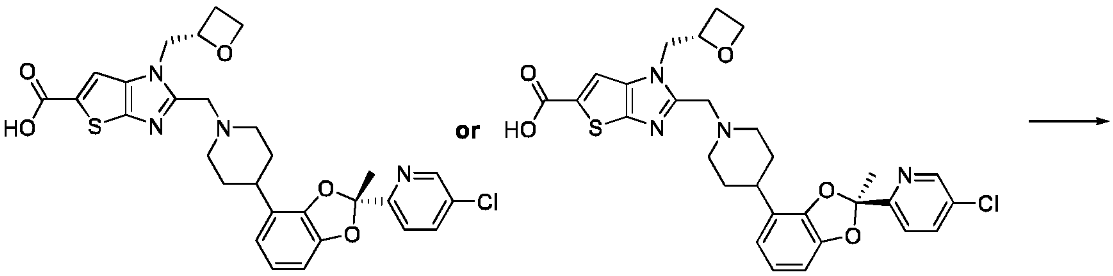

or

002

005

**002** (145.8 mg, 0.24 mmol, 1 *eq*), dichloromethane (12.0 mL), HATU (139.5 mg, 0.36 mmol, 1.5 *eq*), and DIPEA (277.0 μL, 1.59 mmol, 6.5 *eq)* were sequentially added to a dry reaction flask, and the reaction mixture was stirred at 24°C for 0.5 hours. Hydroxylamine hydrochloride (25.5 mg, 0.24 mmol, 1.5 *eq*) was then added thereto, and the reaction mixture was stirred for another 0.5 hours at 24°C. The reaction mixture was added with water (10 mL) and extracted with dichloromethane (10 mL * 3). The organic phase was collected after phase separation, washed with saturated brine (10 mL * 2), and dried over anhydrous sodium sulfate, and the resulting filtrate was concentrated under reduced pressure to obtain the crude product. The crude product was purified by thin-layer chromatography silica gel plate (dichloromethane: methanol = 10:1) and preparative high performance liquid chromatography, method: chromatographic column: Waters Xbridge BEH C18 100 * 30 mm * 10 μm; mobile phase: [water (ammonium bicarbonate)-acetonitrile]; gradient (acetonitrile)%: 30% to 60%, to obtain 005. $^1$H NMR (400 MHz, $CDCl_3$) $\delta$ *ppm* 8.62 (d, *J* = 2.0 Hz, 1H), 7.72 (dd, *J* = 2.0, 8.0 Hz, 1H), 7.65 (dd, *J* = 2.0, 8.0 Hz, 1H), 7.31 (s, 1H), 6.80 (q, *J* = 8.0 Hz, 1H), 6.75 (q, *J* = 8.0 Hz, 2H), 5.22 - 5.12 (m, 1H), 4.90 - 4.82 (m, 1H), 4.70 - 4.60 (m, 2H), 4.41 - 4.31 (m, 2H), 2.95 - 2.88 (m, 1H), 2.78 - 2.74 (m, 1H), 2.52 - 2.47 (m, 1H), 2.32 - 2.17 (m, 2H), 2.08 (s, 3H), 2.01 - 1.96 (m, 2H), 1.62 - 1.47 (m, 3H), 1.20 - 1.17 (m, 2H).

Example 6

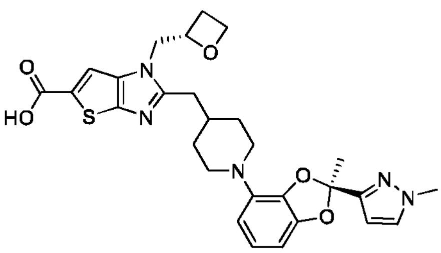

006 or 006'

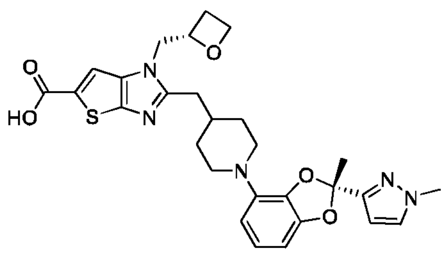

006' or 006

Synthetic route:

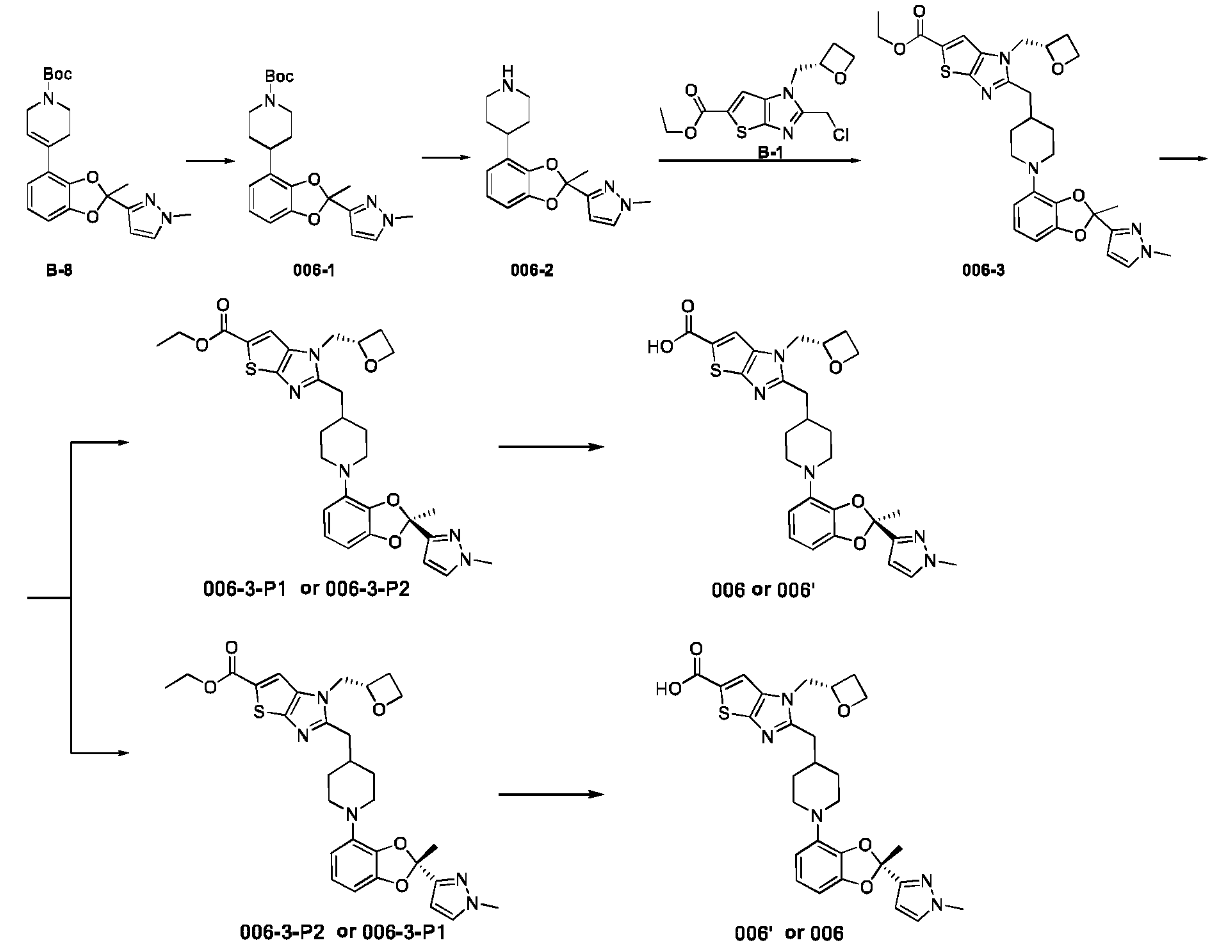


B-8 006-1 006-2 006-3

006-3-P1 or 006-3-P2

006 or 006'

006-3-P2 or 006-3-P1

006' or 006

Step 1: Synthesis of Compound **006-1**

Pd/C (5.0 g, 13.21 mmol, a content of 10%, 1 *eq*) was added to a reaction flask under argon atmosphere, then **B-8** (5.25 g, 13.21 mmol, 1 *eq*) and 60 mL of methanol were sequentially added thereto, and the reaction system was stirred for 12 hours under hydrogen atmosphere (50°C, 45 psi). The reaction mixture was filtered and the filtrate was concentrated. The crude product was purified by column chromatography (petroleum ether: ethyl acetate = 1:0 to 4:1) to obtain **006-1.** LCMS: m/z = 400.2 $[M+1]^+$.

Step 2: Synthesis of Compound **006-2**

**006-1** (1.0 g, 2.50 mmol, 1 *eq*) was dissolved in 10 mL of dichloromethane, then trifluoroacetic acid (25.03 mmol, 1.85 mL, 10 *eq*) was added dropwise thereto, and the reaction mixture was stirred for 2 hours at 24°C. The reaction mixture was added with saturated sodium bicarbonate to adjust the pH of the reaction mixture to about 7, and the reaction mixture was extracted with dichloromethane (50 mL * 2). The organic phases were combined, washed with 20 mL of water, dried over anhydrous sodium sulfate, and then filtered, and the filtrate was concentrated under reduced pressure to obtain a crude product. The crude product was purified by column chromatography (dichloromethane: methanol = 1:0 to 10:1) to obtain **006-2.** LCMS: m/z = 299.9 $[M+1]^+$.

Step 3: Synthesis of Compound **006-3**

**006-2** (0.1 g, 0.33 mmol, 1 *eq*) and **B-1** (95.0 mg, 0.3 mmol, 0.9 *eq*) were dissolved in 1 mL of acetonitrile, then potassium carbonate (0.18 g, 1.34 mmol, 4 *eq*) was added thereto, and the reaction mixture was heated to 60°C and

stirred for 12 hours. Acetonitrile was removed by rotary evaporation under reduced pressure, and the reaction mixture was then diluted with 10 mL of water, and extracted with ethyl acetate (30 mL * 2). The organic phases were combined, dried over anhydrous sodium sulfate, filtered, and the filtrate was concentrated under reduced pressure to obtain the crude product. The crude product was purified by column chromatography (dichloromethane: methanol = 1:0 to 10:1) to obtain **006-3.**

Step 4: Synthesis of compounds **006-3-P1** and **006-3-P2**

**006-3** was purified by preparative SFC (column model: DAICEL CHIRALPAK IC (250 mm * 30 mm * 10 μm); mobile phase: phase A was supercritical carbon dioxide, phase B was ethanol (0.2% ammonia water); gradient: 35% to 35%) to obtain compound **006-3-P1** and compound **006-3-P2,** which were subjected to chiral analysis SFC detection (column model: Chiralpak AD-3, 50 × 4.6 mm I.D., 3 μm); mobile phase: phase A was supercritical carbon dioxide, phase B was ethanol (0.2% ammonia water); gradient (B%): 5% to 35%), showing that the Rt for compound **006-3-P1** was 0.872 minutes, with an ee value of 100%, and the Rt for compound **006-3-P2** was 1.197 minutes, with an ee value of 98.4%. LCMS: m/z = 578.0 $[M+1]^+$.

Step 5: Synthesis of Compounds **006** and **006'**

**006-3-P1** (0.12 g, 0.21 mmol, 1 *eq*), methanol (0.5 mL), tetrahydrofuran (0.5 mL), water (0.5 mL), and lithium hydroxide monohydrate (26.2 mg, 0.62 mmol, 3 *eq*) were sequentially added to a dry reaction flask, and the reaction system was replaced with nitrogen and stirred for 3 hours at 28°C. The reaction mixture was concentrated under reduced pressure and purified by high performance liquid chromatography (method: chromatographic column: Phenomenex C18 75 * 30 mm * 3 μm; mobile phase: [water (ammonium bicarbonate)-acetonitrile]; gradient (acetonitrile)%: 30% to 60%) to obtain **006.** $^1$H NMR (400 MHz, $CDCl_3$) *δ ppm* 8.80 (br s, 1 H), 7.36 - 7.61 (m, 2 H), 6.62 - 6.80 (m, 3 H), 5.07 (br s, 1 H), 4.53 (br s, 2 H), 4.32 (br s, 1 H), 3.94 (s, 3 H), 3.86 - 4.14 (m, 2 H), 3.39 (br s, 4 H), 2.78 (br s, 1 H), 2.63 (br s, 1 H), 2.32 - 2.51 (m, 2 H), 2.08 (s, 3 H), 1.94 (br s, 4 H). LCMS: m/z = 550.3 $[M+1]^+$.

Using compound **006-3-P2** as raw material, compound **006'** was synthesized by referring to the synthesis method of step 5 of Example 6. $^1$H NMR (400 MHz, $CDCl_3$) *δ ppm* 8.81 (br s, 1 H), 7.36 - 7.61 (m, 2 H), 6.62 - 6.80 (m, 3 H), 5.05 (br s, 1 H), 4.53 (br s, 2 H), 4.32 (br s, 1 H), 3.94 (s, 3 H), 3.86 - 4.15 (m, 2 H), 3.39 (br s, 4 H), 2.76 (br s, 1 H), 2.61 (br s, 1 H), 2.32 - 2.51 (m, 2 H), 2.06 (s, 3 H), 1.92 (br s, 4 H). LCMS: m/z = 550.3 $[M+1]^+$.

**Example 7**

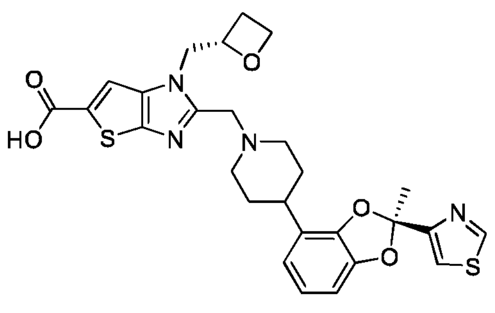

**007 or 007'**

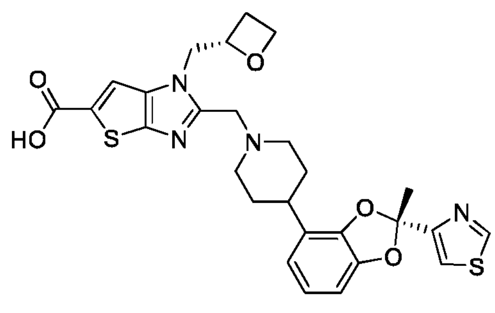

**007' or 007**

Synthetic route:

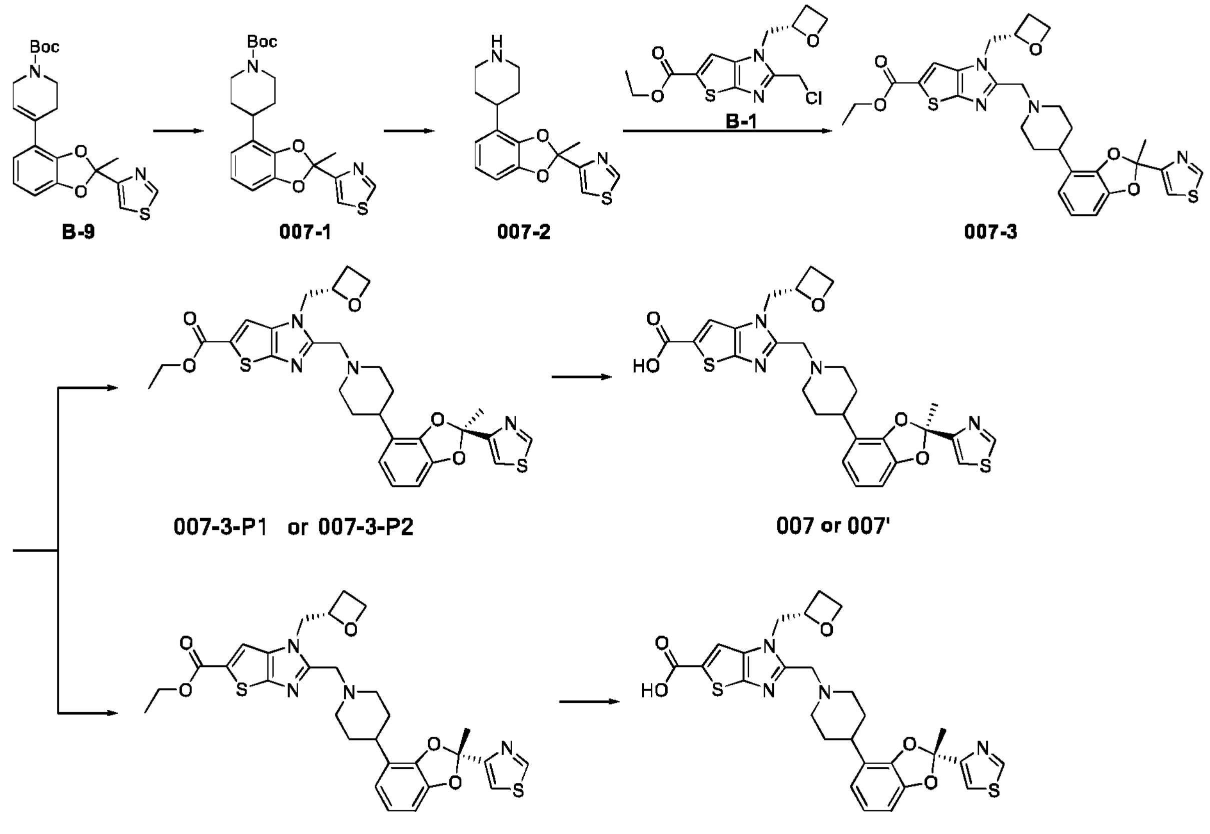

B-9 007-1 007-2 B-1 007-3

007-3-P1 or 007-3-P2 007 or 007'

007-3-P2 or 007-3-P1 007' or 007

Step 1: Synthesis of Compound **007-1**

Pd/C (5.0 g, 13.21 mmol, a content of 10%, 1 *eq*) was added to a reaction flask under argon atmosphere, then **B-9** (5.29 g, 13.21 mmol, 1 *eq*) and 60 mL of methanol were sequentially added thereto, and the reaction system was stirred for 12 hours under hydrogen atmosphere (50°C, 45psi). The reaction mixture was filtered and the filtrate was concentrated. The crude product was purified by column chromatography (petroleum ether: ethyl acetate= 1:0 to 6:1) to obtain **007-1.** LCMS: m/z = 403.2 $[M+1]^+$.

Step 2: Synthesis of Compound **007-2**

**007-1** (1.0 g, 2.50 mmol, 1 *eq)* was dissolved in 10 mL of dichloromethane, then trifluoroacetic acid (25.03 mmol, 1.85 mL, 10 *eq)* was added dropwise thereto, and the reaction mixture was stirred for 2 hours at 24°C. The reaction mixture was added with saturated sodium bicarbonate to adjust the pH of the reaction mixture to about 7, and the reaction mixture was extracted with dichloromethane (50 mL * 2). The organic phases were combined, washed with 20 mL of water, dried over anhydrous sodium sulfate, and then filtered, and the filtrate was concentrated under reduced pressure to obtain a crude product. The crude product was purified by column chromatography (dichloromethane: methanol = 1:0 to 20:1) to obtain **007-2.** LCMS: m/z = 303.1 $[M+1]^+$.

Step 3: Synthesis of Compound **007-3**

**007-2** (0.1 g, 0.33 mmol, 1 *eq*) and **B-1** (95.0 mg, 0.3 mmol, 0.9 *eq*) were dissolved in 1 mL of acetonitrile, then potassium carbonate (0.18 g, 1.34 mmol, 4 *eq*) was added thereto, and the reaction mixture was heated to 60°C and stirred for 12 hours. Acetonitrile was removed by rotary evaporation under reduced pressure, then the reaction mixture was diluted with 10 mL of water and extracted with ethyl acetate (30 mL * 2). The organic phases were combined, dried over anhydrous sodium sulfate, filtered, and the filtrate was concentrated under reduced pressure to obtain the crude product. The crude product was purified by column chromatography (dichloromethane: methanol = 1:0 to 10:1) to obtain **007-3.** LCMS: m/z = 581.2 $[M+1]^+$.

Step 4: Synthesis of compounds **007-3-P1** and **007-3-P2**

**007-3** was purified by preparative SFC (column model: DAICEL CHIRALPAK IC (250 mm * 30 mm * 10 μm); mobile phase: phase A was supercritical carbon dioxide, and phase B was methanol; gradient (B): 5% to 50%) to obtain compound **007-3-P1** and compound **007-3-P2,** which were subjected to chiral analysis SFC detection (column model: Chiralpak IG-3, 50 × 4.6 mm I.D., 3 μm); mobile phase: phase A was supercritical carbon dioxide, and phase B was methanol; gradient (B%): 5% to 50%), showing that the retention time for compound **007-3-P1** was 0.932 minutes, with an ee value of 100%, and the retention time for compound **007-3-P2** was 1.206 minutes, with an ee value of 97.8%.

Step 5: Synthesis of Compounds **007** and **007'**

**007-3-P1** (0.12 g, 0.21 mmol, 1 *eq),* methanol (0.5 mL), tetrahydrofuran (0.5 mL), water (0.5 mL), and lithium hydroxide monohydrate (26.2 mg, 0.62 mmol, 3 *eq)* were sequentially added to a dry reaction flask, and the reaction system was replaced with nitrogen and stirred for 3 hours at 28°C. The reaction mixture was concentrated under reduced pressure and then purified by preparative high performance liquid chromatography (method: column: Phenomenex C18 75 * 30 mm * 3 μm; mobile phase: [water (ammonium bicarbonate) - acetonitrile]; gradient (acetonitrile)%: 15% to 45%) to obtain **007.** $^1$H NMR (400 MHz, $CDCl_3$) *δ ppm* 8.80 (br s, 1 H), 7.36 - 7.66 (m, 2 H), 6.63 - 6.78 (m, 3 H), 5.07 (br s, 1 H), 4.18 - 4.60 (m, 4 H), 3.92 (br s, 2 H), 3.23 (br s, 1 H), 2.74 (br s, 6 H), 2.36 (br s, 2 H), 2.07 (br s, 3 H), 1.86 (br s, 2 H). LCMS: m/z = 553.2 $[M+1]^+$.

Using compound **007-3-P2** as raw material, compound **007'** was synthesized by referring to the synthesis method of step 5 of Example 7. $^1$H NMR (400 MHz, $CDCl_3$) *δ ppm* 8.82 (br s, 1 H), 7.36 - 7.64 (m, 2 H), 6.63 - 6.78 (m, 3 H), 5.06 (br s, 1 H), 4.18 - 4.58 (m, 4 H), 3.93 (br s, 2 H), 3.24 (br s, 1 H), 2.74 (br s, 6 H), 2.36 (br s, 2 H), 2.05 (br s, 3 H), 1.86 (br s, 2 H). LCMS: m/z = 553.2 $[M+1]^+$.

**Example 8**

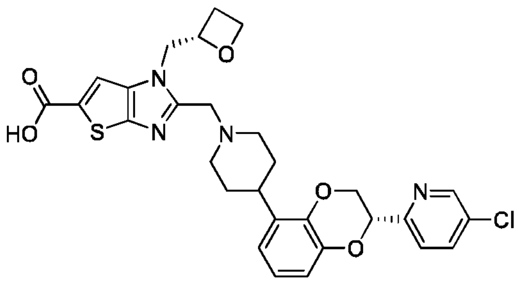

**008 or 008'**

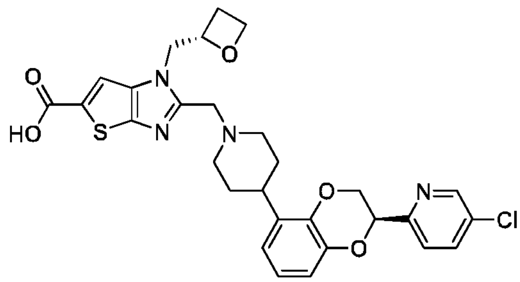

**008' or 008**

Synthetic route:

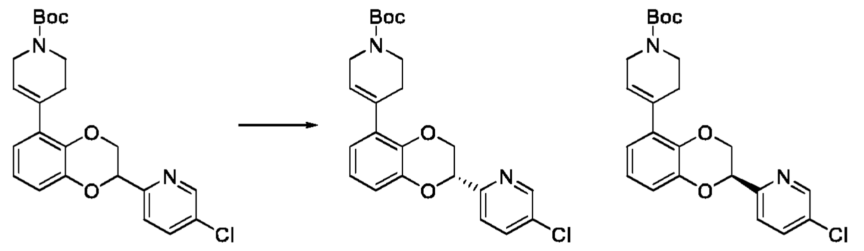

B-10 008-1-P1 or 008-1-P2 008-1-P2 or 008-1-P1

008-1-P1 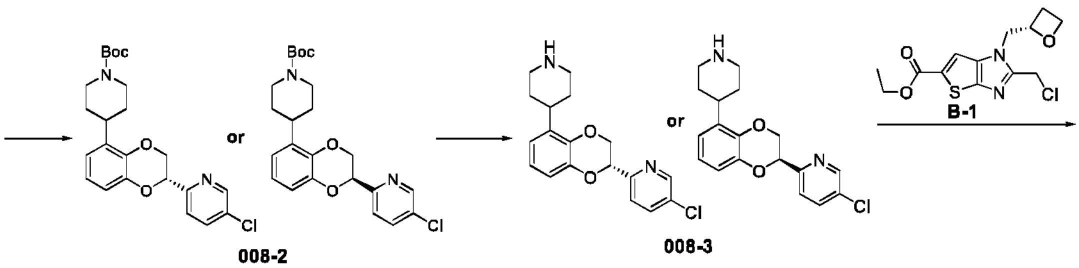


or 008-2 or 008-3

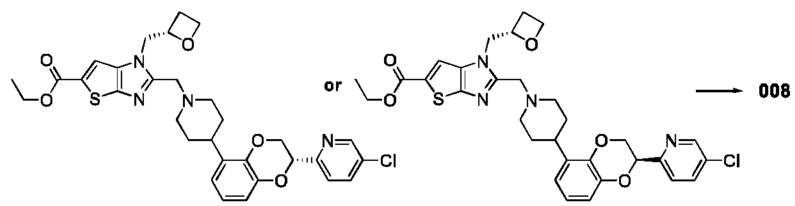

or 008

008-4

Step 1: Synthesis of **008-1-P1** and **008-1-P2**

**B-10** was subjected to chiral separation [separation method: chromatographic column: DAICEL CHIRALCEL OJ (250 mm * 30 mm, 10 μm); mobile phase: phase A was supercritical carbon dioxide, and phase B was methanol; gradient (B%): 30% to 30%] to obtain compound **008-1-P1** and compound **008-1-P2.**

Compound **008-1-P1:** Rt of 2.322 minutes, an ee value of 99.92%. Detection method: chromatographic column Chiralcel OJ-3, 150 × 4.6 mm I.D., 3 μm; mobile phase: phase A was supercritical carbon dioxide, and phase B was methanol (0.1% isopropylamine); gradient (B%): 10% to 10%. $^1H$ NMR (400 MHz, $CDCl_3$) $\delta$ *ppm* 8.58 (s, 1H), 7.73 (dd, *J* = 2.1, 8.4 Hz, 1H), 7.51 (d, *J* = 8.4 Hz, 1H), 7.00 - 6.93 (m, 1H), 6.91 - 6.85 (m, 1H), 6.83 - 6.75 (m, 1H), 5.83 (br s, 1H), 5.27 (br d, *J* = 5.9 Hz, 1H), 4.61 (td, *J* = 1.2, 11.3 Hz, 1H), 4.17 (dd, *J* = 7.5, 11.3 Hz, 1H), 4.05 (br s, 2H), 3.60 (br d, *J* = 3.5 Hz, 2H), 2.50 (br s, 2H), 1.50 (s, 9H). LCMS: m/z=451.2 $[M+ Na]^+$.

Compound **008-1-P2:** Rt of 2.642 minutes, an ee value of 98.58%. Detection method: chromatographic column Chiralcel OJ-3, 150 × 4.6 mm I.D., 3 μm; mobile phase: phase A was supercritical carbon dioxide, and phase B was methanol (0.1% isopropylamine); gradient (B%): 10% to 10%. $^1H$ NMR (400 MHz, $CDCl_3$) $\delta$ 8.57 (d, *J* = 2.1 Hz, 1H), 7.73 (dd, *J* = 2.5, 8.4 Hz, 1H), 7.51 (d, *J* = 8.5 Hz, 1H), 6.98 - 6.93 (m, 1H), 6.87 (t, *J* = 7.8 Hz, 1H), 6.82 - 6.78 (m, 1H), 5.83 (br s, 1H), 5.27 (dd, *J* = 2.5, 7.4 Hz, 1H), 4.61 (dd, *J* = 2.6, 11.3 Hz, 1H), 4.17 (dd, *J* = 7.5, 11.3 Hz, 1H), 4.05 (br s, 2H), 3.60 (br d, *J* = 3.6 Hz, 2H), 2.50 (br s, 2H), 1.50 (s, 9H). LCMS: m/z= 451.2 $[M+Na]^+$.

Step 2: Synthesis of **008-2**

**008-1-P1** (290 mg, 676.14 μmol, 1 *eq*), methanol (30 mL), and tris(triphenylphosphine)rhodium(I) chloride (62.56 mg, 67.61 μmol, 0.1 *eq*) were sequentially added to a dry reaction flask, and the reaction mixture was stirred for 24 hours under $H_2$ atmosphere (60°C, 50 psi). The reaction mixture was filtered with diatomite, and the filter cake was washed with methanol (50 mL). The filtrate was concentrated under reduced pressure. The crude product was purified by preparative thin-layer chromatography (petroleum ether: ethyl acetate = 5:1) to obtain **008-2.** $^1H$ NMR (400 MHz, $CDCl_3$) $\delta$ *ppm* 8.58 (d, *J* = 2.0 Hz, 1H), 7.73 (dd, *J* = 2.2, 8.3 Hz, 1H), 7.51 (d, *J* = 8.3 Hz, 1H), 6.92 - 6.85 (m, 2H), 6.82 - 6.75 (m, 1H), 5.27 (dd, *J* = 2.3, 7.2 Hz, 1H), 4.61 (dd, *J* = 2.3, 11.2 Hz, 1H), 4.22 - 4.10 (m, 5H), 3.10 - 3.00 (m, 1H), 2.90 - 2.73 (m, 2H), 1.87 - 1.79 (m, 1H), 1.78 - 1.70 (m, 1H), 1.49 (s, 9H). LCMS: m/z= 453.2 $[M+Na]^+$.

Step 3: Synthesis of **008-3**

Compound **008-2** (200 mg, 464.12 μmol, 1 *eq*), DCM (5 mL), and TFA (5.86 mmol, 433.79 μL, 12.62 *eq*) were added to a dry reaction flask, and the reaction system was replaced with nitrogen and stirred for 1 hour at 20°C. The reaction mixture was concentrated under reduced pressure to obtain the crude trifluoroacetate salt **008-3,** which was directly used in the next reaction step. LCMS: m/z = 331.1 $[M+H]^+$.

Step 4: Synthesis of **008-4**

**B-1** (146.07 mg, 464.02 μmol, 1 *eq*), **008-3** (153.5 mg, the crude trifluoroacetate salt), acetonitrile (5 mL), and potassium carbonate (192.39 mg, 1.39 mmol, 3 *eq*) were sequentially added to a dry reaction flask, and the reaction mixture was stirred for 12 hours at 60°C. Additional potassium carbonate (192.39 mg, 1.39 mmol, 3 *eq*) was added thereto, and the reaction mixture was stirred for another 2 hours at 60°C. The reaction mixture was cooled to room temperature, then quenched with water (10 mL), and extracted with ethyl acetate (3 * 10 mL). After phase separation, the organic phase was collected, sequentially washed with saturated brine (3 * 10 mL), dried over anhydrous sodium sulfate, and concentrated under reduced pressure to obtain a crude product. The crude product was purified by preparative thin-layer chromatography (petroleum ether: ethyl acetate = 1:1) to obtain **008-4.** $^1H$ NMR (400 MHz, $CDCl_3$) *δ ppm* 8.57 (d, *J* = 2.4 Hz, 1H), 7.78 - 7.69 (m, 2H), 7.54 - 7.48 (m, 1H), 6.96 - 6.76 (m, 2H), 5.32 - 5.13 (m, 2H), 4.70 - 4.52 (m, 4H), 4.45 - 4.33 (m, 3H), 4.24 - 4.08 (m, 2H), 3.87 - 3.77 (m, 2H), 3.07 - 2.88 (m, 3H), 2.81 - 2.68 (m, 1H), 2.59 - 2.40 (m, 1H), 2.36 - 2.18 (m, 2H), 1.94 - 1.63 (m, 4H), 1.39 (br t, *J* = 7.0 Hz, 3H). LCMS: m/z = 609.2 $[M+H]^+$.

Step 5: Synthesis of **008**

**008-4** (180 mg, 295.50 μmol, 1 *eq*), tetrahydrofuran (4 mL), methanol (4 mL), water (4 mL), and sodium hydroxide (130.02 mg, 3.25 mmol, 11 *eq*) were sequentially added to a dry reaction flask, and the reaction mixture was stirred for 4 hours at 20°C. The pH of the reaction mixture was adjusted to 2-3 with 2 N hydrochloric acid aqueous solution, and the reaction mixture was extracted with ethyl acetate (3 * 10 mL). After phase separation, the organic phase was collected, sequentially washed with saturated brine (3 * 10 mL), dried over anhydrous sodium sulfate, filtered, and the filtrate was concentrated under reduced pressure to obtain the crude product. The crude product was purified by preparative HPLC (chromatographic column: Phenomenex C18 80 * 40 mm * 3 μm; mobile phase: [water (ammonium bicarbonate)-acetonitrile]; B%: 20% to 40%, 8 minutes) to obtain compound **008.** Rt of 1.215 minutes, an ee value of 97.1%. Detection method: chromatographic column: Chiralcel OJ-3, 50 × 4.6 mm I.D., 3 μm; mobile phase: A: carbon dioxide, phase B: methanol (0.1% isopropylamine); gradient (B%): 5% to 50%. $^1H$ NMR (400 MHz, $CDCl_3$) *δ ppm* 8.57 (s, 1H), 7.72 (dd, *J* = 1.6, 8.4 Hz, 1H), 7.50 (br d, *J* = 8.3 Hz, 1H), 7.33 (br s, 1H), 6.93 - 6.77 (m, 3H), 5.26 (br d, *J*= 5.1 Hz, 1H), 5.15 - 5.03 (m, 1H), 4.76 - 4.48 (m, 4H), 4.43 - 4.29 (m, 1H), 4.18 (br dd, *J* = 7.3, 10.9 Hz, 2H), 4.07 (br d, *J* = 12.8 Hz, 1H), 3.69 - 3.56 (m, 2H), 3.15 - 2.93 (m, 1H), 2.77 - 2.53 (m, 3H), 2.47 - 2.32 (m, 1H), 2.08 - 1.83 (m, 4H). LCMS: m/z =581.1 $[M+H]^+$.

Using **008-1-P2** as raw material, compound **008'** was prepared by referring to steps 2 to 5 of Example 8. Rt of 1.750 minutes, an ee value of 100%. Detection method: chromatographic column: Chiralcel OJ-3, 50 × 4.6 mm I.D., 3 μm; mobile phase: A: carbon dioxide, phase B: methanol (0.1% isopropylamine); gradient (B%): 5% to 50%. $^1H$ NMR (400 MHz, $CDCl_3$) δ 8.57 (d, *J* = 1.8 Hz, 1H), 7.72 (dd, *J* = 2.0, 8.4 Hz, 1H), 7.50 (d, *J* = 8.4 Hz, 1H), 7.29 (s, 1H), 6.92 - 6.78 (m, 3H), 5.26 (br d, *J* = 5.7 Hz, 1H), 5.12 - 5.02 (m, 1H), 4.77 - 4.65 (m, 1H), 4.65 - 4.49 (m, 3H), 4.43 - 4.32 (m, 1H), 4.28 - 4.05 (m, 3H), 3.79 - 3.55 (m, 2H), 3.12 - 2.97 (m, 1H), 2.74 - 2.55 (m, 3H), 2.47 - 2.30 (m, 1H), 2.12 - 1.85 (m, 4H). LCMS: m/z =581.1 $[M+H]^+$.

## Example 9

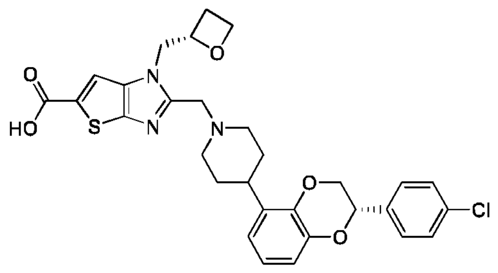

009 or 009'

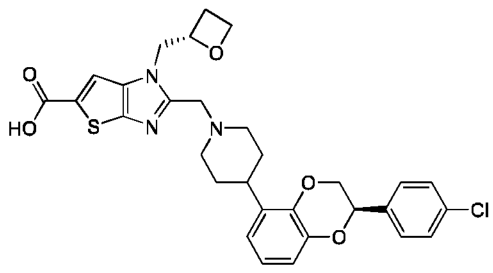

009' or 009

Synthetic route:

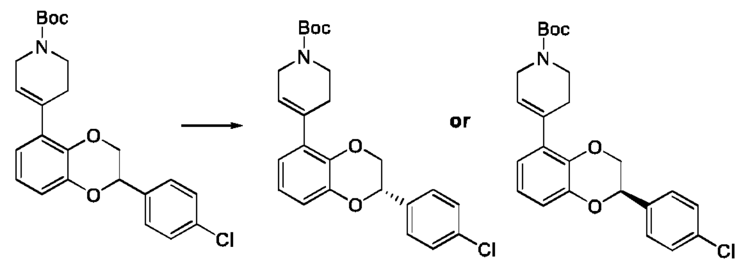


B-11 009-1-P1 or 009-1-P2 009-1-P2 or 009-1-P1

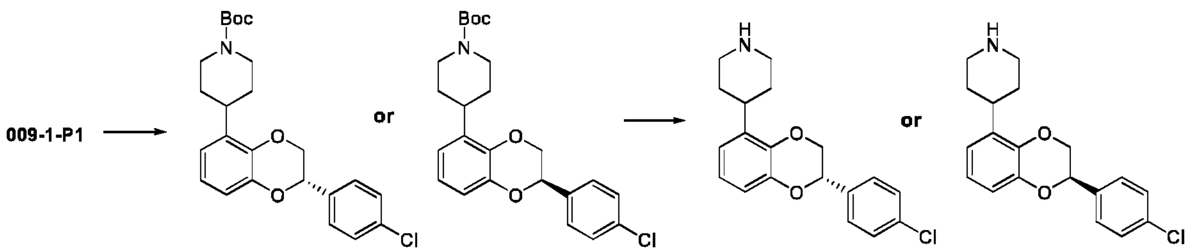


009-2 009-3

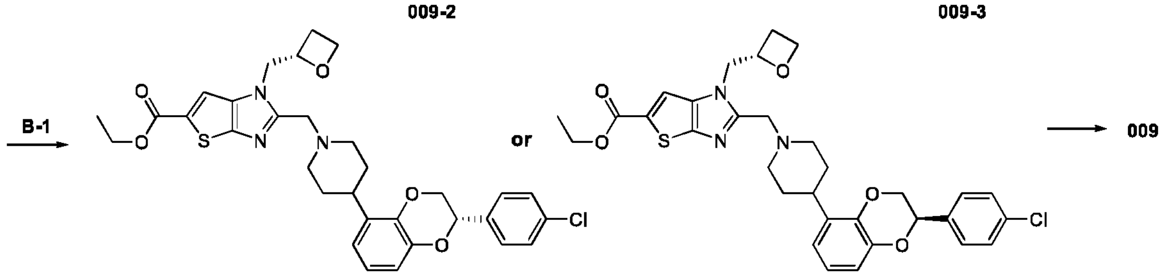


009-4

Step 1: Synthesis of **009-1-P1** and **009-1-P2**

B-11 was separated by chiral HPLC (chromatographic column: DAICEL CHIRALCEL OJ (250 mm * 30 mm, 10 μm); mobile phase: phase A was supercritical carbon dioxide, and phase B was methanol, gradient (B%): 35% to 35%) to obtain **009-1-P1** and **009-1-P2.**

Compound **009-1-P1** was detected by chiral analysis SFC (method: chromatographic column: Chiralcel OJ-3, 150 × 4.6 mm I.D., 3 μm; mobile phase: A: supercritical carbon dioxide, B: methanol (0.1% isopropanol)), with a retention time of 3.094 minutes, an ee value of 100%. $^1$H NMR (400 MHz,$CDCl_3$) *δ ppm* 7.45 - 7.34 (m, 4H), 6.95 - 6.90 (m, 1H), 6.86 (t, *J* = 7.8 Hz, 1H), 6.81 - 6.77 (m, 1H), 5.84 (br s, 1H), 5.11 (dd, *J* = 2.1, 8.7 Hz, 1H), 4.38 (dd, *J* = 2.3, 11.4 Hz, 1H), 4.07 (br s, 2H), 3.97 (dd, *J* = 8.9, 11.4 Hz, 1H), 3.62 (br s, 2H), 2.52 (br s, 2H), 1.50 (s, 9H). LCMS: m/z= 450.2 [M+Na]$^+$.

Compound **009-1-P2** was detected by chiral analysis SFC (method: chromatographic column: Chiralcel OJ-3,

150 × 4.6 mm I.D., 3 μm; mobile phase: A: supercritical carbon dioxide, B: methanol (0.1% isopropanol), gradient (B%): 35% to 35%, retention time of 3.754 minutes, an ee value of 100%). $^1$H NMR (400 MHz, $CDCl_3$) *δ ppm* 7.43 - 7.35 (m, 4H), 6.94 - 6.90 (m, 1H), 6.86 (t, *J* = 7.8 Hz, 1H), 6.81 - 6.77 (m, 1H), 5.84 (br s, 1H), 5.11 (dd, *J* = 2.2, 8.8 Hz, 1H), 4.38 (dd, *J* = 2.4, 11.5 Hz, 1H), 4.06 (br s, 2H), 3.97 (dd, *J* = 8.8, 11.4 Hz, 1H), 3.67 - 3.56 (m, 2H), 2.52 (br s, 2H), 1.50 (s, 9H). LCMS: m/z= 450.2 $[M+Na]^+$.

Step 2: Synthesis of **009-2**

**009-1-P1** (260 mg, 607.59 μmol, 1 *eq*) and tris(triphenylphosphine)rhodium(I) chloride (56.22 mg, 60.76 μmol, 0.1 *eq*) were dissolved in methanol (26 mL), and the reaction mixture was stirred for 24 hours under $H_2$ atmosphere (60°C, 50 psi). The reaction mixture was filtered with diatomite. The filter cake was washed with dichloromethane (10 mL), and the resulting filtrate was concentrated under reduced pressure to obtain crude product. The crude product was purified by preparative thin-layer chromatography (petroleum ether: ethyl acetate = 5:1) to obtain **009-2.** $^1$H NMR (400 MHz, $CDCl_3$) *δ ppm* 7.31 (q, *J* = 8.5 Hz, 4H), 6.79 (d, *J* = 4.5 Hz, 2H), 6.71 (br d, *J* = 4.6 Hz, 1H), 5.03 (dd, *J* = 1.9, 8.7 Hz, 1H), 4.36 - 4.26 (m, 1H), 4.16 (br d, *J* = 1.6 Hz, 1H), 3.89 (dd, *J* = 8.9, 11.3 Hz, 1H), 3.42 (s, 1H), 3.04 - 2.90 (m, 1H), 2.87 - 2.62 (m, 2H), 1.82 - 1.66 (m, 2H), 1.54 (dt, *J* = 3.9, 12.8 Hz, 2H), 1.47 - 1.28 (m, 9H). LCMS: m/z= 452.2 $[M+Na]^+$.

Step 3: Synthesis of **009-3**

Compound **009-2** (0.22 g, 0.51 mmol, 1 *eq*), dichloromethane (2 mL), and trifluoroacetic acid (14.86 mmol, 1.1 mL, 29.03 *eq*) were added to a dry reaction flask, and the reaction system was replaced with nitrogen and stirred for 1 hour at 20°C. The reaction mixture was then concentrated under reduced pressure to obtain the crude trifluoroacetate salt **009-3,** which was directly used in the next reaction step. LCMS: m/z=330.2 $[M+1]^+$.

Step 4: Synthesis of **009-4**

**B-1** (161.08 mg, 511.70 μmol, 1 *eq*), **009-3** (168.77 mg, the crude trifluoroacetate salt), and potassium carbonate (212.17 mg, 1.54 mmol, 3 *eq*) were dissolved in acetonitrile (5 mL), and the reaction mixture was stirred for 3 hours at 60°C. The reaction mixture was quenched with water (10 mL) and extracted with ethyl acetate (10 mL * 3). After phase separation, the organic phase was collected, sequentially washed with saturated brine (10 mL), dried over anhydrous sodium sulfate, and the filtrate was concentrated under reduced pressure to obtain the crude product, which was purified by preparative thin-layer chromatography (dichloromethane: methanol = 20:1) to obtain **009-4.** $^1$H NMR (400 MHz, $CDCl_3$) *δ ppm* 7.75 (s, 1H), 7.43 - 7.34 (m, 4H), 6.88 - 6.84 (m, 2H), 6.80 (br d, *J* = 4.5 Hz, 1H), 5.25 - 5.16 (m, 1H), 5.12 - 5.07 (m, 1H), 4.70 - 4.61 (m, 1H), 4.56 (br d, *J* = 3.5 Hz, 2H), 4.44 - 4.33 (m, 4H), 3.96 (dd, *J* = 9.0, 11.4 Hz, 1H), 3.82 (br s, 1H), 3.02 - 2.92 (m, 2H), 2.80 - 2.70 (m, 1H), 2.50 - 2.41 (m, 1H), 2.34 - 2.21 (m, 2H), 1.71 - 1.62 (m, 6H), 1.40 - 1.35 (m, 3H). LCMS: m/z=608.2 $[M+1]^+$.

Step 5: Synthesis of **009**

**009-4** (200 mg, 328.87 μmol, 1 *eq*) was dissolved in THF (1 mL), MeOH (1 mL), and $H_2O$ (1 mL), then lithium hydroxide monohydrate (41.40 mg, 986.61 μmol, 3 *eq*) was added thereto, and the reaction mixture was reacted at 20°C for 36 hours. The pH of the reaction mixture was adjusted to 2-3 with 2 N hydrochloric acid aqueous solution, and the reaction mixture was extracted with ethyl acetate (3 * 5 mL). After phase separation, the organic phase was collected, sequentially washed with saturated brine (3 * 50 mL), dried over anhydrous sodium sulfate, and the filtrate was concentrated under reduced pressure to obtain the crude product. The crude product was purified by preparative HPLC (chromatographic column: Waters XbridgeBEH C18 100 * 30 mm * 10 μm; mobile phase: [water (ammonium bicarbonate) - acetonitrile]; B%: 40% to 60%) to obtain **009.** Chiral analysis SFC detection (method: chromatographic column: Chiralcel OJ-3, 50 × 4.6 mm I.D., 3 μm; mobile phase: A: carbon dioxide, B: MeOH (0.1% isopropanol), gradient (B%): 5% to 50%) showed an Rt of 1.373 minutes and an ee value of 100%. $^1$H NMR (400 MHz, $CDCl_3$) *δ ppm* 7.41 - 7.35 (m, 4H), 7.32 (s, 1H), 6.86 - 6.82 (m, 3H), 5.09 (br d, *J* = 7.8 Hz, 2H), 4.69 (br dd, *J* = 4.8, 14.9 Hz, 1H), 4.59 - 4.52 (m, 2H), 4.38 (br d, *J* = 10.1 Hz, 2H), 4.28 - 4.17 (m, 1H), 4.14 - 4.05 (m, 1H), 4.00 - 3.91 (m, 1H), 3.76 - 3.66 (m, 1H), 3.64 - 3.54 (m, 1H), 3.17 - 2.96 (m, 1H), 2.78 - 2.54 (m, 3H), 2.49 - 2.31 (m, 1H), 2.13 - 1.86 (m, 4H). LCMS: m/z=580.1 $[M+1]^+$.

Using **009-1-P2** as raw material, compound **009'** was prepared by referring to steps 2 to 5 of Example 9. Rt of 1.914 minutes, an ee value of 100%. Detection method: chromatographic column: Chiralcel OJ-3, 50 × 4.6 mm I.D., 3 μm; mobile phase: A: carbon dioxide, phase B: methanol (0.1% isopropylamine); gradient (B%): 5% to 50%. $^1$H NMR (400 MHz, $CDCl_3$) *δ ppm* 7.38 (q, *J* = 8.3 Hz, 4H), 7.32 (s, 1H), 6.89 - 6.79 (m, 3H), 5.09 (br d, *J* = 7.4 Hz, 2H), 4.76 - 4.66 (m, 1H), 4.63 - 4.50 (m, 2H), 4.45 - 4.34 (m, 2H), 4.28 - 4.19 (m, 1H), 4.17 - 4.08 (m, 1H), 3.96 (dd, *J* = 9.1, 11.0 Hz, 1H), 3.73 - 3.70 (m, 1H), 3.64 - 3.61 (m, 1H), 3.13 - 3.00 (m, 1H), 2.75 - 2.61 (m, 3H), 2.47 - 2.33 (m, 1H), 2.17 -

1.89 (m, 4H). LCMS: m/z=580.1 [M+1]+.

**Example 10**

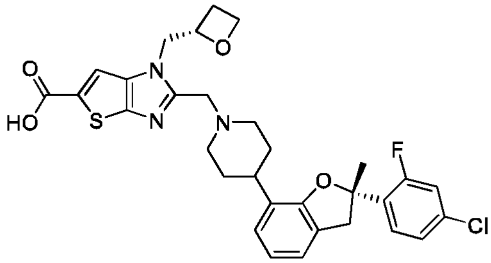

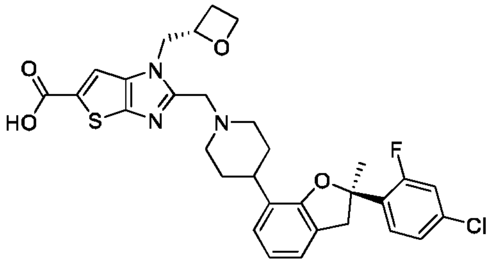

**010 or 010'** **010' or 010**

Synthetic route:

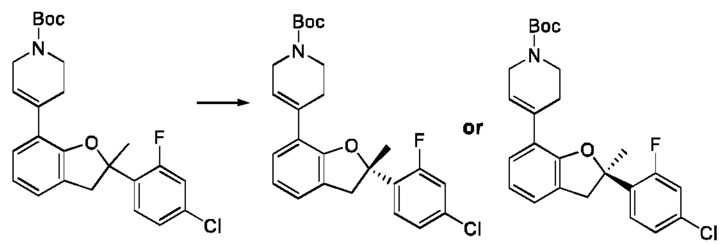


**B-12** **010-1-P1 or 010-1-P2** **010-1-P2 or 010-1-P1**

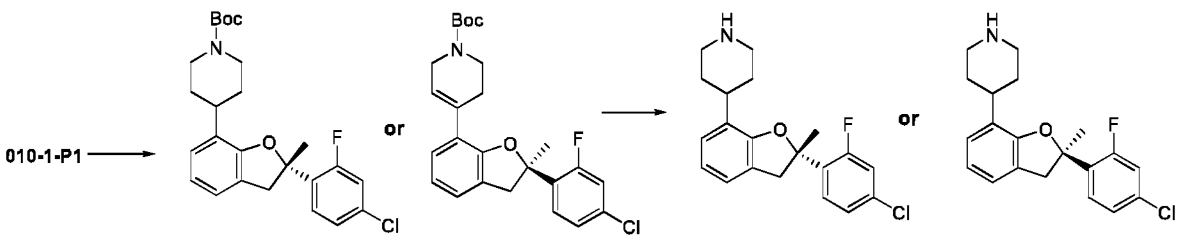


**010-2** **010-3**

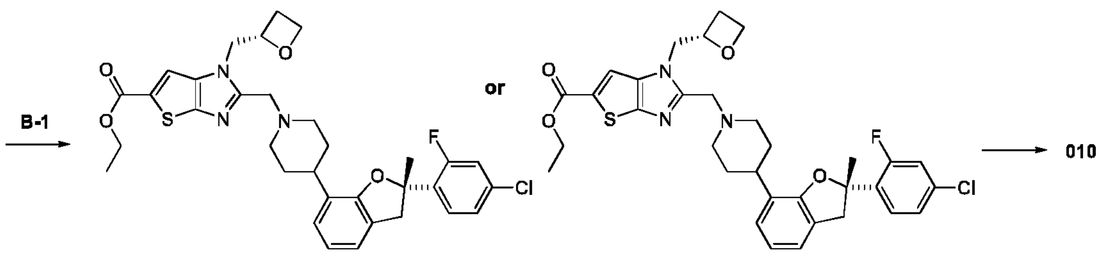


**010-4**

Step 1: Synthesis of **010-1-P1** and **010-1-P2**

**B-12** was separated by chiral HPLC [chromatographic column: DAICEL CHIRALCEL OJ (250 mm * 30 mm, 10 μm); mobile phase: A: supercritical carbon dioxide, B: methanol; gradient (B%): 30% to 50%] to obtain compound **010-1-P1** and compound **010-1-P2.**

**010-1-P1** was detected by chiral analysis SFC (method: chromatographic column: Chiralcel OJ-3, 150 × 4.6 mm I.D., 3 μm; phase A was supercritical carbon dioxide, phase B was methanol (0.1% isopropanol), gradient (B%): 30% to 50%), showing a retention time of 1.642 minutes and an ee value of 100%. LCMS: m/z=444.2 $[M+1]^+$.

**010-1-P2** was detected by chiral analysis SFC [method: chromatographic column: Chiralcel OJ-3, 150 × 4.6 mm I.D., 3 μm; mobile phase A was supercritical carbon dioxide, B was methanol (0.1% isopropanol), gradient (B%): 30% to 50%], showing a retention time of 2.025 minutes and an ee value of 100%. LCMS: m/z=444.2 $[M+1]^+$.

Step 2: Synthesis of **010-2**

**010-1-P1** (550.00 mg, 1.24 mmol, 1 *eq*) and tris(triphenylphosphine)rhodium(I) chloride (114.63 mg, 123.89 μmol, 0.1 *eq*) were dissolved in methanol (10 mL), and the reaction mixture was stirred for 16 hours under $H_2$ atmosphere (60°C, 50 psi). The reaction mixture was filtered, the filter cake was washed with methanol (10 mL), and the resulting filtrate was concentrated under reduced pressure to obtain the crude product. The crude product was purified by column chromatography (petroleum ether: ethyl acetate = 100:1 to 20:1) to obtain **010-2.** $^1H$ NMR (400 MHz, $CDCl_3$) $\delta$ *ppm* 7.62 - 7.53 (m, 1H), 7.19 - 7.10 (m, 2H), 7.05 - 6.92 (m, 3H), 6.85 (d, *J* = 7.5 Hz, 1H), 4.33 - 4.25 (m, 2H), 3.44 (br d, *J* = 13.5 Hz, 2H), 2.99 - 2.90 (m, 2H), 1.88 (br d, *J* = 11.9 Hz, 2H), 1.76 (br s, 2H), 1.55 (s, 3H), 1.50 (s, 9H). LCMS: m/z=468.1 $[M+23]^+$.

Step 3: Synthesis of **010-3**

**010-2** (90.00 mg, 201.82 μmol, 1 *eq*) was dissolved in dichloromethane (1 mL). The reaction system was replaced with nitrogen and cooled to 0°C, then trifluoroacetic acid (0.2 mL) was added thereto, and the reaction mixture was stirred for 1 hour at 20°C. The reaction mixture was directly concentrated under reduced pressure to obtain crude trifluoroacetate salt **010-3,** which was used directly in the next reaction step without purification. LCMS: m/z=346.2 $[M+1]^+$.

Step 4: Synthesis of **010-4**

**010-3** (69.8 mg, crude trifluoroacetate salt) and **B-1** (63.53 mg, 201.83 μmol, 1 *eq*) were dissolved in acetonitrile (1 mL), then potassium carbonate (111.58 mg, 807.32 μmol, 4 *eq*) was added thereto. The reaction system was replaced with nitrogen, and the reaction mixture was heated to 60°C and stirred for 10 hours. The reaction mixture was diluted with water (5 mL) and extracted with ethyl acetate (5 mL * 3). The resulting organic phase was washed with saturated brine (5 mL), dried over anhydrous sodium sulfate, filtered, and the filtrate was concentrated under reduced pressure to obtain the crude product. The crude product was purified by preparative thin-layer chromatography (PE:EA = 1:1) to obtain **010-4.** $^1H$ NMR (400 MHz, $CDCl_3$) $\delta$ *ppm* 7.84 - 7.69 (m, 1H), 7.61 - 7.47 (m, 1H), 7.17 - 7.09 (m, 2H), 7.01 (br s, 2H), 6.89 - 6.79 (m, 1H), 5.38 - 5.22 (m, 2H), 4.68 - 4.52 (m, 3H), 4.44 - 4.37 (m, 2H), 4.18 - 4.09 (m, 1H), 3.85 (br s, 2H), 3.54 - 3.41 (m, 2H), 3.12 - 2.89 (m, 2H), 2.88 - 2.56 (m, 2H), 2.49 - 2.29 (m, 2H), 1.74 (s, 3H), 1.56 (s, 3H), 1.44 - 1.38 (m, 3H), 1.29 - 1.27 (m, 1H). LCMS: m/z=624.2 $[M+1]^+$.

Step 5: Synthesis of **010**

**010-4** (70 mg, 112.15 μmol, 1 *eq*) and lithium hydroxide monohydrate (14.12 mg, 336.45 μmol, 3 *eq*) were dissolved in methanol (0.5 mL), tetrahydrofuran (0.5 mL), and water (0.5 mL), and the reaction system was replaced with nitrogen and stirred for 6 hours at 20°C. The reaction mixture was concentrated under reduced pressure and then purified by preparative HPLC (method: chromatographic column: Phenomenex C18 75 * 30 mm * 3 μm; mobile phase: [Water (ammonium bicarbonate)-acetonitrile]; gradient (acetonitrile)%: 25% to 55%) to obtain **010.** Chiral analysis SFC detection (instrument: CAS-TJ-ANA-SFC-H (Waters UPCC with SQ Detector2; chromatographic column: Chiralpak AD-3, 50 × 4.6 mm I.D., 3 μm; mobile phase: phase A was supercritical carbon dioxide, phase B was methanol (0.1% isopropanol), gradient: B% increased from 5% to 50% in 0.2 minutes and held for 2 minutes, and then decreased from 50% to 5% in 2.2 minutes) showed a retention time of 1.264 minutes and an ee value of 100%. $^1H$ NMR (400 MHz, $CD_3OD$) $\delta$ *ppm* 7.69 - 7.59 (m, 2H), 7.27 - 7.17 (m, 2H), 7.08 - 6.99 (m, 2H), 6.83 (s, 1H), 5.32 - 5.16 (m, 1H), 4.61 (s, 4H), 4.49 - 4.40 (m, 1H), 4.25 (s, 2H), 3.54 - 3.40 (m, 3H), 3.05 - 2.94 (m, 1H), 2.88 - 2.73 (m, 3H), 2.58 - 2.44 (m, 1H), 2.19 - 1.93 (m, 4H), 1.72 (s, 3H). LCMS: m/z=596.1 $[M+1]^+$.

Using **010-1-P2** as raw material, compound **010'** was prepared by referring to steps 2 to 5 of Example 10. Rt of 2.155 minutes, an ee value of 100%. Detection method: chromatographic column: Chiralcel OJ-3, 50 × 4.6 mm I.D., 3 μm; mobile phase: A: carbon dioxide, phase B: methanol (0.1% isopropylamine); gradient (B%): 5% to 50%. $^1H$ NMR (400 MHz, $CDCl_3$) $\delta$ *ppm* 7.67 - 7.60 (m, 2H), 7.26 - 7.17 (m, 2H), 7.06 - 6.99 (m, 2H), 6.85 - 6.79 (m, 1H), 5.26 - 5.17 (m, 1H), 4.72 - 4.54 (m, 3H), 4.41 (td, *J* = 5.9, 9.1 Hz, 1H), 4.25 (br s, 2H), 3.55 - 3.34 (m, 4H), 3.03 - 2.92 (m, 1H), 2.87 - 2.71 (m, 3H), 2.54 - 2.43 (m, 1H), 2.22 - 1.96 (m, 4H), 1.72 (s, 3H). LCMS: m/z=596.1 $[M+1]^+$.

**Example 11**

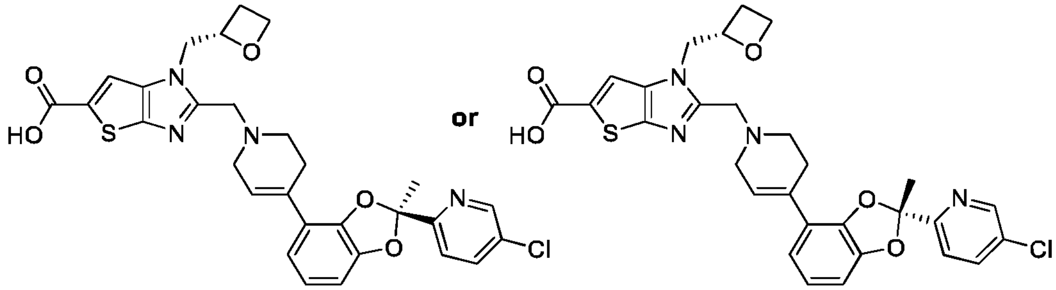

or

**011**

Synthetic route:

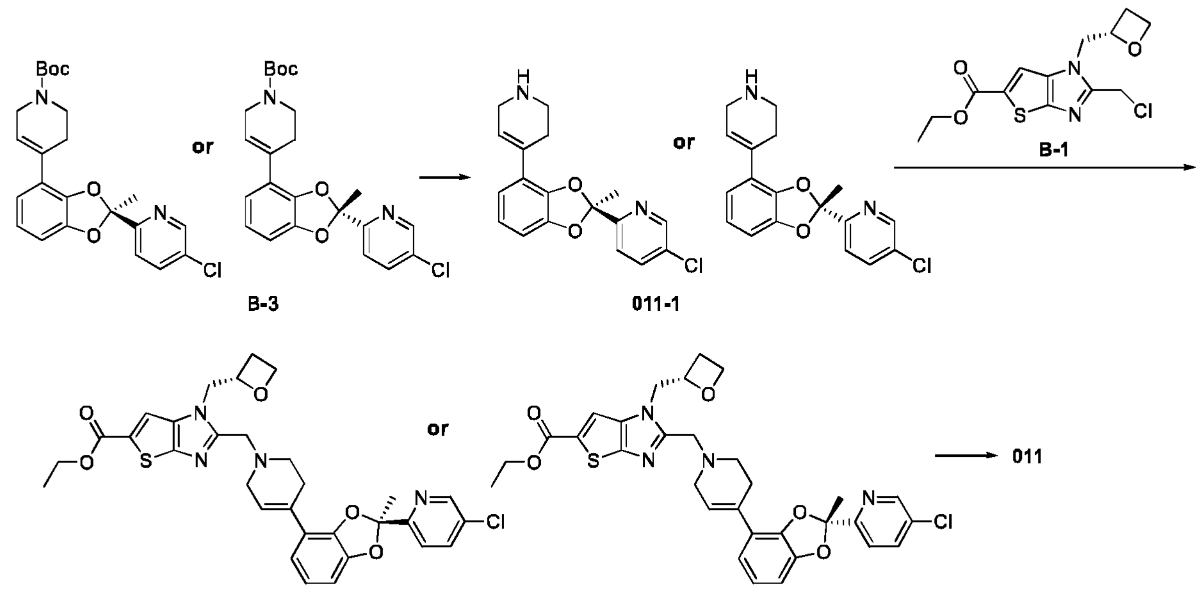

or

B-1

or

B-3

011-1

or

011

011-2

Step 1: Synthesis of **011-1**

**B-3** (0.4 g, 0.93 mmol, 1 *eq*) was dissolved in DCM (5 mL). The reaction system was replaced with nitrogen and cooled to 0°C, then trifluoroacetic acid (1.0 mL) was added thereto, and the reaction mixture was stirred for 1 hour at 20°C. The reaction mixture was directly concentrated under reduced pressure to obtain crude trifluoroacetate salt **011-1,** which was used directly in the next reaction step without purification. LCMS: m/z=329.1 $[M+1]^+$.

Step 2: Synthesis of **011-2**

**011-1** (0.24 g, crude trifluoroacetate salt) and **B-1** (0.13 g, 0.40 mmol, 1 *eq*) were dissolved in acetonitrile (2 mL), then potassium carbonate (0.22 g, 0.16 mmol, 4 *eq*) was added thereto. The reaction system was replaced with nitrogen, and the reaction mixture was heated to 60°C and stirred for 10 hours. The reaction mixture was diluted with water (10 mL) and extracted with ethyl acetate (3 * 15 mL). The resulting organic phase was washed with saturated brine (10 mL), dried over anhydrous sodium sulfate, filtered, and the resulting filtrate was concentrated under reduced pressure to obtain the crude product. The crude product was purified by preparative thin-layer chromatography (PE:EA

= 2:1) to obtain **011-2.** LCMS: m/z=607.2 $[M+1]^+$.

Step 3: Synthesis of **011**

**011-2** (0.2 g, 0.33 mmol, 1 *eq*) was dissolved in methanol (1.5 mL), tetrahydrofuran (1.5 mL), and water (1.5 mL), and after complete dissolution, lithium hydroxide monohydrate (69.1 mg, 1.65 mmol, 5 *eq*) was added thereto, and the reaction mixture was stirred for 3 hours at 20°C. The reaction mixture was concentrated under reduced pressure, then 10 mL of ethyl acetate was added thereto, and the pH of the reaction mixture was adjusted to about 2 with 5 mL of 1 M hydrochloric acid. The resulting organic phase was extracted and concentrated under reduced pressure to obtain the crude product. The crude product was purified by p-HPLC (method: chromatographic column: Phenomenex C18 75 * 30 mm * 3 μm; mobile phase: [Water (ammonium bicarbonate)-acetonitrile]; gradient (acetonitrile)%: 25% to 55%)], and the resulting fraction was lyophilized to obtain **011.** $^1$H NMR (400 MHz, DMSO-$d_6$) *δppm* 8.71-8.73 (m, 1H), 7.95-8.02 (m, 1H), 7.70-7.55 (m, 2H), 6.91-6.78 (m, 3H), 6.34-6.30 (m, 1H), 5.07-4.97 (m, 1H), 5.60-4.40 (m, 4H), 4.35-4.30 (m, 1H), 3.83-3.78 (m, 1H), 3.75-3.68 (m, 1H), 2.72-2.60 (m, 4H), 2.33-2.25 (m, 3H), 2.01 (s, 3H). LCMS: m/z=579.1 $[M+1]^+$.

**Example 12**

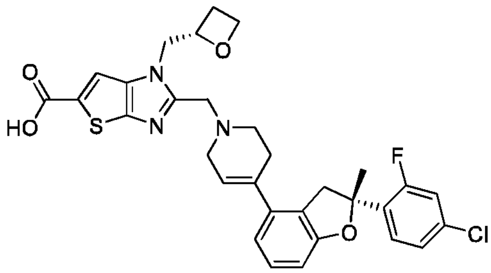

**012 or 012'**

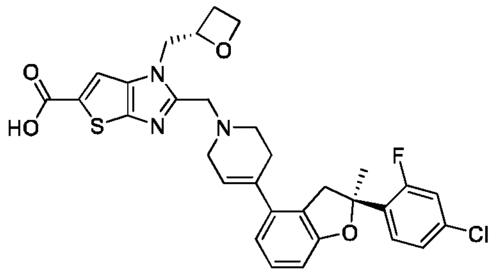

**012' or 012**

Synthetic route:

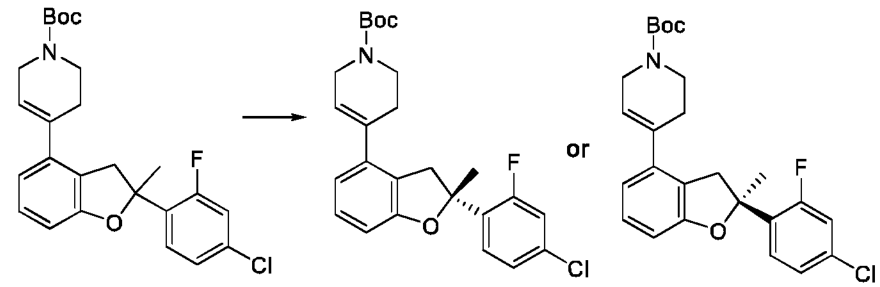

B-13 012-1-P1 or 012-1-P2 012-1-P2 or 012-1-P1

012-1-P1

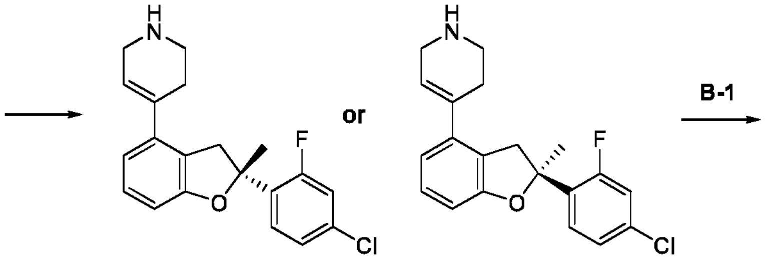


012-2

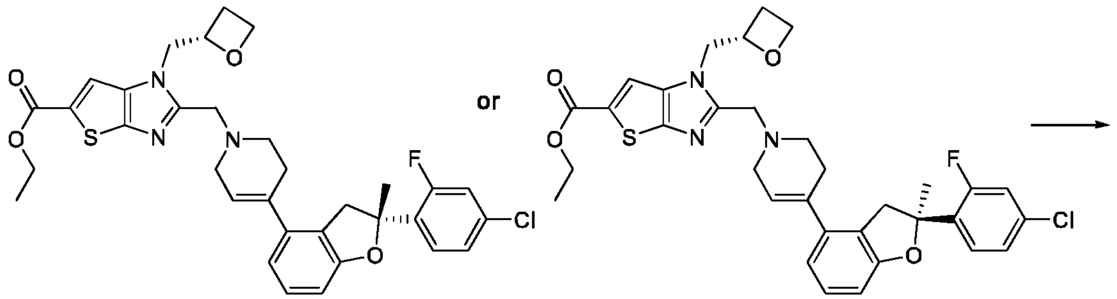

012

012-3

Step 1: Synthesis of **012-1-P1** and **012-1-P2**

**B-13** was separated by chiral HPLC (chromatographic column: DAICEL CHIRALCEL OJ (250 mm * 30 mm, 10 μm); mobile phase: A: carbon dioxide, phase B: methanol; gradient (B%): 10% to 50%) to obtain **012-1-P1** and **012-1-P2.**

**012-1-P1** was detected by chiral analysis SFC (method: chromatographic column: Chiralpak AD-3, 150 * 4.6 mm I.D., 3 μm; mobile phase: A: carbon dioxide, B: ethanol (0.1% isopropanol); gradient: (B%): 10% to 50%)), showing a retention time of 2.027 minutes and an ee value of 100%. LCMS: m/z=444.2 $[M+1]^+$.

**012-1-P2** was detected by chiral analysis SFC (method: chromatographic column: ChiralpakAD-3, 150 * 4.6 mm I.D., 3 μm; mobile phase: A: carbon dioxide, B: methanol (0.1% isopropylamine); gradient: (B%): 10% to 50%)), showing a retention time of 2.221 minutes and an ee value of 100%. LCMS: m/z=444.2 $[M+1]^+$.

Step 2: Synthesis of **012-2**

**012-1-P1** (0.2 g, 450.51 μmol, 1 *eq*) was dissolved in dichloromethane (4 mL). The reaction system was replaced with nitrogen, then trifluoroacetic acid (5.40 mmol, 0.4 mL, 11.99 *eq*) was added thereto, and the reaction mixture was stirred for 3 hours at 20°C. 10 mL of saturated sodium carbonate and 5 mL of dichloromethane were added to the system, the pH of the aqueous phase was about 9, and the phases were separated. The organic phase was dried over anhydrous sodium sulfate, filtered, and the filtrate was concentrated under reduced pressure to obtain **012-2,** which was used directly in the next reaction step without purification. LCMS: m/z=344.1 $[M+1]^+$.

Step 3: Synthesis of **012-3**

**012-2** (0.15 g, 433.73 μmol, 1 *eq*) and **B-1** (136.53 mg, 433.73 μmol, 1 *eq*) were dissolved in acetonitrile (4 mL), then potassium carbonate (179.83 mg, 1.30 mmol, 3 *eq*) was added thereto. The reaction system was replaced with nitrogen, and the reaction mixture was heated to 60°C and stirred for 2 hours. The system was filtered, the filter cake was washed with 10 mL of ethyl acetate, and the filtrate was concentrated under reduced pressure to obtain the crude product of **012-3.** LCMS: m/z=622.2 $[M+1]^+$.

Step 4: Synthesis of **012**

**012-3** (0.15 g, 241.10 μmol, 1 *eq*) and lithium hydroxide monohydrate (50.58 mg, 1.21 mmol, 5 *eq*) were dissolved in methanol (3.0 mL), tetrahydrofuran (3.0 mL), and water (1.0 mL). The reaction system was replaced with nitrogen, and the reaction mixture was stirred for 6 hours at 20°C. The system was concentrated under reduced pressure to remove the solvent, then 10 mL of ethyl acetate was added thereto, and the pH was adjusted to about 3 with 1 M HCl. The phases were separated, the organic phase was concentrated under reduced pressure to obtain the crude product, which was purified by preparative HPLC (chromatographic column: Phenomenex Luna C18 75 * 30 mm * 3 μm; mobile phase: [water (formic acid)-acetonitrile]; gradient (acetonitrile)%: 45% to 85%), and then separated by preparative HPLC (chromatographic column: Waters Xbridge BEH C18 100 * 30 mm * 10 μm; mobile phase: [Water (ammonium bicarbonate)-acetonitrile]; gradient (acetonitrile)%: 30% to 60%) to obtain **012.** Chiral analysis SFC detection (instrument: CAS-TJ-ANA-SFC-H (Waters UPCC with SQ Detector2; chromatographic column: Chiralpak AD-3, 50 × 4.6 mm I.D., 3 μm; mobile phase: A: carbon dioxide, B: ethanol (0.1% isopropanol); gradient: the content of B increased from 5% to 50% in 0.2 minutes and held for 2 minutes, and then decreased from 50% to 5% in 2.2 minutes) showed a retention time of 1.142 minutes and an ee value of 100%. $^1H$ NMR (400 MHz, DMSO-$d_4$) *δ ppm* 7.76 (s, 1H), 7.55 - 7.43 (m, 2H), 7.27 - 7.23 (m, 1H), 7.21 - 7.13 (m, 1H), 6.82 - 6.76 (m, 2H), 5.82 - 5.76 (m, 1H), 5.02 - 4.97 (m, 1H), 4.67 - 4.59 (m, 1H), 4.47 - 4.35 (m, 2H), 4.31 - 4.25 (m, 1H), 3.81 - 3.72 (m, 2H), 3.14 - 3.08 (m, 2H), 2.62 - 2.56 (m, 4H), 2.45 - 2.32 (m, 4H), 1.70 (s, 3H). LCMS: m/z=594.2 $[M+1]^+$.

Using **012-1-P2** as raw material, compound **012’** was prepared by referring to steps 2 to 4 of Example 12. Rt of 1.501 minutes, an ee value of 100%. Analytical method (chromatographic column: Chiralpak AD-3, 50 × 4.6 mm I.D., 3 μm mobile phase: A: carbon dioxide, B: MeOH (0.1% isopropanol); gradient: (B%): 5% to 50%). $^1H$ NMR (400 MHz, $CDCl_3$) *δ ppm* 7.74 (s, 1H), 7.54 - 7.44 (m, 2H), 7.26 - 7.22 (m, 1H), 7.21 - 7.12 (m, 1H), 6.82 - 6.74 (m, 2H), 5.82 - 5.75 (m, 1H), 5.03 - 4.97 (m, 1H), 4.68 - 4.59 (m, 1H), 4.50 - 4.36 (m, 2H), 4.31 - 4.25 (m, 1H), 3.81 - 3.71 (m, 2H), 3.14 - 3.08 (m, 2H), 2.62 - 2.56 (m, 4H), 2.47 - 2.33 (m, 4H), 1.70 (s, 3H). LCMS: m/z=594.2 $[M+1]^+$.

**Example 13**

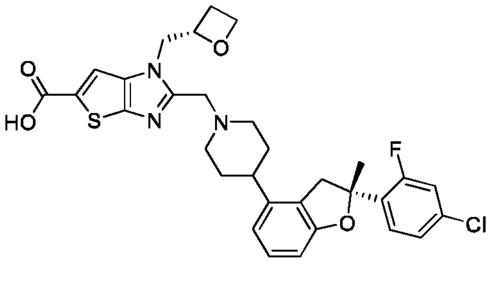

013 or 013'

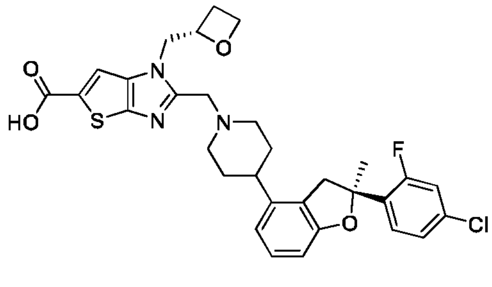

013' or 013

Synthetic route:

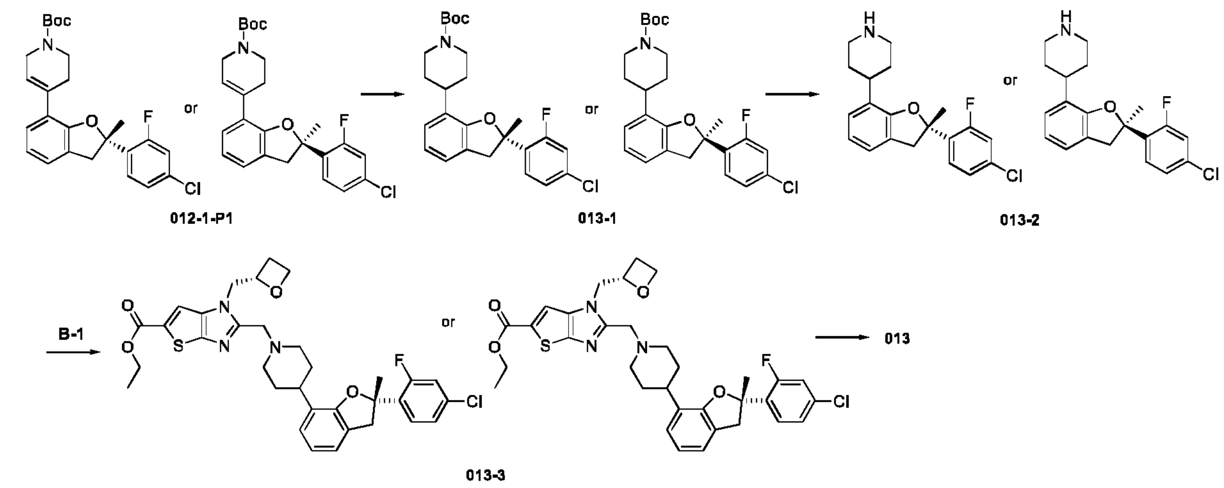


012-1-P1 or → 013-1 or → 013-2 or

013-3 or → 013

Step 1: Synthesis of **013-1**

**012-1-P1** (0.2 g, 450.51 μmol, 1 *eq*) and tris(triphenylphosphine)rhodium(I) chloride (0.1 g, 108.08 μmol, 0.24 *eq*) were dissolved in methanol (10 mL), and the reaction mixture was stirred for 16 hours under $H_2$ atmosphere (50°C, 50 psi), and concentrated under reduced pressure to obtain the crude product of **013-1,** which was used directly in the next reaction step. LCMS: m/z= 390.0 $[M-55]^+$.

Step 2: Synthesis of **013-2**

**013-1** (387.90 mg, 869.82 μmol, 1 *eq*) was dissolved in DCM (4 mL), then TFA(5.40 mmol, 400.00 μL, 6.21 *eq*) was added thereto, and the system was stirred for 16 hours at 20°C. A saturated sodium carbonate aqueous solution was added to the reaction mixture, and the pH of the aqueous phase was adjusted to about 8. After phase separation, the organic phase was dried over anhydrous sodium sulfate, filtered, and the resulting filtrate was concentrated to obtain **013-2.** LCMS: m/z=346.0 $[M+1]^+$.

Step 3: Synthesis of **013-3**

**013-2** (0.1 g, 289.15 μmol, 1 *eq*) and **B-1** (110 mg, 349.44 μmol, 1.21 *eq*) were dissolved in acetonitrile (4 mL), then potassium carbonate (199.81 mg, 1.45 mmol, 5 *eq*) was added thereto. The reaction system was replaced with nitrogen, and the reaction mixture was heated to 60°C and stirred for 2 hours. The reaction mixture was diluted with water (10 mL) and extracted with ethyl acetate (2 * 15 mL). The resulting organic phase was washed with saturated brine (10 mL), dried over anhydrous sodium sulfate, filtered, and the resulting filtrate was concentrated under reduced pressure to obtain the crude product of **013-3.** LCMS: m/z=624.2 $[M+1]^+$.

Step 4: Synthesis of **013**

**013-3** (0.3 g, 480.64 μmol, 1 *eq*) was dissolved in MeOH (3 mL), $H_2O$ (1 mL), and THF (3 mL), then lithium hydroxide monohydrate (100.85 mg, 2.40 mmol, 5 *eq*) was added thereto, and the system was stirred for 16 hours at 20°C. The system was concentrated under reduced pressure to remove the solvent, then 10 mL of ethyl acetate was added thereto, and the pH was adjusted to about 3 with 1 M HCl. The phases were separated, and the organic phase was concentrated under reduced pressure to obtain the crude product. The crude product was purified by preparative HPLC (chromatographic column: Phenomenex Luna C18 75 * 30 mm * 3 μm; mobile phase: [water (formic acid)-acetonitrile]; gradient (acetonitrile)%: 45% to 85%), and then purified by preparative HPLC (chromatographic column: Waters Xbridge BEH C18 100 * 30 mm * 10 μm; mobile phase: [Water (ammonium bicarbonate)-acetonitrile]; gradient (acetonitrile)%: 30% to 60%)] to obtain **013.** $^1$H NMR (400 MHz, DMSO-$d_4$) *δ ppm* 7.76 (s, 1H), 7.55 - 7.45 (m, 2H), 7.29 - 7.25 (m, 1H), 7.23 - 7.18 (m, 1H), 6.78 - 6.62 (m, 2H), 5.06 - 5.01 (m, 1H),4.71 - 4.65 (m, 1H), 4.55 - 4.45 (m, 2H), 4.41 - 4.32 (m, 1H), 3.83 - 3.65 (m, 2H), 3.52 - 3.45 (m, 1H), 2.93 - 2.82 (m, 4H), 2.21 - 2.05 (m, 4H), 1.72 - 1.61 (m, 7H). LCMS: m/z=596.1 $[M+1]^+$.

Using **012-1-P2** as raw material, compound **013'** was prepared by referring to steps 1 to 4 of Example 13. $^1$H NMR (400 MHz, $CDCl_3$) *δ ppm* 7.74 (s, 1H), 7.53 - 7.44 (m, 2H), 7.29 - 7.25 (m, 1H), 7.23 - 7.18 (m, 1H), 6.78 - 6.62 (m, 2H), 5.06 - 5.01 (m, 1H),4.71 - 4.65 (m, 1H), 4.55 - 4.45 (m, 2H), 4.41 - 4.32 (m, 1H), 3.82 - 3.62 (m, 2H), 3.52 - 3.45

(m, 1H), 2.93 - 2.82 (m, 4H), 2.21 - 2.05 (m, 4H), 1.72 - 1.61 (m, 7H). LCMS: m/z=596.1 $[M+1]^+$.

**Experimental example 1: *In vitro* cell activity assay**

**1. Materials**

1) Cell line

The host cell HEK293 for GLP-1 was constructed by WuXi AppTec (Shanghai) Co., Ltd.

2) Reagents

cAMP Detection Kit, Cisbio (Cat # 62AM4PEJ); 1M HEPES, Invitrogen (Cat # 15630-106); 1X HBSS, Invitrogen (Cat # 14025); BSA, Sigma (Cat # B2064); IBMX, Sigma (Cat # I5879); Exenatide, Hao Yuan (HY-13443A).

3) Instruments

OptiPlate-384, White, PerkinElmer (Cat # 6007290); 384 well plate for Echo, Labcyte (Cat#P-05525); EnVision, PerkinElmer; Vi-cell counter, Beckman (Cat# Vi-CELL™ XR Cell Viability Analyzer).

4) Compound preparation

The compounds were prepared into a working solution with a concentration of 30 μM using DMSO. In this assay, each sample was used in an amount of 5 μL.

5) Experimental buffers

24.5 mL of Hanks Buffer Saline Solution (HBSS), final concentration: 1x;
125 μL of HEPES 1M, final concentration: 5 mM;
333 μL of 7.5% BSA Solution, final concentration: 0.1%;
25 μL of IBMX 500 mmol/L, final concentration: 0.5 mmol/L.

6) Preparation of assay reagents

Preparation of cAMP assay reagent: 250 μL of cAMP-D2 and 250 μL of anti-cAMP cryptate reagent were added to 4 mL of lysis buffer and mixed gently.

**2. Experimental methods**

a) Preparation of compound plate:

The compound to be tested was diluted 3-fold at 10 points with a starting concentration of 30 μM, and the dilution was completed using Bravo.
Exenatide, used as a control compound, was diluted 3-fold at 10 points with a starting concentration of 500 nM, and the dilution was completed using Bravo.

b) Compound transferring:

1) 100 nL of compound was transferred to an OptiPlate-384 plate using Echo.
2) The OptiPlate-384 plate was centrifuged at 1000 rpm for 5 seconds.

c) Preparation of cell suspension

1) A GLP-1 cell cryopreservation tube was rapidly thawed in a 37°C water bath.
2) The cell suspension was transferred to a 15 mL Transfer centrifuge tube and gently rinsed with 10 mL of HBSS.
3) The centrifuge tube was centrifuged at 1000 rpm for 1 minute at room temperature.
4) The supernatant was discarded.
5) The cells at the bottom were gently dispersed, then rinsed with 10 mL of HBSS, centrifuged to sedimentation, and finally resuspended in the experimental buffer.
6) The cell density and activity were measured using Vi-cell.
7) The concentration of GLP-1 cells was diluted to 2.0 * $10^5$ /mL with the experimental buffer.
8) 100 nL of the diluted cell suspension was transfered to the OptiPlate-384 plate.
9) The plate was incubated at room temperature for 30 minutes.

d) The assay reagent was added:

1) 10 μL of 800 nM gradient-diluted cAMP standard was added to the empty wells of the OptiPlate-384 plate.
2) 10 μL of cAMP assay reagent was added.
3) The OptiPlate-384 plate was covered with TopSeal-A film and incubated for 60 minutes at room temperature.

TopSeal-A was removed and the result was read in EnVision.
The experimental results are shown in Table 1:

Table 1 Assay results of cell activity *in vitro*

| Compound | Human-GLP-1, $EC_{50}$ (nM) |
|---|---|
| **001** | 0.54 |
| **002** | 0.88 |
| **003** | 0.51 |
| **006** | 4.81 |
| **008** | 2.90 |
| **009** | 1.49 |
| **011** | 1.20 |

Conclusion: The compounds of the present disclosure exhibit potent agonistic ability on GLP-1 receptors.

**Experimental example 2: PK parameter testing *in vitro* - study on time-dependent inhibition (TDI)**

Testing purposes

The time-dependent inhibitory effect of the test compound on the activity of human liver microsomal cytochrome P450 isoenzyme CYP2C19 was determined.

Experimental method

The experiment was divided into two groups. In the first group, human liver microsomes (HLM) were used as the incubation system. The test articles with a series of concentrations were added to the incubation system, followed by the addition of a coenzyme factor (NADPH) solution. The preincubation was carried out at 37°C for 30 minutes. After preincubation, a probe substrate solution was added thereto. After a certain period of incubation, the reaction was terminated. The amount of probe substrate metabolites generated in the incubation solution was determined, and the enzyme activity was calculated. In the second group, human liver microsomes (HLM) were used as the incubation system. The test articles with a series of concentrations were added to the incubation system, followed by the addition of potassium phosphate buffer. The preincubation was carried out at 37°C for 30 minutes. After preincubation, a mixture of NADPH and the probe substrate was added thereto. After a certain period of incubation, the reaction was terminated. The amount of probe substrate metabolites generated in the incubation solution was determined, and the enzyme activity

was calculated.

First, the test compound (10.0 mM) was diluted by gradient to prepare a working solution (100× the final concentration) with concentrations of 5.00, 1.65, 0.500, 0.165, 0.0500, 0.0165, and 0.00500 mM, respectively. Simultaneously, working solutions were respectively prepared for positive inhibitors of the P450 isoenzyme CYP2C19, probe substrate, and NADPH. Human liver microsomes, stored in a refrigerator at a temperature below -60°C, were thawed on ice, and once fully dissolved, were diluted with potassium phosphate buffer (PB) to prepare a working solution of a specific concentration (0.169 mg/mL).

Then, 147.5 μL of human liver microsomal working solution was added to the reaction plate, and the reaction plate was placed on ice for use; at this time, 2.50 μL of the test compound (N = 1) and the working solution (N = 2) of the positive control inhibitor at various concentrations were added to the corresponding wells, and the corresponding organic solvent was added to the group without inhibitors (test compound or positive inhibitor); the reaction plate was incubated for 10 minutes at 37°C, and then 50.0 μL of NADPH solution or potassium phosphate buffer was added to reaction wells of the first or second group, respectively, to start the reaction; the reaction plate was pre-incubated at 37°C for 30 minutes; 50.0 μL of the substrate solution or a mixture of NADPH and the substrate was added to the reaction wells of the first or second group, respectively, to start the reaction. 20 minutes later, 250 μL of precooled acetonitrile solution (containing internal standards of 200 ng/mL tolbutamide and labetalol) was added to terminate the reaction; the reaction plate was placed on a shaker and shaken for 10 minutes to mix uniformly; then, the plate was centrifuged for 20 minutes at 4°C and 4000 rpm; 200 μL of the supernatant was added to 200 μL of water to dilute the sample; the plate was finally sealed and shaken for 10 minutes to mix uniformly; the sample was then analyzed by LC/MS/MS.

Experimental results: as shown in Table 2.

**Table 2 *In vitro* TDI test results**

| CYP enzyme substrate | Compound | $IC_{50}$ (μM) | |
|---|---|---|---|
| CYP2C19 | 002 | (-)NADPH | >50 |
| | | (+)NADPH | 29.2 |
| | 003 | (-)NADPH | >50 |
| | | (+)NADPH | >50 |

Conclusion: The compounds of the present disclosure have a low risk of time-dependent inhibition of human liver microsomal cytochrome P450 isoenzyme 2C19.

**Experimental example 3: *in vitro* PK parameter testing - HMS CLint (liver) test**

Experimental purpose: to test the metabolic stability of the test sample in human and rat liver cells.

Experimental materials:

(1) test compound (10 mM), reference substance: 7-ethoxycoumarin (30 mM), 7-hydroxycoumarin (reference substance, 30 mM);

mouse hepatocytes with cell viability of 73.8%, supplier Cat No.: BioreclamationIVTM005052;
rat hepatocytes with cell viability of 78.3%, supplier Cat No.: BioreclamationIVTM00005-T;
canine hepatocytes with cell viability of 80.2%, supplier Cat No.: BioreclamationIVTM00205;
monkey liver cells with cell viability of 80.9%, supplier Cat No.: RILD HP-SXH-02M;
human hepatocytes with cell viability of 94.6%, supplier Cat No.: Bioreclamation IVTX008001.

(2) Buffer system:

Thawing medium: Williams' medium E containing 5% fetal bovine serum, 30% Percoll solution, and other auxiliary materials.
Incubation medium: Williams' medium E (without phenol red) containing 2 mM L-glutamine and 25 mM 4-(2-hydroxyethyl)-1-piperazineethanesulfonic acid.
Termination solution: acetonitrile containing 200 ng/mL tolbutamide and labetalol as internal standard.

Dilution solution: ultrapure water.

Experimental method:

1) An accurate amount of the positive control compound was dissolved in dimethyl sulfoxide (DMSO) to form a 30 mM solution
2) 10 mM of the test compound and 30 mM of the positive control compound were diluted into 1 mM and 3 mM, respectively, in a 96-well plate using DMSO.
3) 1 mM of the test compound and 3 mM of the positive control compound were diluted into 100 μM and 300 μM quantitative solutions using acetonitrile.
4) The frozen cells were thawed, separated, and suspended in culture medium, and then diluted to 0.5 × $10^6$ cells/mL using preheated culture medium.
5) 198 μL of preheated cell suspension was added to a 96-well plate.
6) 100 μL of termination solution (acetonitrile containing 200 ng/mL of tolbutamide and 200 ng/mL of labetalol as internal standards) was transfered in a set of pre-labeled 96-well plates.
7) 2 μL of 100 μM test compound or 300 μM positive control quantification solution was added, in duplicate, to each well of the 96-well plate.
8) T0 samples were mixed for about 1 minute to achieve a uniform suspension, then 20 μL of each sample was immediately transferred into wells containing 100μL of ice-cold termination solution, followed by mixing.
9) All plates were incubated at 37°C in a 95% humidified incubator in 5% $CO_2$, and the reaction was started with a constant shaking at about 600 rpm.
10) At the time points of 15 minutes, 30 minutes, 60 minutes, and 90 minutes, the samples were mixed, and then at each time point, 20 μL of each sample was transferred to the wells containing 100 μL of ice-cold termination solution and mixed.
11) Medium control (MC) sample plates were prepared at T0 and T90 (labeled T0-MC and T90-MC) by adding the same ingredients except the cell suspension to each well. A final concentration table was generated.
12) At each corresponding time point, the reaction was terminated by removing the plate from the incubator and mixing it with 100 μL of ice-cold termination solution.
13) The plate was immediately vortexed and shaken on a plate shaker for 10 minutes at 500 rpm. Then, all sample plates were centrifuged at 3220 × g for 20 minutes at 4°C.
14) After centrifugation, the supernatant from the 35 μL/well sample plate was transferred to another set of pre-labeled 96-well plates, and 70 μL of ultrapure water was added to the 96-well plates according to the plate diagram.
15) The analytical plate was sealed and stored at 4°C until LC-MS-MS analysis.

The residual rates of the test compound and the control compound were determined using the following formula:

$$\text{Residual rate (\%)} = \frac{\text{peak area ratio of compound to internal standard at any time point}}{\text{peak area ratio of compound to internal standard at 0 min}} \times 100\%$$

The elimination rate constant (k) of the test compound and the control compound in hepatocytes was calculated by plotting the logarithm of the residual rate against time. The half-life ($T_{1/2}$) and the *in vitro* intrinsic clearance rate ($CL_{int}$) were obtained from the elimination rate constant *k*, using the following formulas:

$$T_{1/2} = 0.693 / k;$$

$$CL_{int\ (hep)} = k \text{ / cells per milliliter (million cells / mL)}$$

$CL_{int(liver)} = CL_{int(hep)}$ × ratio of liver weight to body weight × number of hepatocytes per gram of liver

The parameters of the various species in the formula are shown in Table 3:

Table 3 Parameters of various species

| Species | Ratio of liver weight to body weight (g/kg) | Hepatic blood flow ($Q_h$) (mL/min/kg) | Number of hepatocytes (Number of cells per gram of liver) |
|---|---|---|---|
| Rat | 40 | 55.2 | $117 \times 10^6$ |
| Dog | 32 | 30.9 | $215 \times 10^6$ |
| Monkey | 30 | 43.6 | $120 \times 10^6$ |
| Human | 20 | 20.7 | $139 \times 10^6$ |

Experimental results: the experimental results are shown in Table 4.

Table 4 Results of HMS tests for different species

| **Compound** | **HMS (mL/min/kg)** | | | |
|---|---|---|---|---|
| | **Human** | **Cynomolgus monkeys** | **Dog** | **Rat** |
| **002** | <17.8 | <23.0 | 115.4 | 138.7 |
| **003** | 19.4 | 41.6 | 58.7 | 5.1 |

Conclusion: The compounds of the present disclosure are slowly metabolized in hepatocytes of various species with little species variation.

**Experimental example 4: *In vivo* PK parameter testing**

Testing purposes:

Male SD rats and male cynomolgus monkeys were used as test animals, and the plasma concentration of the compound was determined and the pharmacokinetic behavior was evaluated after a single oral administration.

Experimental method:

Two groups of healthy rats (fasted) were administered by intravenous injection (IV) and oral gavage (PO), each group with 2 rats. The vehicle was a mixture of 20% PEG400, 10% solutol, and 70% water. After mixing the compound to be tested with the vehicle, the mixture was vortexed and sonicated to prepare clear solutions of 0.2 mg/mL and 0.5 mg/mL. The intravenous injection dose for rats was 1.0 mg/kg, and the oral gavage dose was 5.0 mg/kg. After administration, the whole blood was collected at the time points of 0.083 hours, 0.25 hours, 0.5 hours, 1.0 hour, 2.0 hours, 4.0 hours, 8.0 hours, 12.0 hours, and 24.0 hours. Plasma was prepared from the whole blood and analyzed for drug concentration by LC-MS/MS. The pharmacokinetic parameters were calculated using Phoenix WinNonlin 6.3, and the results are shown in Table 5.

Two groups of healthy male cynomolgus monkeys (fasted) were administered by intravenous injection and oral gavage, each group with 2 monkeys. The vehicle was a 20% hydroxypropyl-β-cyclodextrin aqueous solution. The compound to be tested **002** was mixed with the vehicle, then vortexed, and sonicated to prepare clear solutions of 1.0 mg/mL and 2.0 mg/mL. The intravenous injection dose was 1.0 mg/kg, and the oral gavage dose was 2.0 mg/kg. The compound to be tested **003** was mixed with the vehicle, then vortexed, and sonicated to prepare clear solutions of 0.25 mg/mL and 10.0 mg/mL. The intravenous injection dose was 0.5 mg/kg, and the oral gavage dose was 10.0 mg/kg. After administration, the whole blood was collected at the time points of 0.083 hours, 0.25 hours, 0.5 hours, 1.0 hour, 2.0 hours, 4.0 hours, 8.0 hours, 12.0 hours, and 24.0 hours. Plasma was prepared from the whole blood and analyzed for drug concentration by LC-MS/MS. The pharmacokinetic parameters were calculated using Phoenix WinNonlin 6.3, and the results are shown in Table 6. Parameter meaning: $C_{max}$ is the maximum plasma concentration; AUC is the exposure; $T_{1/2}$ is the half-life; Vd is the apparent volume of distribution; Cl is the clearance rate; F% is the oral bioavailability.

**Table 5 PK test results of compounds of the present disclosure in rats**

| Compound | AUC (PO) h*nmol/L | $T_{1/2}$ (PO) h | Vd L/kg | Cl mL/minutes/ kg | F% |
|---|---|---|---|---|---|
| **002** | 3439 | 1.61 | 0.392 | 12.0 | 27.1 |

**Table 6 PK test results of compounds of the present disclosure in cynomolgus monkeys**

| Compound | AUC (PO) h*nmol/L | $T_{1/2}$ (PO) h | Vd L/kg | Cl mL/minutes/kg | F% |
|---|---|---|---|---|---|
| **002** | 42966 | 11.3 | 0.155 | 0.505 | 35.3 |
| **003** | 10716 | 8.52 | 0.489 | 2.85 | 23.6 |

Conclusion: The compound of the present disclosure has high oral exposure, long half-life, high bioavailability and good *in vivo* pharmacokinetic properties.

**Claims**

**1.** A compound of formula (P) or a pharmaceutically acceptable salt thereof,

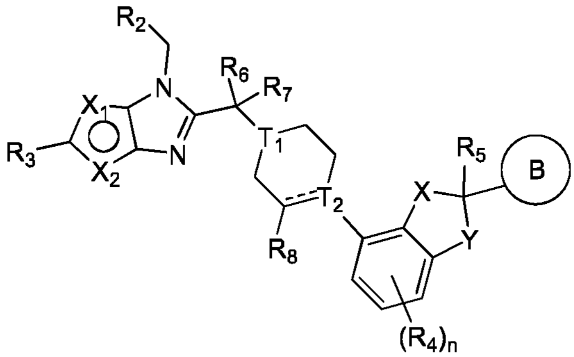

(P)

wherein

$\overline{- - -}$ is selected from a single bond and a double bond, and when $T_2$ is selected from N, $\overline{- - -}$ is selected from a single bond;
$T_1$ and $T_2$ are selected from N and $CR_9$;
X and Y are each independently selected from $CH_2$, $O\text{-}CH_2$, NH, O, and C(=O);
Xi and $X_2$ are each independently selected from CH, N, O, and S, and the CH is optionally substituted by one F, Cl, Br, and $CH_3$;
ring B is selected from 5- to 6-membered heteroaryl, and the 5- to 6-membered heteroaryl is optionally substituted by 1, 2, or 3 Ri;
or, ring B is selected from phenyl, and the phenyl is optionally substituted by 1, 2, or 3 Ri, and in this case, X and Y are not simultaneously selected from O;
$R_1$ is selected from F, Cl, Br, I, OH, $NH_2$, CN, $CH_3$, and $OCH_3$;
$R_2$ is selected from

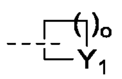 and 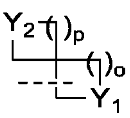 ,

and the

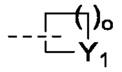 and 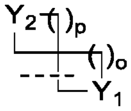

are optionally substituted by 1, 2, or 3 $R_a$;
$Y_1$ and $Y_2$ are each independently selected from $CH_2$, NH, and O;
o and p are each independently selected from 0, 1, 2, and 3;
$R_3$ is selected from -C(=O)-NH-$R_b$, -C(=O)-$R_b$, -C(=O)-NH-S(=O)$_2$-$R_b$, -S(=O)$_2$-NH-$R_b$, -S(=O)$_2$-$R_b$, -P(=O)($R_b$)$_2$, $C_{1-3}$ alkyl, tetrazolyl, isoxazolyl,

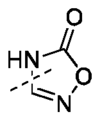, and 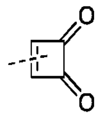,

and the $C_{1-3}$ alkyl, tetrazolyl, isoxazolyl,

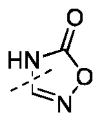, and 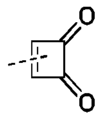

are optionally substituted by 1, 2, or 3 $R_b$;
$R_4$ is selected from D, F, Cl, Br, I, and $C_{1-3}$ alkyl, and the $C_{1-3}$ alkyl is optionally substituted by 1, 2, or 3 R;
$R_5$ is selected from H, D, and $CH_3$, and the $CH_3$ is optionally substituted by 1, 2, or 3 R;
$R_6$, $R_7$, $R_8$, and $R_9$ are each independently selected from H, D, and $C_{1-3}$ alkyl, and the $C_{1-3}$ alkyl is optionally substituted by 1, 2, or 3 R;
n is selected from 0, 1, and 2;
each $R_a$ is independently selected from F, Cl, Br, and I;
each $R_b$ is independently selected from OH, CN, $C_{1-3}$ alkyl, $C_{1-3}$ alkoxy, $C_{1-3}$ alkylamino, and oxazolyl, and the $C_{1-3}$ alkyl, $C_{1-3}$ alkoxy, and oxazolyl are optionally substituted by 1, 2, or 3 R;
each R is independently selected from D, F, Cl, Br, and I.

2. The compound or the pharmaceutically acceptable salt thereof according to claim 1, wherein each $R_b$ is independently selected from OH, CN, $CH_3$, $CF_3$, and $OCH_3$.

3. The compound or the pharmaceutically acceptable salt thereof according to claim 1, wherein $R_2$ is selected from

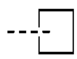 and 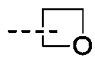,

and the

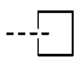 and 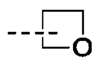

are optionally substituted by 1, 2, or 3 $R_a$; or, $R_2$ is selected from

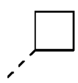 and 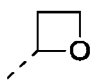.

4. The compound or the pharmaceutically acceptable salt thereof according to claim 1 or 2, wherein $R_3$ is selected

from -COOH, -C(=O)-NH-CN, -C(=O)-NH-OH, -C(=O)-NH-OCH$_3$, -C(=O)-CF$_3$, -S(=O)$_2$-NH-CH$_3$, and -S(=O)$_2$-OH; or, $R_3$ is selected from -COOH.

**5.** The compound or the pharmaceutically acceptable salt thereof according to claim 1, wherein $R_4$ is selected from F and $CH_3$.

**6.** The compound or the pharmaceutically acceptable salt thereof according to claim 1, wherein $R_5$ is selected from H, D, $CH_3$, $CF_3$, $CHF_2$, $CHD_2$, and $CD_3$; or, $R_5$ is selected from $CH_3$.

**7.** The compound or the pharmaceutically acceptable salt thereof according to claim 1, wherein $R_6$, $R_7$, $R_8$, and $R_9$ are each independently selected from H, D, and $CH_3$, and the $CH_3$ is optionally substituted by 1, 2, or 3 R; or, $R_6$, $R_7$, $R_8$, and $R_9$ are each independently selected from H, D, and $CH_3$.

**8.** The compound or the pharmaceutically acceptable salt thereof according to claim 1, wherein ring B is selected from pyridyl, pyrimidinyl, pyrazinyl, pyridazinyl, pyrazolyl, imidazolyl, thiazolyl, and oxazolyl, and the pyridyl, pyrimidinyl, pyrazinyl, pyridazinyl, pyrazolyl, imidazolyl, thiazolyl, and oxazolyl are optionally substituted by 1, 2, or 3 Ri; or, ring B is selected from

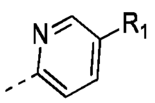, 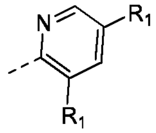, 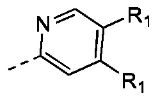, 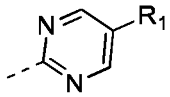, 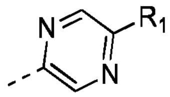,

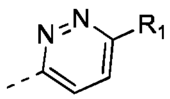, 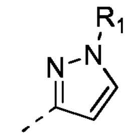, 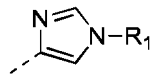, 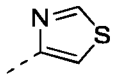, and 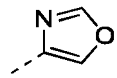.

**9.** The compound or the pharmaceutically acceptable salt thereof according to claim 8, wherein ring B is selected from

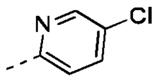, 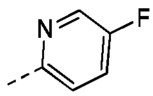, 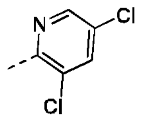, 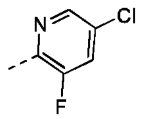,

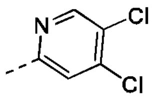, 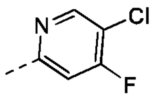, 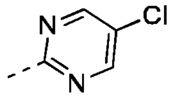, 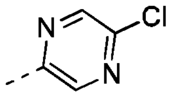, 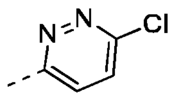, 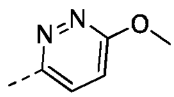,

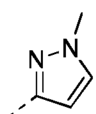, 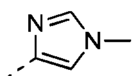, 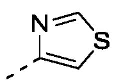, and 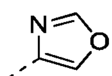;

or, ring B is selected from

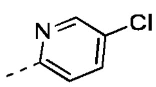,

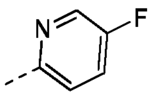, 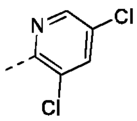, 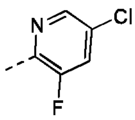, 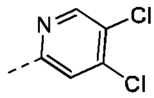, and 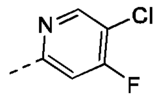.

**10.** The compound or the pharmaceutically acceptable salt thereof according to claim 1, wherein the structural moiety

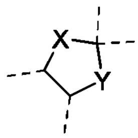

is selected from

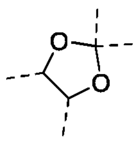, 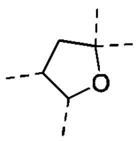,

and

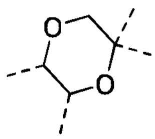.

**11.** The compound or the pharmaceutically acceptable salt thereof according to claim 1, wherein ring B is phenyl, and the phenyl is optionally substituted by 1, 2, or 3 $R_1$, and the structural moiety

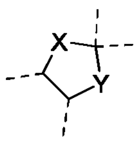

is selected from

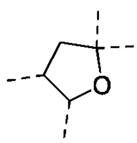 and 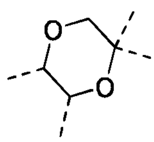;

or, ring B is selected from

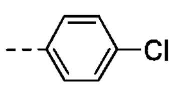 and 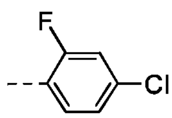,

and the structural moiety

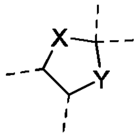

is selected from

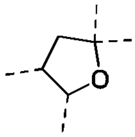 and 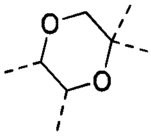.

**12.** The compound or the pharmaceutically acceptable salt thereof according to claim 1, wherein the structural moiety

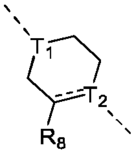

is selected from

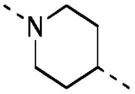, 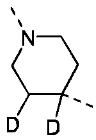, 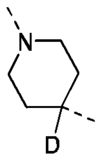,

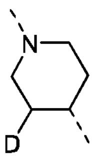, 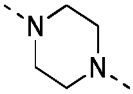, and 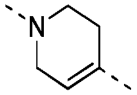;

or, the structural moiety

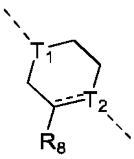

is selected from

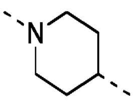, 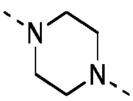, and 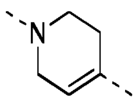.

**13.** The compound or the pharmaceutically acceptable salt thereof according to claim 1, wherein the structural moiety

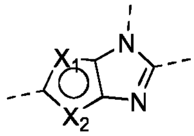

is selected from

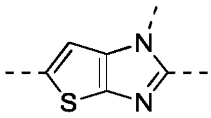

,

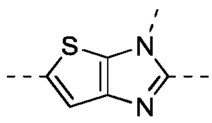

,

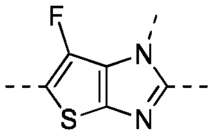

,

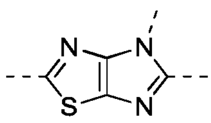

, and

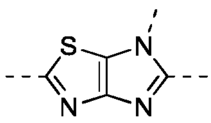

;

or, the structural moiety

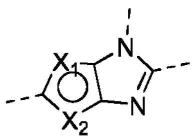

is selected from

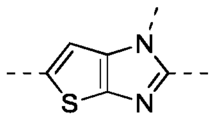

.

**14.** The compound or the pharmaceutically acceptable salt thereof according to any one of claims 1 to 10 or claim 13, wherein the compound is selected from:

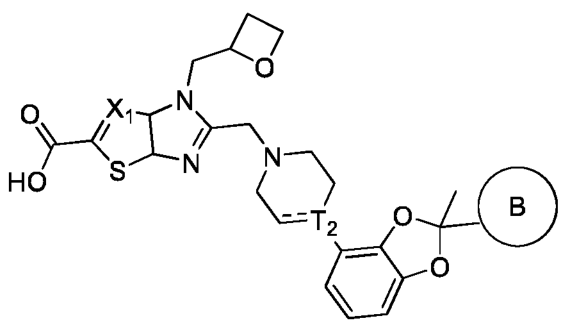

(I-1)

,

wherein

- - - is selected from a single bond and a double bond, and when $T_2$ is selected from N, - - - is selected from a single bond;

$X_1$ and $T_2$ are as defined in any one of claims 1 to 10 or claim 13;

ring B is as defined in any one of claims 1 to 10 or claim 13.

**15.** The compound or the pharmaceutically acceptable salt thereof according to claim 14, wherein the compound is selected from:

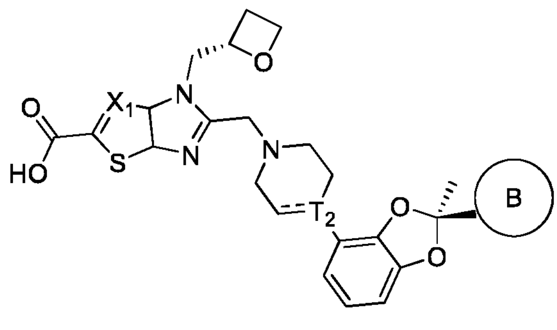

(I-1a) ,

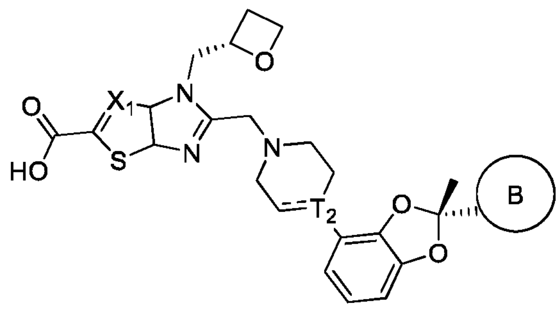

(I-1b) ,

wherein - - - is selected from a single bond and a double bond, and when $T_2$ is selected from N, - - - is selected from a single bond;
$X_1$, $T_2$, and ring B are as defined in claim 14.

**16.** A compound of the following formula or a pharmaceutically acceptable salt thereof,

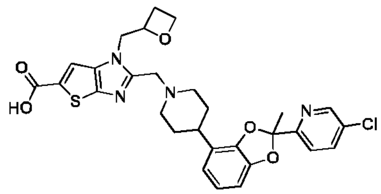

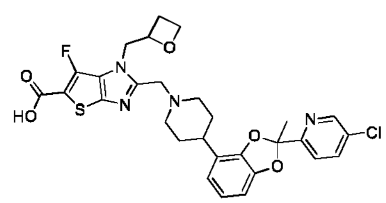

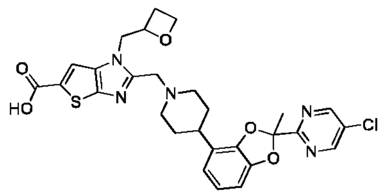

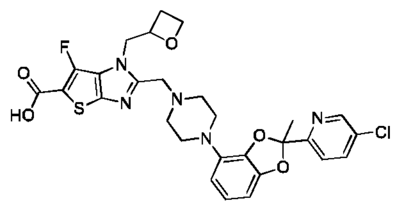

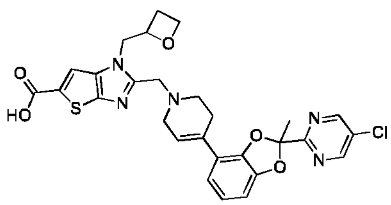

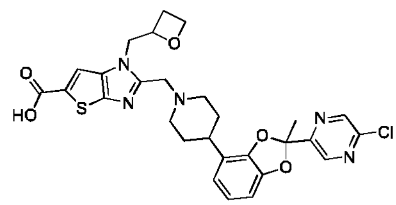

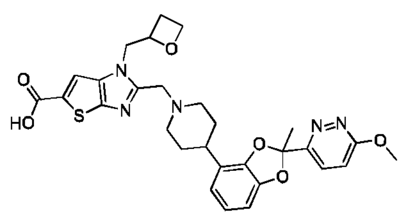

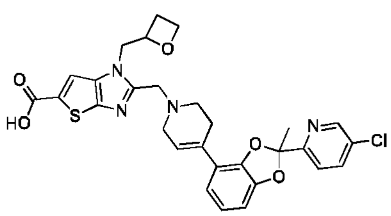

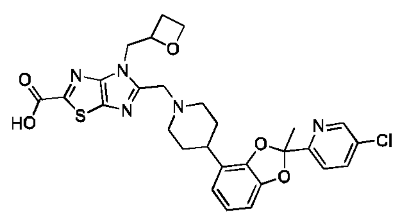

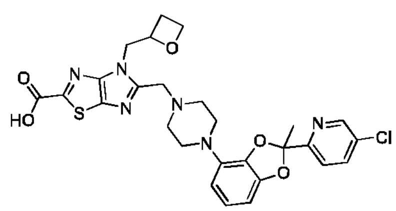

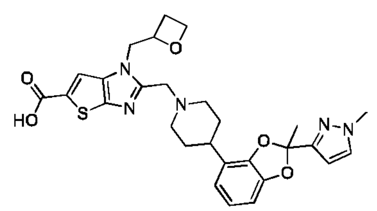

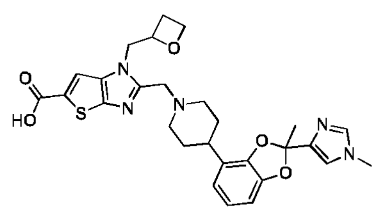

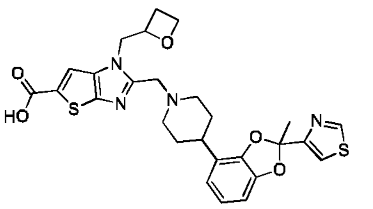

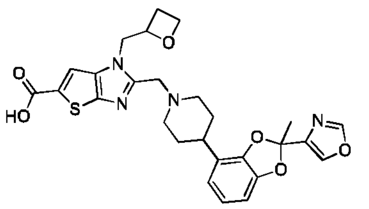

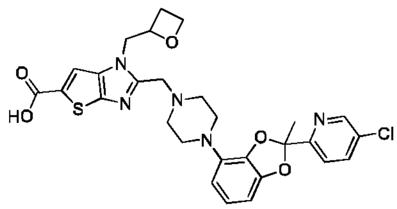

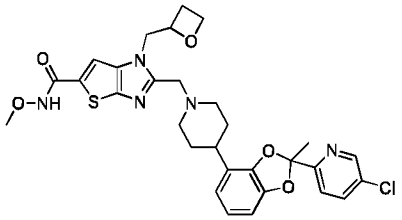

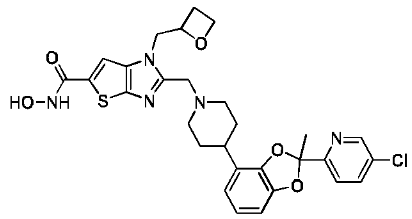

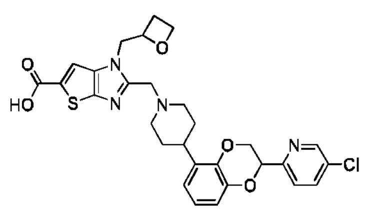

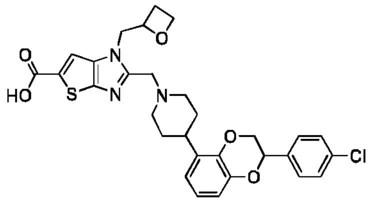

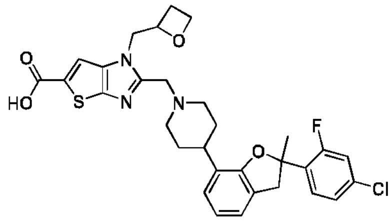

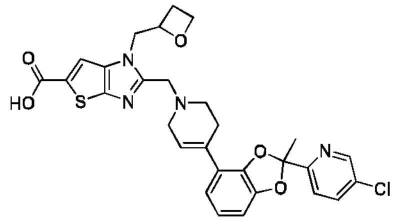

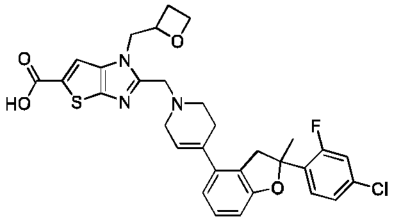

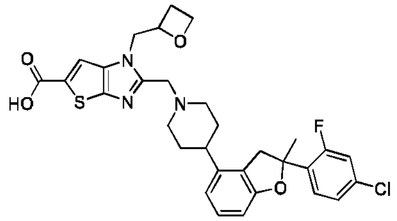

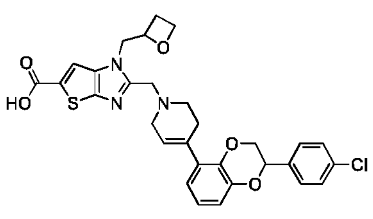

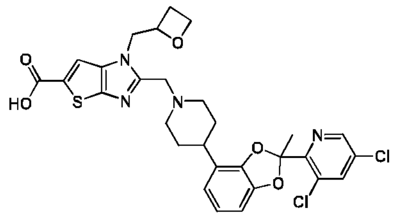

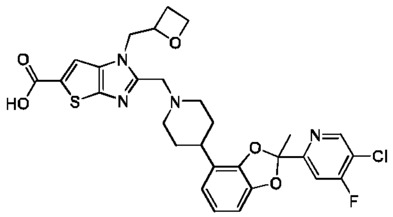

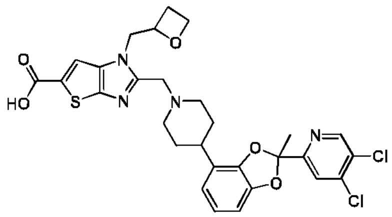

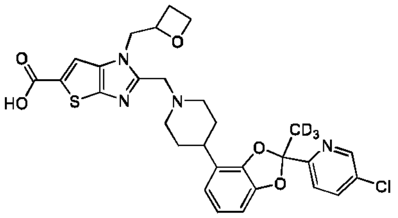

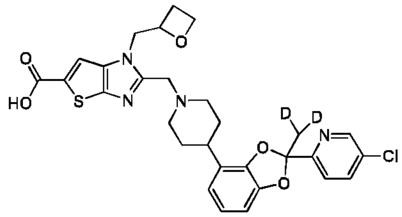

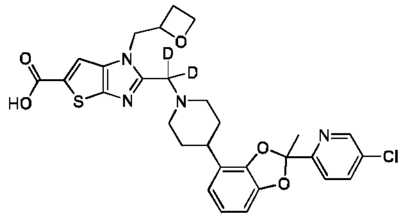

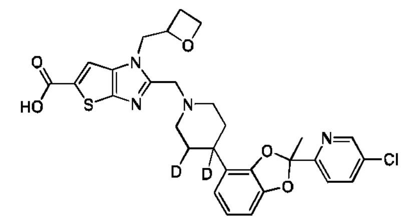 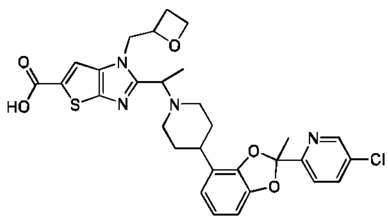 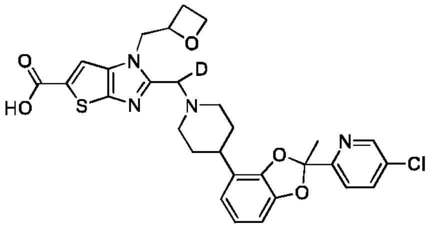 .

**17.** The compound or the pharmaceutically acceptable salt thereof according to claim 16, selected from,

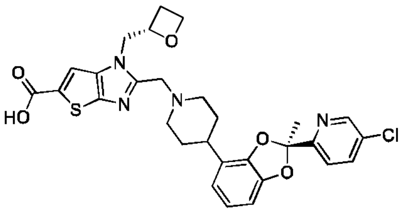 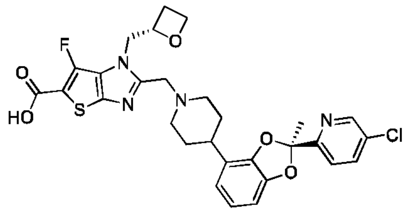 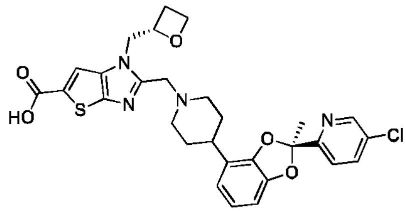

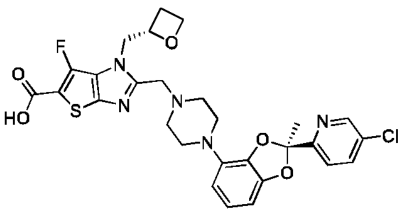 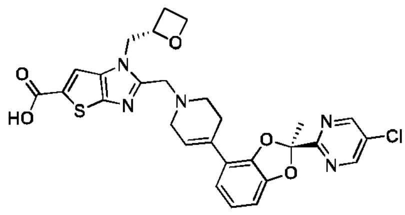 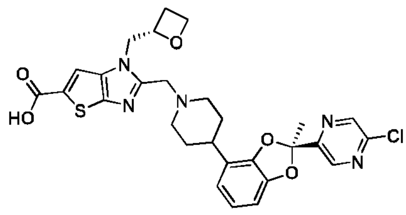

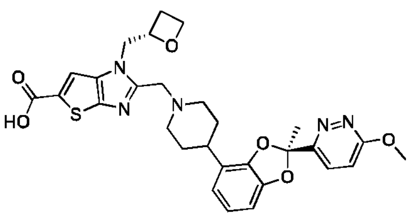 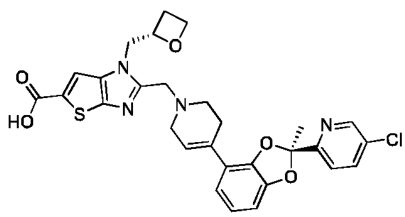 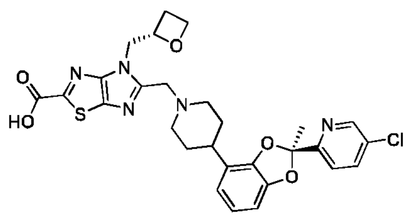

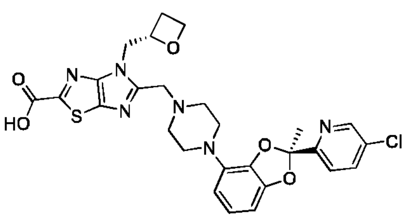 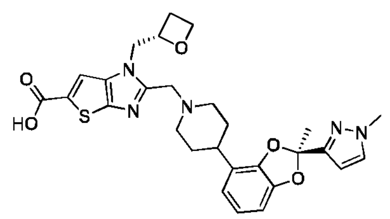 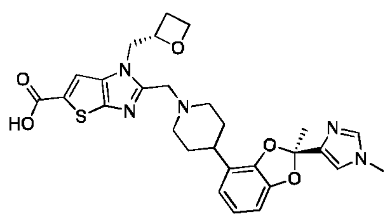

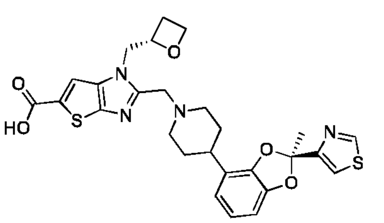 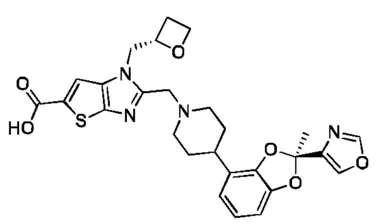 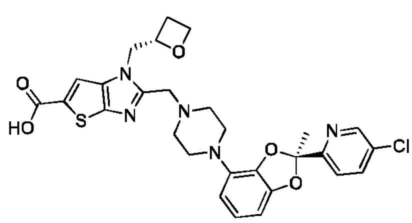

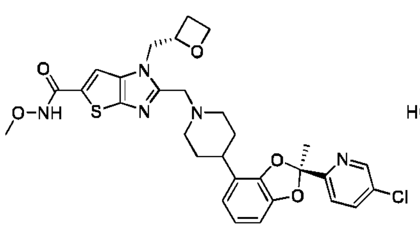 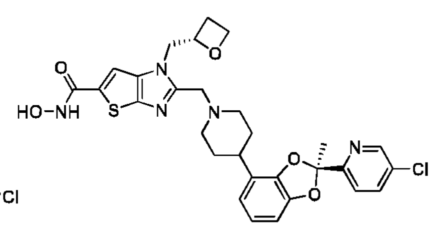 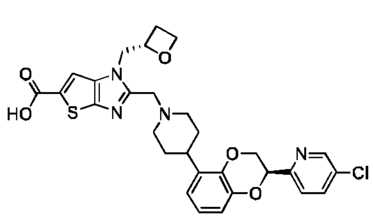

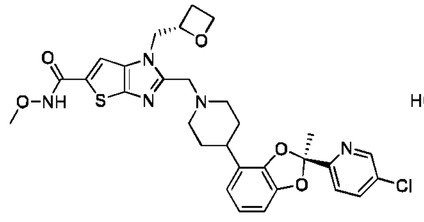
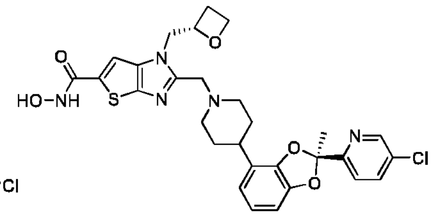
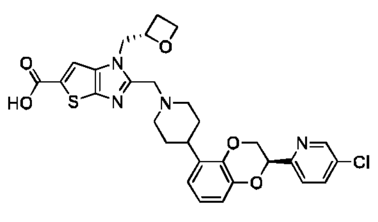

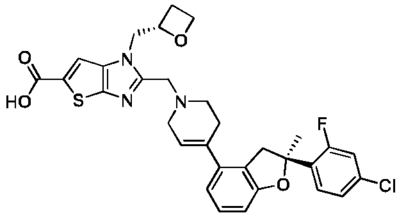
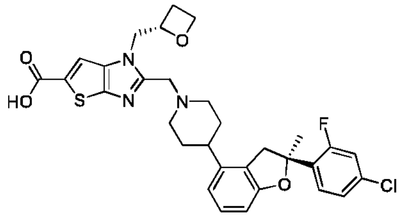
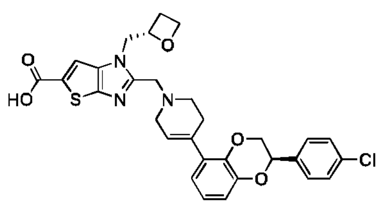

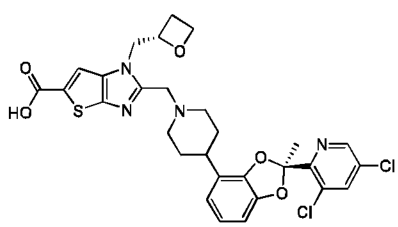
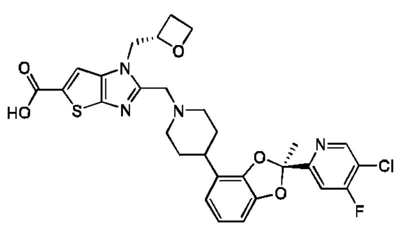
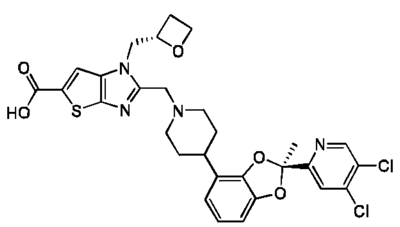

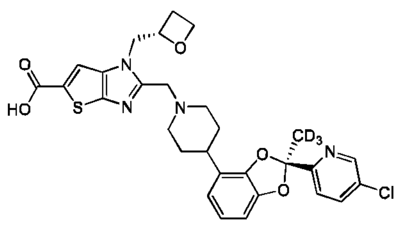
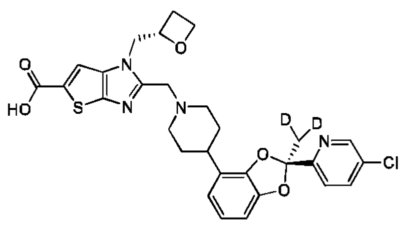
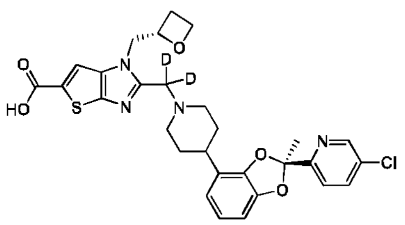

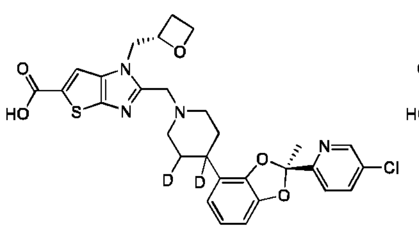
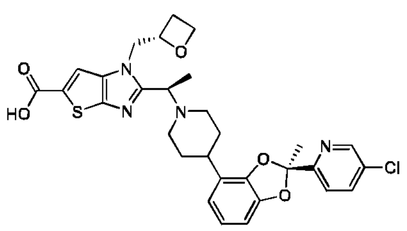
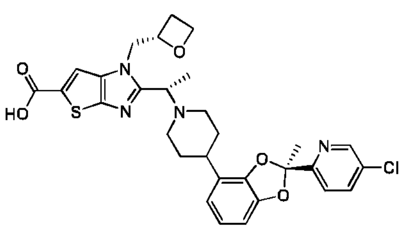

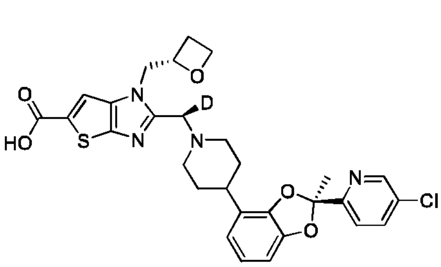
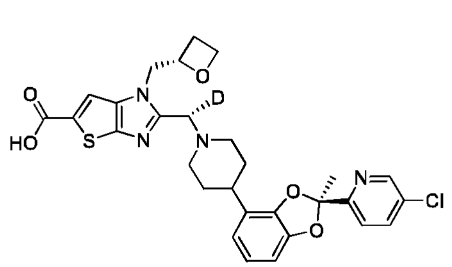

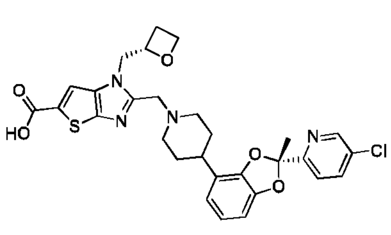
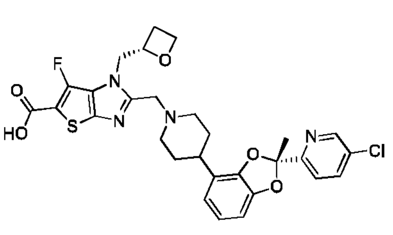
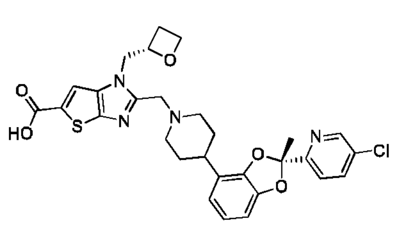

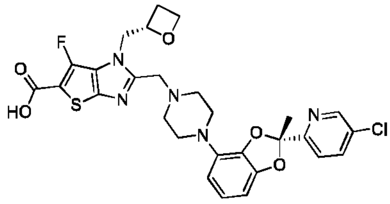
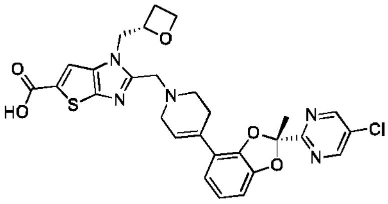
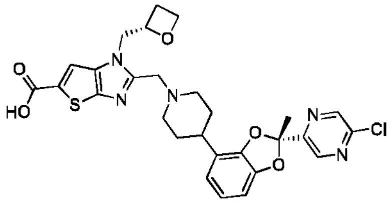
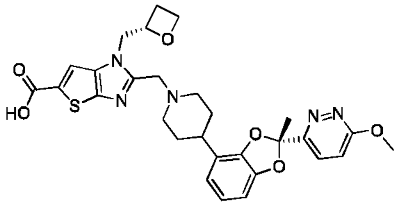
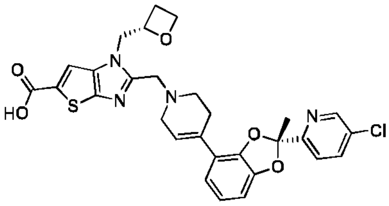
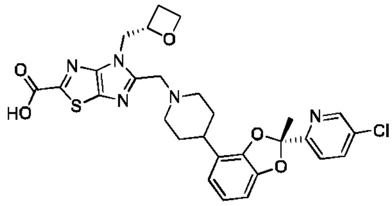
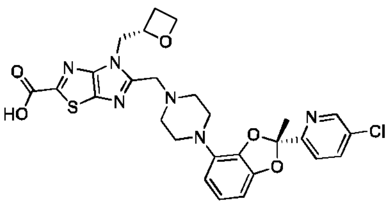
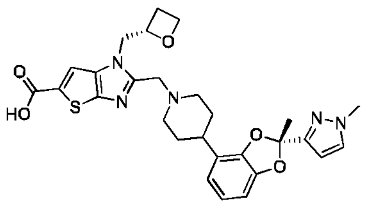
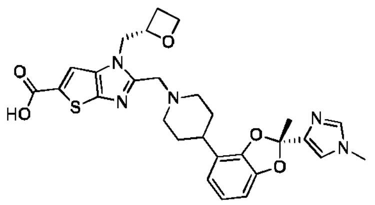
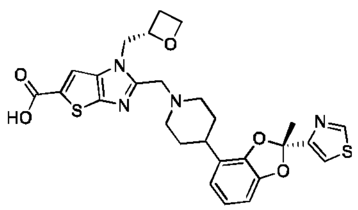
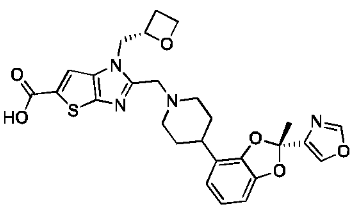
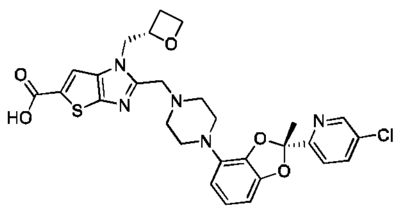
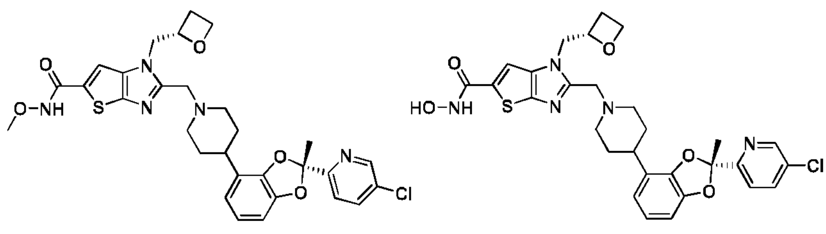
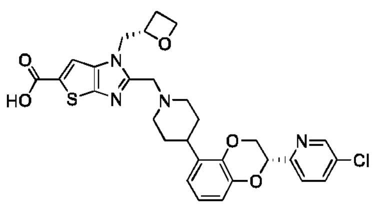
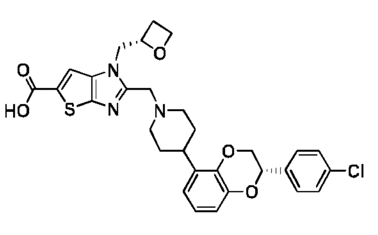
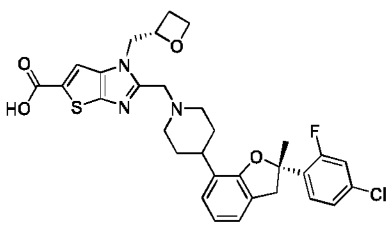
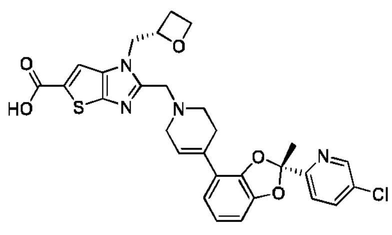
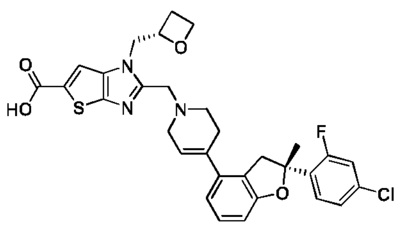
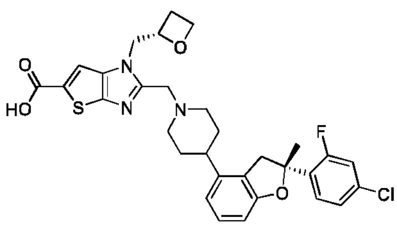
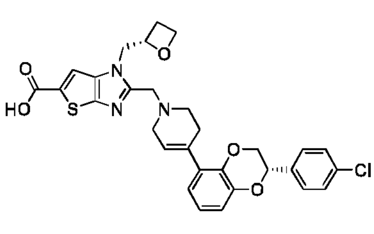

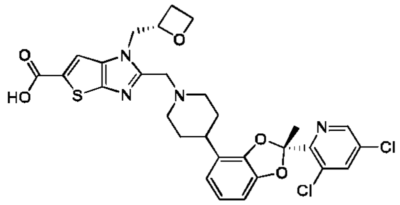
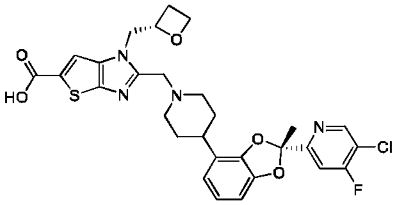
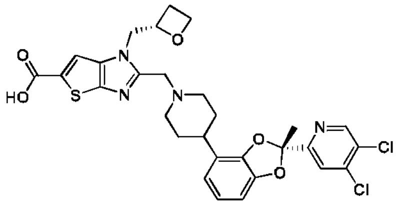

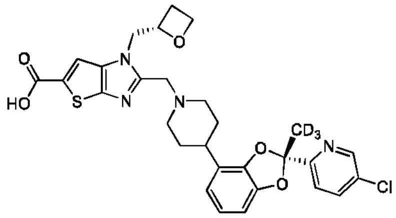
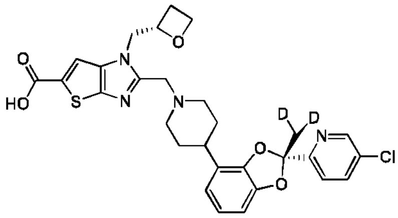
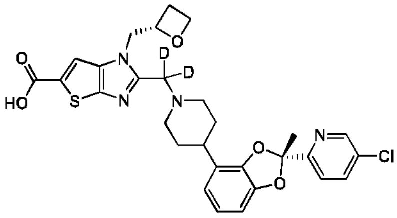

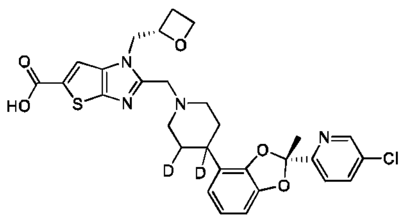
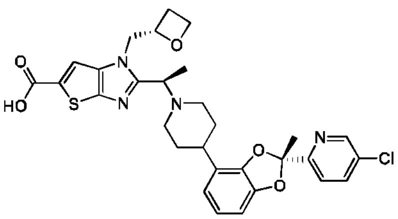
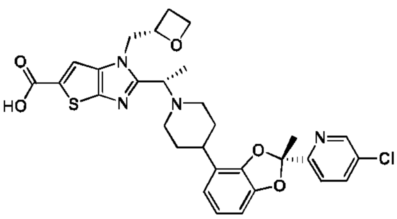

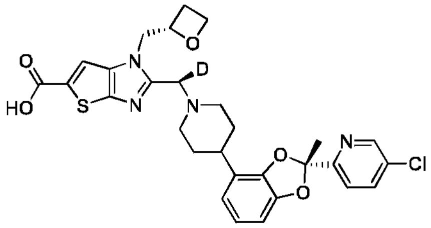
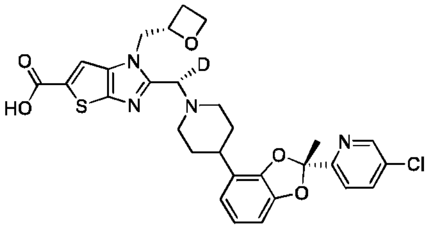
.

18. A use of the compound or the pharmaceutically acceptable salt thereof according to any one of claims 1 to 17 in the manufacture of a medicament for treating diabetes.

## INTERNATIONAL SEARCH REPORT

International application No.
**PCT/CN2022/114560**

### A. CLASSIFICATION OF SUBJECT MATTER

C07D 401/04(2006.01)i; C07D 413/14(2006.01)i; C07D 513/04(2006.01)i; C07D 405/14(2006.01)i; C07D 495/04(2006.01)i; A61K 31/4439(2006.01)i; A61K 31/444(2006.01)i; A61K 31/497(2006.01)i; A61K 31/4164(2006.01)i; A61P 3/10(2006.01)i

According to International Patent Classification (IPC) or to both national classification and IPC

### B. FIELDS SEARCHED

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

WPI, CAPLUS (STN), EPODOC, CNPAT, CNKI: 南京明德新药研发有限公司, 咪唑, 胰高血糖素样肽, 糖尿病, GLP, glucagon like peptide, diabete?, STN structural formula search

### C. DOCUMENTS CONSIDERED TO BE RELEVANT

| Category* | Citation of document, with indication, where appropriate, of the relevant passages | Relevant to claim No. |
|---|---|---|
| PX | CN 113801136 A (JIANGSU HENGRUI MEDICINE CO., LTD. et al.) 17 December 2021 (2021-12-17)<br>claims 1 and 22-31, and description, embodiments 2-3 | 1-18 |
| PX | WO 2022007979 A1 (JIANGSU HENGRUI MEDICINE CO., LTD. et al.) 13 January 2022 (2022-01-13)<br>claims 1 and 29-31, and description, embodiment 15 | 1-18 |
| PX | WO 2022135572 A1 (SICHUAN HAISCO PHARMACEUTICAL CO., LTD.) 30 June 2022 (2022-06-30)<br>claims 1-10, and description, embodiments 4-5 | 1-18 |
| A | CN 112533674 A (PFIZER INC.) 19 March 2021 (2021-03-19)<br>claims 1-21, 41, and 43, and description, embodiment 5 | 1-18 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 November 2022** | **23 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/114560**

| Patent document cited in search report | | | Publication date (day/month/year) | Patent family member(s) | | | Publication date (day/month/year) |
|---|---|---|---|---|---|---|---|
| CN | 113801136 | A | 17 December 2021 | None | | | |
| WO | 2022007979 | A1 | 13 January 2022 | TW | 202220985 | A | 01 June 2022 |
| WO | 2022135572 | A1 | 30 June 2022 | None | | | |
| CN | 112533674 | A | 19 March 2021 | US | 2021163455 | A1 | 03 June 2021 |
| | | | | SG | 11202011465 Q | A | 30 December 2020 |
| | | | | EP | 3806955 | A1 | 21 April 2021 |
| | | | | CO | 2020015305 | A2 | 08 March 2021 |
| | | | | IL | 279300 | A | 31 January 2021 |
| | | | | CU | 20200099 | A7 | 06 August 2021 |
| | | | | PH | 12020552069 | A1 | 31 May 2021 |
| | | | | KR | 20210019529 | A | 22 February 2021 |
| | | | | UA | 124371 | C2 | 01 September 2021 |
| | | | | US | 2019382387 | A1 | 19 December 2019 |
| | | | | CR | 20200612 | A | 20 January 2021 |
| | | | | US | 2019382388 | A1 | 19 December 2019 |
| | | | | JP | 6916968 | B1 | 11 August 2021 |
| | | | | RU | 2769715 | C1 | 05 April 2022 |
| | | | | US | 2019382384 | A1 | 19 December 2019 |
| | | | | EC | SP20078651 | A | 29 January 2021 |
| | | | | AU | 2019285491 | A1 | 17 December 2020 |
| | | | | NI | 202000091 | A | 23 March 2021 |
| | | | | BR | 112020024470 | A2 | 02 March 2021 |
| | | | | UY | 38261 | A | 31 January 2020 |
| | | | | WO | 2019239319 | A1 | 19 December 2019 |
| | | | | MA | 52882 | A | 21 April 2021 |
| | | | | CL | 2020003222 | A1 | 30 April 2021 |
| | | | | TW | 202015679 | A | 01 May 2020 |
| | | | | CA | 3045644 | A1 | 13 December 2019 |
| | | | | PE | 20211601 | A1 | 18 August 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110976089
- CN 202111154964
- CN 202111162621